(19) Europäisches Patentamt / European Patent Office / Office européen des brevets

(11) EP 3 692 065 B1

# (12) EUROPEAN PATENT SPECIFICATION

(45) Date of publication and mention of the grant of the patent:
**16.02.2022 Bulletin 2022/07**

(21) Application number: **18778497.0**

(22) Date of filing: **01.10.2018**

(51) International Patent Classification (IPC):
***C07K 16/28*** $^{(2006.01)}$ ***C07K 16/32*** $^{(2006.01)}$

(52) Cooperative Patent Classification (CPC):
**C07K 16/2809; C07K 16/2803; C07K 16/2818; C07K 16/32;** A61K 2039/505; C07K 2317/21; C07K 2317/24; C07K 2317/31; C07K 2317/41; C07K 2317/524; C07K 2317/70; C07K 2317/71; C07K 2317/76; C07K 2317/92; C07K 2317/94;
(Cont.)

(86) International application number:
**PCT/EP2018/076631**

(87) International publication number:
**WO 2019/068632 (11.04.2019 Gazette 2019/15)**

## (54) IGG1 FC MUTANTS WITH ABLATED EFFECTOR FUNCTIONS

IGG1-FC-MUTANTEN MIT ABLATIERTEN EFFEKTORFUNKTIONEN

MUTANTS FC IGG1 AVEC FONCTIONS D’EFFECTEUR SUPPRIMÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2017 EP 17194368**
**24.04.2018 EP 18168959**

(43) Date of publication of application:
**12.08.2020 Bulletin 2020/33**

(73) Proprietor: **Cilag GmbH International**
**6300 Zug (CH)**

(72) Inventors:
- **BRACK, Simon, Sebastian**
**2333 CP Leiden (NL)**
- **ATTINGER-TOLLER, Isabella**
**2333 CN Leiden (NL)**
- **BULLER, Fabian**
**2333 CN Leiden (NL)**
- **GRABULOVSKI, Dragan**
**2333 CN Leiden (NL)**
- **BERTSCHINGER, Julian**
**2333 CN Leiden (NL)**
- **ZUMSTEG, Adrian**
**2333 CN Leiden (NL)**

(74) Representative: **Verhage, Richard Abraham et al**
**Janssen Vaccines & Prevention B.V.**
**Archimedesweg 4-6**
**2333 CN Leiden (NL)**

(56) References cited:
**WO-A1-2012/130831 WO-A1-2014/170063**
**WO-A1-2016/050721 WO-A1-2016/081643**

- **MEGAN LO ET AL: "Effector-attenuating Substitutions That Maintain Antibody Stability and Reduce Toxicity in Mice", JOURNAL OF BIOLOGICAL CHEMISTRY, vol. 292, no. 9, 11 January 2017 (2017-01-11), pages 3900-3908, XP055428854, ISSN: 0021-9258, DOI: 10.1074/jbc.M116.767749**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 692 065 B1

(52) Cooperative Patent Classification (CPC): (Cont.)
C07K 2318/20; C07K 2319/30

SEQUENCE LISTING

<110> BRACK, SIMON
ATTINGER-TOLLER, ISABELLA
BULLER, FABIAN
GRABULOVSKI, DRAGAN
BERTSCHINGER, JULIAN
ZUMSTEG, ADRIAN

<120> IGG1 FC MUTANTS WITH ABLATED EFFECTOR FUNCTIONS

<130> COV5009WOPCT1

<140>
<141>

<160> 61

<170> PatentIn version 3.5

<210> 1
<211> 1356
<212> DNA
<213> Artificial Sequence

<220>
<221> source
<223> /note="Description of Artificial Sequence: Synthetic polynucleotide"

<400> 1

```
caggtccagc tgcagcagag tggggccgaa ctggcaagac ccggagcaag cgtcaaaatg      60
tcatgtaaag caagcggtta tactttcact aggagcacca tgcactgggt gaaacagagg     120
cccggccagg gactggagtg gatcgggtac attaaccctt ccagcgctta caccaactat     180
aatcagaagt tcaaagacaa ggccaccctg acagctgata agtctagttc aacagcatat     240
atgcagctgt ccagcctgac ttctgaagac agtgcagtgt actattgcgc ctccccacag     300
gtccactacg attacaatgg ttttccttac tgggggcagg gcacactggt gactgtctcc     360
gccgctagca caaagggccc tagtgtgttt cctctggctc cctcttccaa atccacttct     420
ggtggcactg ctgctctggg atgcctggtg aaggattact ttcctgaacc tgtgactgtc     480
tcatggaact ctggtgctct gacttctggt gtccacactt tccctgctgt gctgcagtct     540
agtggactgt actctctgtc atctgtggtc actgtgccct cttcatctct gggaacccag     600
acctacattt gtaatgtgaa ccacaaacca tccaacacta aagtggacaa aaaagtggaa     660
cccaaatcct gtgacaaaac ccacacctgc ccaccttgtc ctgcccctga actgctggga     720
ggaccttctg tgtttctgtt ccccccccaaa ccaaaggata ccctgatgat ctctagaacc    780
cctgaggtga catgtgtggt ggtggatgtg tctcatgagg accctgaggt caaattcaac     840
tggtacgtgg atggagtgga agtccacaat gccaaaacca agcctagaga ggaacagtac     900
aattcaacct acagagttgt cagtgtgctg actgtgctgc atcaggattg gctgaatggc     960

aaggaataca agtgtaaagt ctcaaacaag gccctgcctg ctccaattga gaaaacaatc    1020
tcaaaggcca agggacagcc tagggaaccc caggtctaca ccctgccacc ttcaagagag    1080
gaaatgacca aaaaccaggt gtccctgaca tgcctggtca aaggcttcta cccttctgac    1140
attgctgtgg agtgggagtc aaatggacag cctgagaaca actacaaaac aacccccccct   1200
gtgctggatt ctgatggctc tttctttctg tactccaaac tgactgtgga caagtctaga    1260
tggcagcagg ggaatgtctt ttcttgctct gtcatgcatg aggctctgca taaccactac    1320
actcagaaat ccctgtctct gtctcccggg aaatga                              1356
```

<210> 2
<211> 451
<212> PRT
<213> Artificial Sequence

<220>
<221> source
<223> /note="Description of Artificial Sequence: Synthetic polypeptide"

<400> 2

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
```

145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Leu Leu Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Glu Glu Met Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 3
<211> 642
<212> DNA
<213> Artificial Sequence

<220>
<221> source
<223> /note="Description of Artificial Sequence: Synthetic polynucleotide"

<400> 3

caggtggtgc tgacccagag ccctgctatt atgtccgcat tccccggtga aaaagtgact 60

atgacttgtt ccgcttcttc ctccgtctcc tacatgaact ggtatcagca gaagtcagga 120

acatctccca aaaggtggat ctacgactcc agcaagctgg catccggcgt gcctgcacga 180

ttctcaggct ccggaagcgg gacctcttat agtctgacaa tttctagtat ggagactgaa 240

gatgccgcta cctactattg ccagcagtgg tcaagaaacc ctccaacatt cggggggggg 300

actaaactgc agattactcg tacggtcgcg gcgccttctg tgttcatttt cccccccatct 360

gatgaacagc tgaaatctgg cactgcttct gtggtctgtc tgctgaacaa cttctaccct 420

agagaggcca aagtccagtg gaaagtggac aatgctctgc agagtgggaa ttcccaggaa 480

tctgtcactg agcaggactc taaggatagc acatactccc tgtcctctac tctgacactg 540

agcaaggctg attacgagaa acacaaagtg tacgcctgtg aagtcacaca tcaggggctg 600

tctagtcctg tgaccaaatc cttcaatagg ggagagtgct ga 642

<210> 4
<211> 213
<212> PRT
<213> Artificial Sequence

<220>
<221> source
<223> /note="Description of Artificial Sequence: Synthetic polypeptide"

<400> 4

```
Gln Val Val Leu Thr Gln Ser Pro Ala Ile Met Ser Ala Phe Pro Gly
1               5                   10                  15

Glu Lys Val Thr Met Thr Cys Ser Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Ser Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Thr Glu
65                  70                  75                  80

Asp Ala Ala Thr Tyr Tyr Cys Gln Gln Trp Ser Arg Asn Pro Pro Thr
                85                  90                  95

Phe Gly Gly Gly Thr Lys Leu Gln Ile Thr Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210
```

<210> 5
<211> 1353
<212> DNA
<213> Artificial Sequence

<220>
<221> source

<223> /note="Description of Artificial Sequence: Synthetic polynucleotide"

<400> 5

```
caggtgcagc tccagcagag tggcgcagag ctggtgaagc ccggagcctc agtcaagatg      60
tcctgcaagg ccttcggcta cacttttacc acatatccta tcgagtggat gaagcagaac     120
cacgggaaaa gcctggaatg gattggtaac ttccatccat acaatgacga taccaagtat     180
aatgagaagt ttaaaggcaa ggcaaaactg acagtggaga aatccagcac taccgtctac     240
ctggaactgt ccaggctgac atctgacgat agtgccgtgt actattgtgc tcgggaaaac     300
tacggaagcc acggcggatt cgtctattgg gggcagggta cactggtgac tgtctctgcc     360
gctagcacaa agggccctag tgtgtttcct ctggctccct cttccaaatc cacttctggt     420
ggcactgctg ctctgggatg cctggtgaag gattactttc ctgaacctgt gactgtctca     480
tggaactctg gtgctctgac ttctggtgtc cacactttcc ctgctgtgct gcagtctagt     540
ggactgtact ctctgtcatc tgtggtcact gtgccctctt catctctggg aacccagacc     600
tacatttgta atgtgaacca caaaccatcc aacactaaag tggacaaaaa agtggaaccc     660
aaatcctgtg acaaaaccca cacctgccca ccttgtcctg cccctgaact gctgggagga     720
ccttctgtgt ttctgttccc ccccaaacca aaggataccc tgatgatctc tagaacccct     780
gaggtgacat gtgtggtggt ggctgtgtct catgaggacc ctgaggtcaa attcaactgg     840
tacgtggatg gagtggaagt ccacaatgcc aaaaccaagc ctagagagga acagtacgct     900
tcaacctaca gagttgtcag tgtgctgact gtgctgcatc aggattggct gaatggcaag     960
gaatacaagt gtaaagtctc aaacaaggcc ctggctgctc caattgagaa aacaatctca    1020
aaggccaagg gacagcctag ggaaccccag gtctacaccc tgccaccttc aagagaggaa    1080
atgaccaaaa accaggtgtc cctgacatgc ctggtcaaag gcttctaccc ttctgacatt    1140
gctgtggagt gggagtcaaa tggacagcct gagaacaact acaaaacaac cccccctgtg    1200
ctggattctg atggctcttt ctttctgtac tccaaactga ctgtggacaa gtctagatgg    1260
cagcagggga atgtcttttc ttgctctgtc atgcatgagg ctctgcataa ccactacact    1320
cagaaatccc tgtctctgtc tcccgggaaa tga                                 1353
```

<210> 6
<211> 450
<212> PRT
<213> Artificial Sequence

<220>
<221> source
<223> /note="Description of Artificial Sequence: Synthetic polypeptide"

<400> 6

```
Gln Val Gln Leu Gln Gln Ser Gly Ala Glu Leu Val Lys Pro Gly Ala
1               5                   10                  15
```

```
Ser Val Lys Met Ser Cys Lys Ala Phe Gly Tyr Thr Phe Thr Thr Tyr
            20                  25                  30

Pro Ile Glu Trp Met Lys Gln Asn His Gly Lys Ser Leu Glu Trp Ile
        35                  40                  45

Gly Asn Phe His Pro Tyr Asn Asp Asp Thr Lys Tyr Asn Glu Lys Phe
    50                  55                  60

Lys Gly Lys Ala Lys Leu Thr Val Glu Lys Ser Ser Thr Thr Val Tyr
65                  70                  75                  80

Leu Glu Leu Ser Arg Leu Thr Ser Asp Asp Ser Ala Val Tyr Tyr Cys
                85                  90                  95

Ala Arg Glu Asn Tyr Gly Ser His Gly Gly Phe Val Tyr Trp Gly Gln
            100                 105                 110

Gly Thr Leu Val Thr Val Ser Ala Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Ala Val Ser His Glu
            260                 265                 270
```

```
Asp Pro Glu Val Lys Phe Asn Trp Tyr Val Asp Gly Val Glu Val His
        275                 280                 285

Asn Ala Lys Thr Lys Pro Arg Glu Glu Gln Tyr Ala Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Ala Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

<210> 7
<211> 648
<212> DNA
<213> Artificial Sequence

<220>
<221> source
<223> /note="Description of Artificial Sequence: Synthetic polynucleotide"

<400> 7

```
gagaacgtgc tgacccagtc ccccgcaatc atgtctgcca gtcctggaga aaaggtcacc        60
```

atgacatgca gggcatccag ctctgtcatc agttcatacc tgcactggta tcagcagaag 120

agcggagctt ctccaaaact gtggatctac tcaacctcca acctggcaag cggggtgccc 180

gaccggttca gcggctctgg aagtgggact tcatatagtc tgaccatctc gtcggtcgag 240

gccgaagatg ccgctacata ctattgtcag cagtacaatg gctatcccct gacatttggt 300

gctggtacca aactcgagat taagcgtacg gtcgcggcgc cttctgtgtt cattttcccc 360

ccatctgatg aacagctgaa atctggcact gcttctgtgg tctgtctgct gaacaacttc 420

taccctagag aggccaaagt ccagtggaaa gtggacaatg ctctgcagag tgggaattcc 480

caggaatctg tcactgagca ggactctaag gatagcacat actccctgtc ctctactctg 540

acactgagca aggctgatta cgagaaacac aaagtgtacg cctgtgaagt cacacatcag 600

gggctgtcta gtcctgtgac caaatccttc aatagggag agtgctga 648

<210> 8
<211> 215
<212> PRT
<213> Artificial Sequence

<220>
<221> source
<223> /note="Description of Artificial Sequence: Synthetic polypeptide"

<400> 8

```
Glu Asn Val Leu Thr Gln Ser Pro Ala Ile Met Ser Ala Ser Pro Gly
1               5                   10                  15

Glu Lys Val Thr Met Thr Cys Arg Ala Ser Ser Ser Val Ile Ser Ser
            20                  25                  30

Tyr Leu His Trp Tyr Gln Gln Lys Ser Gly Ala Ser Pro Lys Leu Trp
        35                  40                  45

Ile Tyr Ser Thr Ser Asn Leu Ala Ser Gly Val Pro Asp Arg Phe Ser
    50                  55                  60

Gly Ser Gly Ser Gly Thr Ser Tyr Ser Leu Thr Ile Ser Ser Val Glu
65                  70                  75                  80

Ala Glu Asp Ala Ala Thr Tyr Tyr Cys Gln Gln Tyr Asn Gly Tyr Pro
                85                  90                  95

Leu Thr Phe Gly Ala Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 9
<211> 1353
<212> DNA
<213> Artificial Sequence

<220>

<221> source
<223> /note="Description of Artificial Sequence: Synthetic polynucleotide"

<400> 9

gaggttcagc tggtggagtc tggcggtggc ctggtgcagc caggggggctc actccgtttg 60
tcctgtgcag cttctggctt caacattaaa gacacctata tacactgggt gcgtcaggcc 120
ccgggtaagg gcctggaatg ggttgcaagg atttatccta cgaatggtta tactagatat 180
gccgatagcg tcaagggccg tttcactata agcgcagaca catccaaaaa cacagcctac 240
ctgcagatga acagcctgcg tgctgaggac actgccgtct attattgttc tagatgggga 300
ggggacggct tctatgctat ggactactgg ggtcaaggaa ccctggtcac cgtctcctcg 360
gctagcacaa agggccctag tgtgtttcct ctggctccct cttccaaatc cacttctggt 420
ggcactgctg ctctgggatg cctggtgaag gattactttc ctgaacctgt gactgtctca 480
tggaactctg gtgctctgac ttctggtgtc cacactttcc ctgctgtgct gcagtctagt 540
ggactgtact ctctgtcatc tgtggtcact gtgccctctt catctctggg aacccagacc 600
tacatttgta atgtgaacca caaaccatcc aacactaaag tggacaaaaa agtggaaccc 660
aaatcctgtg acaaaaccca cacctgccca ccttgtcctg cccctgaact gctgggagga 720
ccttctgtgt ttctgttccc ccccaaacca aaggataccc tgatgatctc tagaacccct 780
gaggtgacat gtgtggtggt ggctgtgtct catgaggacc ctgaggtcaa attcaactgg 840
tacgtggatg gagtggaagt ccacaatgcc aaaaccaagc ctagagagga acagtacgct 900
tcaacctaca gagttgtcag tgtgctgact gtgctgcatc aggattggct gaatggcaag 960
gaatacaagt gtaaagtctc aaacaaggcc ctggctgctc caattgagaa aacaatctca 1020
aaggccaagg gacagcctag ggaaccccag gtctacaccc tgccaccttc aagagaggaa 1080
atgaccaaaa accaggtgtc cctgacatgc ctggtcaaag gcttctaccc ttctgacatt 1140
gctgtggagt gggagtcaaa tggacagcct gagaacaact acaaaacaac ccccccctgtg 1200
ctggattctg atggctcttt ctttctgtac tccaaactga ctgtggacaa gtctagatgg 1260
cagcagggga atgtcttttc ttgctctgtc atgcatgagg ctctgcataa ccactacact 1320
cagaaatccc tgtctctgtc tcccgggaaa tga 1353

<210> 10
<211> 450
<212> PRT
<213> Artificial Sequence

<220>
<221> source
<223> /note="Description of Artificial Sequence: Synthetic polypeptide"

<400> 10

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125
```

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Ala Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Ala Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Ala Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 11
<211> 645
<212> DNA
<213> Artificial Sequence

<220>
<221> source
<223> /note="Description of Artificial Sequence: Synthetic polynucleotide"

<400> 11

gatatccaga tgacccagtc cccgagctcc ctgtccgcct ctgtgggcga tagggtcacc 60

atcacctgcc gtgccagtca ggatgtgaat actgctgtag cctggtatca acagaaacca 120

ggaaaagctc cgaaactact gatttactcg gcatccttcc tctactctgg agtcccttct 180

cgcttctctg ggtccagatc tgggacggat ttcactctga ccatcagcag tctgcagccg 240

gaagacttcg caacttatta ctgtcagcaa cattatacta ctcctcccac gttcggacag 300

gggaccaagg tggagatcaa acgtacggtc gcggcgcctt ctgtgttcat tttcccccca 360

tctgatgaac agctgaaatc tggcactgct tctgtggtct gtctgctgaa caacttctac 420

cctagagagg ccaaagtcca gtggaaagtg gacaatgctc tgcagagtgg gaattcccag 480

gaatctgtca ctgagcagga ctctaaggat agcacatact ccctgtcctc tactctgaca 540

ctgagcaagg ctgattacga gaaacacaaa gtgtacgcct gtgaagtcac acatcagggg 600

ctgtctagtc ctgtgaccaa atccttcaat aggggagagt gctga 645

<210> 12
<211> 214
<212> PRT
<213> Artificial Sequence

<220>
<221> source
<223> /note="Description of Artificial Sequence: Synthetic polypeptide"

<400> 12

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 13
<211> 1368
<212> DNA
<213> Artificial Sequence

<220>
<221> source
<223> /note="Description of Artificial Sequence: Synthetic polynucleotide"

<400> 13

```
gaggtgcagc tggtcgagtc tggaggagga ttggtgcagc ctggagggtc attgaaactc      60
tcatgtgcag cctctggatt caccttcaat acctacgcca tgaactgggt ccgccaggct     120
ccaggaaagg gtttggaatg ggttgctcgc ataagaagta aatataataa ttatgcaaca     180
tattatgccg attcagtgaa agacaggttc accatctcca gagatgattc aaaaaacact     240
gcctatctac aaatgaacag cttgaaaact gaggacactg ccgtgtacta ctgtgtgaga     300
catgggaact tcggtgatag ctacgtttcc tggtttgctt actggggcca agggactctg     360
gtcaccgtct cgagcgctag cacaaagggc cctagtgtgt ttcctctggc tccctcttcc     420
aaatccactt ctggtggcac tgctgctctg ggatgcctgg tgaaggatta ctttcctgaa     480
cctgtgactg tctcatggaa ctctggtgct ctgacttctg gtgtccacac tttccctgct     540
gtgctgcagt ctagtggact gtactctctg tcatctgtgg tcactgtgcc ctcttcatct     600
ctgggaaccc agacctacat ttgtaatgtg aaccacaaac catccaacac taaagtggac     660
aaaaaagtgg aacccaaatc ctgtgacaaa acccacacct gcccaccttg tcctgcccct     720
gaactgctgg gaggaccttc tgtgtttctg ttccccccca aaccaaagga taccctgatg     780
atctctagaa cccctgaggt gacatgtgtg gtggtggctg tgtctcatga ggaccctgag     840
gtcaaattca actggtacgt ggatggagtg gaagtccaca atgccaaaac caagcctaga     900
gaggaacagt acgcttcaac ctacagagtt gtcagtgtgc tgactgtgct gcatcaggat     960
tggctgaatg gcaaggaata caagtgtaaa gtctcaaaca aggccctggc tgctccaatt    1020
gagaaaacaa tctcaaaggc caagggacag cctagggaac cccaggtcta caccctgcca    1080
ccttcaagag aggaaatgac caaaaaccag gtgtccctga catgcctggt caaaggcttc    1140
tacccttctg acattgctgt ggagtgggag tcaaatggac agcctgagaa caactacaaa    1200
acaacccccc ctgtgctgga ttctgatggc tctttctttc tgtactccaa actgactgtg    1260
gacaagtcta gatggcagca ggggaatgtc ttttcttgct ctgtcatgca tgaggctctg    1320
cataaccact acactcagaa atccctgtct ctgtctcccg ggaaatga                1368
```

<210> 14
<211> 455
<212> PRT
<213> Artificial Sequence

<220>
<221> source
<223> /note="Description of Artificial Sequence: Synthetic polypeptide"

<400> 14

```
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Ser Leu Lys Thr Glu Asp Thr Ala Val Tyr
                85                  90                  95

Tyr Cys Val Arg His Gly Asn Phe Gly Asp Ser Tyr Val Ser Trp Phe
            100                 105                 110

Ala Tyr Trp Gly Gln Gly Thr Leu Val Thr Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn His Lys Pro Ser Asn Thr Lys Val Asp Lys Lys Val Glu
    210                 215                 220

Pro Lys Ser Cys Asp Lys Thr His Thr Cys Pro Pro Cys Pro Ala Pro
225                 230                 235                 240

Glu Leu Leu Gly Gly Pro Ser Val Phe Leu Phe Pro Pro Lys Pro Lys
```

```
                245                 250                 255

Asp Thr Leu Met Ile Ser Arg Thr Pro Glu Val Thr Cys Val Val Val
            260                 265                 270

Ala Val Ser His Glu Asp Pro Glu Val Lys Phe Asn Trp Tyr Val Asp
        275                 280                 285

Gly Val Glu Val His Asn Ala Lys Thr Lys Pro Arg Glu Glu Gln Tyr
    290                 295                 300

Ala Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Ala Ala Pro Ile Glu Lys Thr Ile Ser Lys Ala Lys Gly Gln Pro Arg
            340                 345                 350

Glu Pro Gln Val Tyr Thr Leu Pro Pro Ser Arg Glu Glu Met Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
        435                 440                 445

Leu Ser Leu Ser Pro Gly Lys
    450                 455
```

<210> 15
<211> 648
<212> DNA
<213> Artificial Sequence

<220>
<221> source

<223> /note="Description of Artificial Sequence: Synthetic polynucleotide"

<400> 15

```
cagaccgttg tgactcagga accttcactc accgtatcac ctggtggaac agtcacactc      60
acttgtcgct cgtcgactgg ggctgttaca actagcaact atgccaactg ggtccaacaa     120
aaaccgggtc aggcaccccg tggtctaata ggtggtacca acaagcgcgc accaggtact     180
cctgccagat tctcaggctc cctgcttgga ggcaaggctg ccctcaccct ctcgggggta     240
cagccagagg atgaggcaga atattactgt gctctatggt acagcaacct ctgggtgttc     300
ggtggaggaa ccaaactgac tgtcctaggc cagcctaaag cggcgccatc cgtcaccctg     360
ttccctccct catccgagga actgcaggcc aataaggcta cactggtctg tctgattagc     420
gacttctacc ctggggccgt gactgtggct tggaaagccg attcttctcc cgtgaaagct     480
ggagtggaaa caaccacccc ctctaaacag agcaacaaca aatacgctgc ctcttcatac     540
ctgtccctga cccctgaaca gtggaaatct caccggtctt actcatgcca ggtgacacac     600
gagggatcaa ctgtggagaa aaccgtggct cctaccgaat gttcatga                  648
```

<210> 16
<211> 215
<212> PRT
<213> Artificial Sequence

<220>
<221> source
<223> /note="Description of Artificial Sequence: Synthetic polypeptide"

<400> 16

```
Gln Thr Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Gly Gln Pro
            100                 105                 110

Lys Ala Ala Pro Ser Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
    130                 135                 140

Gly Ala Val Thr Val Ala Trp Lys Ala Asp Ser Ser Pro Val Lys Ala
145                 150                 155                 160

Gly Val Glu Thr Thr Thr Pro Ser Lys Gln Ser Asn Asn Lys Tyr Ala
                165                 170                 175

Ala Ser Ser Tyr Leu Ser Leu Thr Pro Glu Gln Trp Lys Ser His Arg
            180                 185                 190

Ser Tyr Ser Cys Gln Val Thr His Glu Gly Ser Thr Val Glu Lys Thr
        195                 200                 205

Val Ala Pro Thr Glu Cys Ser
    210                 215
```

<210> 17
<211> 1356
<212> DNA
<213> Artificial Sequence

<220>

<221> source
<223> /note="Description of Artificial Sequence: Synthetic polynucleotide"

<400> 17

caggtccagc tgcagcagag tggggccgaa ctggcaagac ccggagcaag cgtcaaaatg 60
tcatgtaaag caagcggtta tactttcact aggagcacca tgcactgggt gaaacagagg 120
cccggccagg gactggagtg gatcgggtac attaaccctt ccagcgctta caccaactat 180
aatcagaagt tcaaagacaa ggccaccctg acagctgata agtctagttc aacagcatat 240
atgcagctgt ccagcctgac ttctgaagac agtgcagtgt actattgcgc ctccccacag 300
gtccactacg attacaatgg ttttccttac tggggggcagg gcacactggt gactgtctcc 360
gccgctagca caaagggccc tagtgtgttt cctctggctc cctcttccaa atccacttct 420
ggtggcactg ctgctctggg atgcctggtg aaggattact ttcctgaacc tgtgactgtc 480
tcatggaact ctggtgctct gacttctggt gtccacactt tccctgctgt gctgcagtct 540
agtggactgt actctctgtc atctgtggtc actgtgccct cttcatctct gggaacccag 600
acctacattt gtaatgtgaa ccacaaacca tccaacacta aagtggacaa aaaagtggaa 660
cccaaatcct gtgacaaaac ccacacctgc ccaccttgtc ctgcccctga actgctggga 720
ggaccttctg tgtttctgtt cccccccaaa ccaaaggata ccctgatgat ctctagaacc 780
cctgaggtga catgtgtggt ggtgaatgtg tctcatgagg accctgaggt caaattcaac 840
tggtacgtgg atggagtgga agtccacaat gccaaaacca agcctagaga ggaacagtac 900
gattcaacct acagagttgt cagtgtgctg actgtgctgc atcaggattg gctgaatggc 960
aaggaataca agtgtaaagt ctcaaacaag gccctgggtg ctccaattga gaaaacaatc 1020
tcaaaggcca agggacagcc tagggaaccc caggtctaca ccctgccacc ttcaagagag 1080
gaaatgacca aaaaccaggt gtccctgaca tgcctggtca aaggcttcta cccttctgac 1140
attgctgtgg agtgggagtc aaatggacag cctgagaaca actacaaaac aacccccccct 1200
gtgctggatt ctgatggctc tttctttctg tactccaaac tgactgtgga caagtctaga 1260
tggcagcagg ggaatgtctt ttcttgctct gtcatgcatg aggctctgca taaccactac 1320
actcagaaat ccctgtctct gtctcccggg aaatga 1356

<210> 18
<211> 451
<212> PRT
<213> Artificial Sequence

<220>
<221> source
<223> /note="Description of Artificial Sequence: Synthetic polypeptide"

<400> 18

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110
```

```
Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asn Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asp Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365
```

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 19
<211> 1356
<212> DNA
<213> Artificial Sequence

<220>
<221> source
<223> /note="Description of Artificial Sequence: Synthetic polynucleotide"

<400> 19

caggtccagc tgcagcagag tggggccgaa ctggcaagac ccggagcaag cgtcaaaatg 60

tcatgtaaag caagcggtta tactttcact aggagcacca tgcactgggt gaaacagagg 120

cccggccagg gactggagtg gatcgggtac attaaccctt ccagcgctta caccaactat 180

aatcagaagt tcaaagacaa ggccaccctg acagctgata agtctagttc aacagcatat 240

atgcagctgt ccagcctgac ttctgaagac agtgcagtgt actattgcgc ctccccacag 300

gtccactacg attacaatgg ttttccttac tgggggcagg gcacactggt gactgtctcc 360

gccgctagca caaagggccc tagtgtgttt cctctggctc cctcttccaa atccacttct 420

ggtggcactg ctgctctggg atgcctggtg aaggattact ttcctgaacc tgtgactgtc 480

tcatggaact ctggtgctct gacttctggt gtccacactt tccctgctgt gctgcagtct 540

agtggactgt actctctgtc atctgtggtc actgtgccct cttcatctct gggaacccag 600

acctacattt gtaatgtgaa ccacaaacca tccaacacta aagtggacaa aaaagtggaa 660

cccaaatcct gtgacaaaac ccacacctgc ccaccttgtc ctgcccctga actgctggga 720

ggaccttctg tgtttctgtt ccccccaaa ccaaaggata ccctgatgat ctctagaacc 780

cctgaggtga catgtgtggt ggtggaggtg tctcatgagg accctgaggt caaattcaac 840

tggtacgtgg atggagtgga agtccacaat gccaaaacca agcctagaga ggaacagtac 900

caatcaacct acagagttgt cagtgtgctg actgtgctgc atcaggattg gctgaatggc 960

aaggaataca agtgtaaagt ctcaaacaag gccctgtctg ctccaattga gaaaacaatc 1020

tcaaaggcca agggacagcc tagggaaccc caggtctaca ccctgccacc ttcaagagag 1080

gaaatgacca aaaaccaggt gtccctgaca tgcctggtca aaggcttcta cccttctgac 1140

attgctgtgg agtgggagtc aaatggacag cctgagaaca actacaaaac aacccccct 1200

gtgctggatt ctgatggctc tttctttctg tactccaaac tgactgtgga caagtctaga 1260

tggcagcagg ggaatgtctt ttcttgctct gtcatgcatg aggctctgca taaccactac 1320

actcagaaat ccctgtctct gtctcccggg aaatga 1356

<210> 20
<211> 451
<212> PRT
<213> Artificial Sequence

<220>
<221> source
<223> /note="Description of Artificial Sequence: Synthetic polypeptide"

<400> 20

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110

Gln Gly Thr Leu Val Thr Val Ser Ala Ala Ser Thr Lys Gly Pro Ser
        115                 120                 125
```

```
Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Glu Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Gln Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Ser Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380
```

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 21
<211> 885
<212> DNA
<213> Artificial Sequence

<220>
<221> source
<223> /note="Description of Artificial Sequence: Synthetic polynucleotide"

<400> 21

cagaccgttg tgactcagga accttcactc accgtatcac ctggtggaac agtcacactc 60

acttgtcgct cgtcgactgg ggctgttaca actagcaact atgccaactg ggtccaacaa 120

aaaccgggtc aggcaccccg tggtctaata ggtggtacca acaagcgcgc accaggtact 180

cctgccagat tctcaggctc cctgcttgga ggcaaggctg ccctcaccct ctcgggggta 240

cagccagagg atgaggcaga atattactgt gctctatggt acagcaacct ctgggtgttc 300

ggtggaggaa ccaaactgac tgtcctaggc cagcctaaag cggcgccatc cgtcaccctg 360

ttccctccct catccgagga actgcaggcc aataaggcta cactggtctg tctgattagc 420

gacttctacc ctggggccgt gactgtggct tggaaagccg attcttctcc cgtgaaagct 480

ggagtggaaa caaccacccc ctctaaacag agcaacaaca aatacgctgc ctcttcatac 540

ctgtccctga cccctgaaca gtggaaatct caccggtctt actcatgcca ggtgacacac 600

gagggatcaa ctgtggagaa aaccgtggct cctaccgaat gttcaggcgg tggaggatcc 660

gggggtgggg gaagcggcgg aggaggtagc ggcgtgactc tgttcgtcgc tctgtacgac 720

tatgaggccc tgggggctca cgaactgtcc ttccataagg gcgagaaatt tcagatcctg 780

tcccccagga gcgagggacc tttttgggaa gcacactctc tgaccacagg cgaaaccgga 840

tggattccct ctaactacgt ggcccccgtc gatagtattc agtga 885

<210> 22
<211> 294
<212> PRT
<213> Artificial Sequence

<220>
<221> source
<223> /note="Description of Artificial Sequence: Synthetic polypeptide"

<400> 22

```
Gln Thr Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Gly Gln Pro
            100                 105                 110

Lys Ala Ala Pro Ser Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
    130                 135                 140

Gly Ala Val Thr Val Ala Trp Lys Ala Asp Ser Ser Pro Val Lys Ala
145                 150                 155                 160

Gly Val Glu Thr Thr Thr Pro Ser Lys Gln Ser Asn Asn Lys Tyr Ala
                165                 170                 175

Ala Ser Ser Tyr Leu Ser Leu Thr Pro Glu Gln Trp Lys Ser His Arg
            180                 185                 190

Ser Tyr Ser Cys Gln Val Thr His Glu Gly Ser Thr Val Glu Lys Thr
        195                 200                 205
```

```
Val Ala Pro Thr Glu Cys Ser Gly Gly Gly Gly Ser Gly Gly Gly Gly
    210                 215                 220

Ser Gly Gly Gly Gly Ser Gly Val Thr Leu Phe Val Ala Leu Tyr Asp
225                 230                 235                 240

Tyr Glu Ala Leu Gly Ala His Glu Leu Ser Phe His Lys Gly Glu Lys
                245                 250                 255

Phe Gln Ile Leu Ser Pro Arg Ser Glu Gly Pro Phe Trp Glu Ala His
            260                 265                 270

Ser Leu Thr Thr Gly Glu Thr Gly Trp Ile Pro Ser Asn Tyr Val Ala
        275                 280                 285

Pro Val Asp Ser Ile Gln
    290
```

<210> 23
<211> 885
<212> DNA
<213> Artificial Sequence

<220>
<221> source
<223> /note="Description of Artificial Sequence: Synthetic polynucleotide"

<400> 23

cagaccgttg tgactcagga accttcactc accgtatcac ctggtggaac agtcacactc 60

acttgtcgct cgtcgactgg ggctgttaca actagcaact atgccaactg ggtccaacaa 120

aaaccgggtc aggcaccccg tggtctaata ggtggtacca acaagcgcgc accaggtact 180

cctgccagat tctcaggctc cctgcttgga ggcaaggctg ccctcaccct ctcgggggta 240

cagccagagg atgaggcaga atattactgt gctctatggt acagcaacct ctgggtgttc 300

ggtggaggaa ccaaactgac tgtcctaggc cagcctaaag cggcgccatc cgtcaccctg 360

ttccctccct catccgagga actgcaggcc aataaggcta cactggtctg tctgattagc 420

gacttctacc ctggggccgt gactgtggct tggaaagccg attcttctcc cgtgaaagct 480

ggagtggaaa caaccacccc ctctaaacag agcaacaaca aatacgctgc ctcttcatac 540

ctgtccctga cccctgaaca gtggaaatct caccggtctt actcatgcca ggtgacacac 600

gagggatcaa ctgtggagaa aaccgtggct cctaccgaat gttcaggcgg tggaggatcc 660

gggggtgggg gaagcggcgg aggaggtagc ggcgtgactc tgttcgtcgc tctgtacgac 720

tatgaggccc tgggggctca cgaactgtcc ttccataagg gcgagaaatt tcagatcctg 780

tccagcctgg cagtgggacc attttgggag gcccactctc tgaccacagg cgaaaccgga 840

tggattccct ctaactacgt ggcacctgtc gatagtattc agtga 885

<210> 24
<211> 294
<212> PRT
<213> Artificial Sequence

<220>
<221> source
<223> /note="Description of Artificial Sequence: Synthetic polypeptide"

<400> 24

```
Gln Thr Val Val Thr Gln Glu Pro Ser Leu Thr Val Ser Pro Gly Gly
1               5                   10                  15

Thr Val Thr Leu Thr Cys Arg Ser Ser Thr Gly Ala Val Thr Thr Ser
            20                  25                  30

Asn Tyr Ala Asn Trp Val Gln Gln Lys Pro Gly Gln Ala Pro Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu Gly Gln Pro
            100                 105                 110

Lys Ala Ala Pro Ser Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
    130                 135                 140

Gly Ala Val Thr Val Ala Trp Lys Ala Asp Ser Ser Pro Val Lys Ala
145                 150                 155                 160

Gly Val Glu Thr Thr Thr Pro Ser Lys Gln Ser Asn Asn Lys Tyr Ala
                165                 170                 175

Ala Ser Ser Tyr Leu Ser Leu Thr Pro Glu Gln Trp Lys Ser His Arg
            180                 185                 190
```

```
Ser Tyr Ser Cys Gln Val Thr His Glu Gly Ser Thr Val Glu Lys Thr
        195                 200                 205

Val Ala Pro Thr Glu Cys Ser Gly Gly Gly Gly Ser Gly Gly Gly Gly
    210                 215                 220

Ser Gly Gly Gly Gly Ser Gly Val Thr Leu Phe Val Ala Leu Tyr Asp
225                 230                 235                 240

Tyr Glu Ala Leu Gly Ala His Glu Leu Ser Phe His Lys Gly Glu Lys
                245                 250                 255

Phe Gln Ile Leu Ser Ser Leu Ala Val Gly Pro Phe Trp Glu Ala His
            260                 265                 270

Ser Leu Thr Thr Gly Glu Thr Gly Trp Ile Pro Ser Asn Tyr Val Ala
        275                 280                 285

Pro Val Asp Ser Ile Gln
    290
```

<210> 25
<211> 1350
<212> DNA
<213> Artificial Sequence

<220>
<221> source
<223> /note="Description of Artificial Sequence: Synthetic polynucleotide"

<400> 25

caggtgcagc tggtgcagtc tggcggcgga gtggtgcagc ctggaagatc cctgcggctg 60
tcctgcaagg cctccggcta caccttcacc cggtacacca tgcactgggt gcgacaggcc 120
cctggcaagg gcctggaatg gatcggctac atcaacccct cccggggcta caccaactac 180
aaccagaaag tgaaggaccg gttcaccatc tcccgggaca actccaagaa caccgccttt 240
ctgcagatgg acagcctgcg gcctgaggat accggcgtgt acttctgcgc ccggtactac 300
gacgaccact actgcctgga ctactggggc cagggcaccc ctgtgacagt gtcctctgct 360
agcacaaagg gccctagtgt gtttcctctg gctccctctt ccaaatccac ttctggtggc 420
actgctgctc tgggatgcct ggtgaaggat tactttcctg aacctgtgac tgtctcatgg 480
aactctggtg ctctgacttc tggtgtccac actttccctg ctgtgctgca gtctagtgga 540
ctgtactctc tgtcatctgt ggtcactgtg ccctcttcat ctctgggaac ccagacctac 600
atttgtaatg tgaaccacaa accatccaac actaaagtgg acaaaaaagt ggaacccaaa 660
tcctgtgaca aaacccacac ctgcccacct tgtcctgccc ctgaagccgc cggaggacct 720
tctgtgtttc tgttcccccc caaaccaaag gataccctga tgatctctag aacccctgag 780
gtgacatgtg tggtggtgga tgtgtctcat gaggaccctg aggtcaaatt caactggtac 840
gtggatggag tggaagtcca caatgccaaa accaagccta gagaggaaca gtacaattca 900
acctacagag tggtcagtgt gctgactgtg ctgcatcagg attggctgaa tggcaaggaa 960
tacaagtgta aagtctcaaa caaggccctg cctgctccaa ttgagaaaac aatctcaaag 1020
gccaagggac agcctaggga accccaggtc tacaccctgc caccttcaag agaggaaatg 1080
accaaaaacc aggtgtccct gacatgcctg gtcaaaggct tctacccttc tgacattgct 1140
gtggagtggg agtcaaatgg acagcctgag aacaactaca aaacaacccc ccctgtgctg 1200
gattctgatg gctctttctt tctgtactcc aaactgactg tggacaagtc tagatggcag 1260
caggggaatg tcttttcttg ctctgtcatg catgaggctc tgcataacca ctacactcag 1320
aaatccctgt ctctgtctcc cgggaaatga 1350

<210> 26
<211> 449
<212> PRT
<213> Artificial Sequence

<220>
<221> source
<223> /note="Description of Artificial Sequence: Synthetic polypeptide"

<400> 26

```
Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
                85                  90                  95

Ala Arg Tyr Tyr Asp Asp His Tyr Cys Leu Asp Tyr Trp Gly Gln Gly
            100                 105                 110

Thr Pro Val Thr Val Ser Ser Ala Ser Thr Lys Gly Pro Ser Val Phe
```

115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Gln Thr Tyr Ile Cys Asn Val Asn His Lys Pro
195 200 205

Ser Asn Thr Lys Val Asp Lys Lys Val Glu Pro Lys Ser Cys Asp Lys
210 215 220

Thr His Thr Cys Pro Pro Cys Pro Ala Pro Glu Ala Ala Gly Gly Pro
225 230 235 240

Ser Val Phe Leu Phe Pro Pro Lys Pro Lys Asp Thr Leu Met Ile Ser
245 250 255

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Glu Glu Met Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 27
<211> 876
<212> DNA
<213> Artificial Sequence

<220>
<221> source
<223> /note="Description of Artificial Sequence: Synthetic polynucleotide"

<400> 27

gacatccaga tgacccagtc cccctccagc ctgtctgcct ctgtgggcga cagagtgaca 60

attacctgct ccgcctcctc ctccgtgtcc tacatgaact ggtatcagca gacccccggc 120

aaggccccca agcggtggat ctacgacacc tccaagctgg cctctggcgt gccctccaga 180

ttctccggct ctggctctgg caccgactat accttcacca tcagctccct gcagcccgag 240

gatatcgcca cctactactg ccagcagtgg tcctccaacc ccttcacctt tggccagggc 300

accaagctgc agatcacccg tacggtcgcg gcgccttctg tgttcatttt cccccccatct 360

gatgaacagc tgaaatctgg cactgcttct gtggtctgtc tgctgaacaa cttctaccct 420

agagaggcca aagtccagtg gaaagtggac aatgctctgc agagtgggaa ttcccaggaa 480

tctgtcactg agcaggactc taaggatagc acatactccc tgtcctctac tctgacactg 540

agcaaggctg attacgagaa acacaaagtg tacgcctgtg aagtcacaca tcaggggctg 600

tctagtcctg tgaccaaatc cttcaatagg ggagagtgcg gcggtggagg atccgggggt 660

gggggaagcg gcggaggagg tagcggcgtg accctgtttg tggccctgta cgactacgag 720

gccctgggcg ctcacgagct gtctttccac aagggcgaga agttccagat cctgaactcc 780

tccgagggcc ccttctggga ggctcactct ctgacaaccg gcgagacagg ctggattccc 840

tccaactatg tggcccccgt ggactccatc cagtga 876

<210> 28
<211> 291
<212> PRT
<213> Artificial Sequence

<220>
<221> source
<223> /note="Description of Artificial Sequence: Synthetic polypeptide"

<400> 28

```
Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

Asn Trp Tyr Gln Gln Thr Pro Gly Lys Ala Pro Lys Arg Trp Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Tyr Thr Phe Thr Ile Ser Ser Leu Gln Pro Glu
65                  70                  75                  80

Asp Ile Ala Thr Tyr Tyr Cys Gln Gln Trp Ser Ser Asn Pro Phe Thr
                85                  90                  95

Phe Gly Gln Gly Thr Lys Leu Gln Ile Thr Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
```

```
        195                 200                 205

Asn Arg Gly Glu Cys Gly Gly Gly Gly Ser Gly Gly Gly Gly Ser Gly
    210                 215                 220

Gly Gly Gly Ser Gly Val Thr Leu Phe Val Ala Leu Tyr Asp Tyr Glu
225                 230                 235                 240

Ala Leu Gly Ala His Glu Leu Ser Phe His Lys Gly Glu Lys Phe Gln
                245                 250                 255

Ile Leu Asn Ser Ser Glu Gly Pro Phe Trp Glu Ala His Ser Leu Thr
            260                 265                 270

Thr Gly Glu Thr Gly Trp Ile Pro Ser Asn Tyr Val Ala Pro Val Asp
        275                 280                 285

Ser Ile Gln
    290
```

<210> 29
<211> 1353
<212> DNA
<213> Artificial Sequence

<220>
<221> source
<223> /note="Description of Artificial Sequence: Synthetic polynucleotide"

<400> 29

```
cagatcaccc tgaaggagtc cgggcccaca ctggtgaaac ctactcagac cctgacactg        60
acttgcacct tctccggttt ttctctgagt acctcgggca tgggagtgag ctggatcagg       120
cagccccctg gcaaggcact ggaatggctg gcccacatct actgggacga tgacaagagg       180
tacaaccctt cactgaaatc ccggctgaca attactaagg ataccagcaa aaaccaggtg       240
gtcctgacca tgacaaatat ggaccccgtg gacactgcta cctactattg tgcaagactg       300
tacggcttca cctatggatt tgcttactgg gggcagggca ccctggtcac agtctcgagc       360
gctagcacaa agggccctag tgtgtttcct ctggctccct cttccaaatc cacttctggt       420
ggcactgctg ctctgggatg cctggtgaag gattactttc ctgaacctgt gactgtctca       480
tggaactctg gtgctctgac ttctggtgtc cacactttcc ctgctgtgct gcagtctagt       540
ggactgtact ctctgtcatc tgtggtcact gtgccctctt catctctggg aacccagacc       600
tacatttgta atgtgaacca caaaccatcc aacactaaag tggacaaaaa agtggaaccc       660
aaatcctgtg acaaaaccca cacctgccca ccttgtcctg cccctgaact gctgggagga       720
ccttctgtgt ttctgttccc ccccaaacca aaggataccc tgatgatctc tagaacccct       780
gaggtgacat gtgtggtggt ggatgtgtct catgaggacc ctgaggtcaa attcaactgg       840
tacgtggatg gagtggaagt ccacaatgcc aaaaccaagc ctagagagga acagtacaat       900
tcaacctaca gagttgtcag tgtgctgact gtgctgcatc aggattggct gaatggcaag       960
gaatacaagt gtaaagtctc aaacaaggcc ctgcctgctc caattgagaa aacaatctca      1020
aaggccaagg gacagcctag ggaaccccag gtctacaccc tgccaccttc aagagaggaa      1080
atgaccaaaa accaggtgtc cctgacatgc ctggtcaaag gcttctaccc ttctgacatt      1140
gctgtggagt gggagtcaaa tggacagcct gagaacaact acaaaacaac cccccctgtg      1200
ctggattctg atggctcttt ctttctgtac tccaaactga ctgtggacaa gtctagatgg      1260
cagcagggga atgtcttttc ttgctctgtc atgcatgagg ctctgcataa ccactacact      1320
cagaaatccc tgtctctgtc tcccgggaaa tga                                   1353
```

<210> 30
<211> 450
<212> PRT
<213> Artificial Sequence

<220>
<221> source
<223> /note="Description of Artificial Sequence: Synthetic polypeptide"

<400> 30

```
Gln Ile Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Ser Trp Ile Arg Gln Pro Pro Gly Lys Ala Leu Glu
        35                  40                  45

Trp Leu Ala His Ile Tyr Trp Asp Asp Asp Lys Arg Tyr Asn Pro Ser
    50                  55                  60

Leu Lys Ser Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Leu Tyr Gly Phe Thr Tyr Gly Phe Ala Tyr Trp Gly Gln
            100                 105                 110

Gly Thr Leu Val Thr Val Ser Ser Ala Ser Thr Lys Gly Pro Ser Val
        115                 120                 125
```

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp

370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 31
<211> 657
<212> DNA
<213> Artificial Sequence

<220>
<221> source
<223> /note="Description of Artificial Sequence: Synthetic polynucleotide"

<400> 31

gacatcgtga tgacacagag cccagattct ctggccgtca gcctgggcga aagggccact 60

atcaactgcc gggcctccca gtctgtggac tacaatggaa tttcttacat gcactggtat 120

cagcagaagc ctggccagcc ccctaaactg ctgatctatg ccgcttcaaa ccctgagtcc 180

ggcgtgccag accgattcag tggctcaggc tccgggaccg attttaccct gacaatttcc 240

agcctgcaag ctgaggacgt ggcagtctac tattgccagc agatcattga agatccctgg 300

acattcggtc agggcactaa ggtggagatc aaacgtacgg tcgcggcgcc ttctgtgttc 360

attttccccc catctgatga acagctgaaa tctggcactg cttctgtggt ctgtctgctg 420

aacaacttct accctagaga ggccaaagtc cagtggaaag tggacaatgc tctgcagagt 480

gggaattccc aggaatctgt cactgagcag gactctaagg atagcacata ctccctgtcc 540

tctactctga cactgagcaa ggctgattac gagaaacaca aagtgtacgc ctgtgaagtc 600

acacatcagg ggctgtctag tcctgtgacc aaatccttca ataggggaga gtgctga 657

<210> 32
<211> 218
<212> PRT
<213> Artificial Sequence

<220>
<221> source
<223> /note="Description of Artificial Sequence: Synthetic polypeptide"

<400> 32

```
Asp Ile Val Met Thr Gln Ser Pro Asp Ser Leu Ala Val Ser Leu Gly
1               5                   10                  15

Glu Arg Ala Thr Ile Asn Cys Arg Ala Ser Gln Ser Val Asp Tyr Asn
            20                  25                  30

Gly Ile Ser Tyr Met His Trp Tyr Gln Gln Lys Pro Gly Gln Pro Pro
        35                  40                  45

Lys Leu Leu Ile Tyr Ala Ala Ser Asn Pro Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Ile Ile
                85                  90                  95

Glu Asp Pro Trp Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 33
<211> 1353
<212> DNA
<213> Artificial Sequence

<220>
<221> source
<223> /note="Description of Artificial Sequence: Synthetic polynucleotide"

<400> 33

```
cagatcaccc tgaaggagtc cgggcccaca ctggtgaaac ctactcagac cctgacactg      60
acttgcacct tctccggttt ttctctgagt acctcgggca tgggagtgag ctggatcagg     120
cagccccctg gcaaggcact ggaatggctg gcccacatct actgggacga tgacaagagg     180
tacaaccctt cactgaaatc ccggctgaca attactaagg ataccagcaa aaaccaggtg     240
gtcctgacca tgacaaatat ggaccccgtg gacactgcta cctactattg tgcaagactg     300
tacggcttca cctatggatt tgcttactgg gggcagggca ccctggtcac agtctcgagc     360
gctagcacaa agggccctag tgtgtttcct ctggctccct cttccaaatc cacttctggt     420
ggcactgctg ctctgggatg cctggtgaag gattactttc ctgaacctgt gactgtctca     480
tggaactctg gtgctctgac ttctggtgtc cacactttcc ctgctgtgct gcagtctagt     540
ggactgtact ctctgtcatc tgtggtcact gtgccctctt catctctggg aacccagacc     600
tacatttgta atgtgaacca caaaccatcc aacactaaag tggacaaaaa agtggaaccc     660
aaatcctgtg acaaaaccca cacctgccca ccttgtcctg cccctgaagc cgccggagga     720
ccttctgtgt ttctgttccc cccaaaacca aaggataccc tgatgatctc tagaacccct     780
gaggtgacat gtgtggtggt ggatgtgtct catgaggacc ctgaggtcaa attcaactgg     840
tacgtggatg gagtggaagt ccacaatgcc aaaaccaagc ctagagagga acagtacaat     900
tcaacctaca gagtggtcag tgtgctgact gtgctgcatc aggattggct gaatggcaag     960
gaatacaagt gtaaagtctc aaacaaggcc ctgcctgctc caattgagaa aacaatctca    1020
aaggccaagg gacagcctag ggaaccccag gtctacaccc tgccaccttc aagagaggaa    1080
atgaccaaaa accaggtgtc cctgacatgc ctggtcaaag gcttctaccc ttctgacatt    1140
gctgtggagt gggagtcaaa tggacagcct gagaacaact acaaaacaac cccccctgtg    1200
ctggattctg atggctcttt ctttctgtac tccaaactga ctgtggacaa gtctagatgg    1260
cagcagggga atgtcttttc ttgctctgtc atgcatgagg ctctgcataa ccactacact    1320
cagaaatccc tgtctctgtc tcccgggaaa tga                                  1353
```

<210> 34
<211> 450
<212> PRT

<213> Artificial Sequence

<220>
<221> source
<223> /note="Description of Artificial Sequence: Synthetic polypeptide"

<400> 34

```
Gln Ile Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Ser Trp Ile Arg Gln Pro Pro Gly Lys Ala Leu Glu
        35                  40                  45

Trp Leu Ala His Ile Tyr Trp Asp Asp Asp Lys Arg Tyr Asn Pro Ser
    50                  55                  60

Leu Lys Ser Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Leu Tyr Gly Phe Thr Tyr Gly Phe Ala Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Ala Ala Gly Gly
225                 230                 235                 240
```

```
Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

<210> 35
<211> 195
<212> DNA
<213> Artificial Sequence

<220>

<221> source
<223> /note="Description of Artificial Sequence: Synthetic polynucleotide"

<400> 35

```
ggcgtgactc tgttcgtcgc tctgtacgac tatgaggccc tgggggctca cgaactgtcc      60
ttccataagg gcgagaaatt tcagatcctg tcccccagga gcgagggacc tttttgggaa     120
gcacactctc tgaccacagg cgaaaccgga tggattccct ctaactacgt ggcccccgtc     180
gatagtattc agtga                                                      195
```

<210> 36
<211> 64
<212> PRT
<213> Artificial Sequence

<220>
<221> source
<223> /note="Description of Artificial Sequence: Synthetic polypeptide"

<400> 36

```
Gly Val Thr Leu Phe Val Ala Leu Tyr Asp Tyr Glu Ala Leu Gly Ala
1               5                   10                  15

His Glu Leu Ser Phe His Lys Gly Glu Lys Phe Gln Ile Leu Ser Pro
            20                  25                  30

Arg Ser Glu Gly Pro Phe Trp Glu Ala His Ser Leu Thr Thr Gly Glu
        35                  40                  45

Thr Gly Trp Ile Pro Ser Asn Tyr Val Ala Pro Val Asp Ser Ile Gln
    50                  55                  60
```

<210> 37
<211> 195
<212> DNA
<213> Artificial Sequence

<220>
<221> source
<223> /note="Description of Artificial Sequence: Synthetic polynucleotide"

<400> 37

```
ggcgtgactc tgttcgtcgc tctgtacgac tatgaggccc tgggggctca cgaactgtcc      60
ttccataagg gcgagaaatt tcagatcctg tccagcctgg cagtgggacc attttgggag     120
gcccactctc tgaccacagg cgaaaccgga tggattccct ctaactacgt ggcacctgtc     180
gatagtattc agtga                                                      195
```

<210> 38
<211> 64
<212> PRT
<213> Artificial Sequence

<220>
<221> source
<223> /note="Description of Artificial Sequence: Synthetic polypeptide"

<400> 38

```
Gly Val Thr Leu Phe Val Ala Leu Tyr Asp Tyr Glu Ala Leu Gly Ala
1               5                   10                  15

His Glu Leu Ser Phe His Lys Gly Glu Lys Phe Gln Ile Leu Ser Ser
            20                  25                  30

Leu Ala Val Gly Pro Phe Trp Glu Ala His Ser Leu Thr Thr Gly Glu
        35                  40                  45

Thr Gly Trp Ile Pro Ser Asn Tyr Val Ala Pro Val Asp Ser Ile Gln
    50                  55                  60
```

<210> 39
<211> 45
<212> DNA
<213> Artificial Sequence

<220>
<221> source
<223> /note="Description of Artificial Sequence: Synthetic oligonucleotide"

<400> 39
ggcggtggag gatccggggg tgggggaagc ggcggaggag gtagc 45

<210> 40
<211> 15
<212> PRT
<213> Artificial Sequence

<220>
<221> source
<223> /note="Description of Artificial Sequence: Synthetic peptide"

<400> 40

```
Gly Gly Gly Gly Ser Gly Gly Gly Gly Ser Gly Gly Gly Gly Ser
1               5                   10                  15
```

<210> 41
<211> 57
<212> DNA
<213> Artificial Sequence

<220>
<221> source
<223> /note="Description of Artificial Sequence: Synthetic oligonucleotide"

<400> 41
atgaattttg gactgaggct gattttcctg gtgctgaccc tgaaaggcgt ccagtgt 57

<210> 42
<211> 19
<212> PRT
<213> Artificial Sequence

<220>
<221> source
<223> /note="Description of Artificial Sequence: Synthetic peptide"

<400> 42

```
Met Asn Phe Gly Leu Arg Leu Ile Phe Leu Val Leu Thr Leu Lys Gly
1               5                   10                  15

Val Gln Cys
```

<210> 43
<211> 222
<212> PRT
<213> Homo sapiens

<400> 43

```
Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Glu Glu Met Thr Lys Asn Gln Val Ser Leu Thr Cys Leu Val

    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly Lys
    210                 215                 220
```

<210> 44
<211> 1356
<212> DNA
<213> Artificial Sequence

<220>
<221> source
<223> /note="Description of Artificial Sequence: Synthetic polynucleotide"

<400> 44

```
caggtccagc tgcagcagag tggggccgaa ctggcaagac ccggagcaag cgtcaaaatg      60
tcatgtaaag caagcggtta tactttcact aggagcacca tgcactgggt gaaacagagg     120
cccggccagg gactggagtg gatcgggtac attaaccctt ccagcgctta caccaactat     180
aatcagaagt tcaaagacaa ggccaccctg acagctgata agtctagttc aacagcatat     240
atgcagctgt ccagcctgac ttctgaagac agtgcagtgt actattgcgc ctccccacag     300
gtccactacg attacaatgg ttttccttac tgggggcagg gcacactggt gactgtctcc     360
gccgctagca caaagggccc tagtgtgttt cctctggctc cctcttccaa atccacttct     420
ggtggcactg ctgctctggg atgcctggtg aaggattact ttcctgaacc tgtgactgtc     480
tcatggaact ctggtgctct gacttctggt gtccacactt tccctgctgt gctgcagtct     540
agtggactgt actctctgtc atctgtggtc actgtgccct cttcatctct gggaacccag     600
acctacattt gtaatgtgaa ccacaaacca tccaacacta aagtggacaa aaaagtggaa     660
cccaaatcct gtgacaaaac ccacacctgc ccaccttgtc ctgcccctga actgctggga     720
ggaccttctg tgtttctgtt cccccccaaa ccaaaggata ccctgatgat ctctagaacc     780
cctgaggtga catgtgtggt ggtggctgtg tctcatgagg accctgaggt caaattcaac     840
tggtacgtgg atggagtgga agtccacaat gccaaaacca agcctagaga ggaacagtac     900

gcttcaacct acagagttgt cagtgtgctg actgtgctgc atcaggattg gctgaatggc     960
aaggaataca agtgtaaagt ctcaaacaag gccctggctg ctccaattga gaaaacaatc    1020
tcaaaggcca agggacagcc tagggaaccc caggtctaca ccctgccacc ttcaagagag    1080
gaaatgacca aaaaccaggt gtccctgaca tgcctggtca aaggcttcta cccttctgac    1140
attgctgtgg agtgggagtc aaatggacag cctgagaaca actacaaaac aaccccccct    1200
gtgctggatt ctgatggctc tttctttctg tactccaaac tgactgtgga caagtctaga    1260
tggcagcagg ggaatgtctt ttcttgctct gtcatgcatg aggctctgca taaccactac    1320
actcagaaat ccctgtctct gtctcccggg aaatga                              1356
```

<210> 45
<211> 451
<212> PRT
<213> Artificial Sequence

<220>
<221> source

<223> /note="Description of Artificial Sequence: Synthetic polypeptide"

<400> 45

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140
```

```
Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Ala Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Ala Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Ala Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
```

385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 46
<211> 1356
<212> DNA
<213> Artificial Sequence

<220>
<221> source
<223> /note="Description of Artificial Sequence: Synthetic polynucleotide"

<400> 46

```
caggtccagc tgcagcagag tggggccgaa ctggcaagac ccggagcaag cgtcaaaatg      60
tcatgtaaag caagcggtta tactttcact aggagcacca tgcactgggt gaaacagagg     120
cccggccagg gactggagtg gatcgggtac attaaccctt ccagcgctta caccaactat     180
aatcagaagt tcaaagacaa ggccaccctg acagctgata agtctagttc aacagcatat     240
atgcagctgt ccagcctgac ttctgaagac agtgcagtgt actattgcgc ctccccacag     300
gtccactacg attacaatgg ttttccttac tgggggcagg gcacactggt gactgtctcc     360
gccgctagca caaagggccc tagtgtgttt cctctggctc cctcttccaa atccacttct     420
ggtggcactg ctgctctggg atgcctggtg aaggattact ttcctgaacc tgtgactgtc     480
tcatggaact ctggtgctct gacttctggt gtccacactt tccctgctgt gctgcagtct     540
agtggactgt actctctgtc atctgtggtc actgtgccct cttcatctct gggaacccag     600
acctacattt gtaatgtgaa ccacaaacca tccaacacta aagtggacaa aaaagtggaa     660
cccaaatcct gtgacaaaac ccacacctgc ccaccttgtc ctgcccctga actgctggga     720
ggaccttctg tgtttctgtt ccccccccaa ccaaaggata ccctgatgat ctctagaacc     780
cctgaggtga catgtgtggt ggtggctgtg tctcatgagg accctgaggt caaattcaac     840
tggtacgtgg atggagtgga agtccacaat gccaaaacca agcctagaga ggaacagtac     900
aattcaacct acagagttgt cagtgtgctg actgtgctgc atcaggattg gctgaatggc     960
aaggaataca agtgtaaagt ctcaaacaag gccctggctg ctccaattga gaaaacaatc    1020

tcaaaggcca agggacagcc tagggaaccc caggtctaca ccctgccacc ttcaagagag    1080
gaaatgacca aaaaccaggt gtccctgaca tgcctggtca aaggcttcta cccttctgac    1140
attgctgtgg agtgggagtc aaatggacag cctgagaaca actacaaaac aacccccccct    1200
gtgctggatt ctgatggctc tttctttctg tactccaaac tgactgtgga caagtctaga    1260
tggcagcagg ggaatgtctt ttcttgctct gtcatgcatg aggctctgca taaccactac    1320
actcagaaat ccctgtctct gtctcccggg aaatga                              1356
```

<210> 47
<211> 451
<212> PRT
<213> Artificial Sequence

<220>
<221> source
<223> /note="Description of Artificial Sequence: Synthetic polypeptide"

<400> 47

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160
```

```
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Ala Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Ala Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
```

405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 48
<211> 1356
<212> DNA
<213> Artificial Sequence

<220>
<221> source
<223> /note="Description of Artificial Sequence: Synthetic polynucleotide"

<400> 48

```
caggtccagc tgcagcagag tggggccgaa ctggcaagac ccggagcaag cgtcaaaatg      60
tcatgtaaag caagcggtta tactttcact aggagcacca tgcactgggt gaaacagagg     120
cccggccagg gactggagtg gatcgggtac attaaccctt ccagcgctta caccaactat     180
aatcagaagt tcaaagacaa ggccaccctg acagctgata agtctagttc aacagcatat     240
atgcagctgt ccagcctgac ttctgaagac agtgcagtgt actattgcgc ctccccacag     300
gtccactacg attacaatgg ttttccttac tggggggcagg gcacactggt gactgtctcc     360
gccgctagca caaagggccc tagtgtgttt cctctggctc cctcttccaa atccacttct     420
ggtggcactg ctgctctggg atgcctggtg aaggattact ttcctgaacc tgtgactgtc     480
tcatggaact ctggtgctct gacttctggt gtccacactt tccctgctgt gctgcagtct     540
agtggactgt actctctgtc atctgtggtc actgtgccct cttcatctct gggaacccag     600
acctacattt gtaatgtgaa ccacaaacca tccaacacta aagtggacaa aaaagtggaa     660
cccaaatcct gtgacaaaac ccacacctgc ccaccttgtc ctgcccctga actgctggga     720
ggaccttctg tgtttctgtt cccccccaaa ccaaaggata ccctgatgat ctctagaacc     780
cctgaggtga catgtgtggt ggtggatgtg tctcatgagg accctgaggt caaattcaac     840
tggtacgtgg atggagtgga agtccacaat gccaaaacca agcctagaga ggaacagtac     900
gcttcaacct acagagttgt cagtgtgctg actgtgctgc atcaggattg gctgaatggc     960
aaggaataca agtgtaaagt ctcaaacaag gccctgcctg ctccaattga gaaaacaatc    1020
tcaaaggcca agggacagcc tagggaaccc caggtctaca ccctgccacc ttcaagagag    1080
gaaatgacca aaaaccaggt gtccctgaca tgcctggtca aaggcttcta cccttctgac    1140
attgctgtgg agtgggagtc aaatggacag cctgagaaca actacaaaac aacccccccct    1200
gtgctggatt ctgatggctc tttctttctg tactccaaac tgactgtgga caagtctaga    1260
tggcagcagg ggaatgtctt ttcttgctct gtcatgcatg aggctctgca taaccactac    1320
actcagaaat ccctgtctct gtctcccggg aaatga                               1356
```

<210> 49
<211> 451
<212> PRT
<213> Artificial Sequence

<220>
<221> source
<223> /note="Description of Artificial Sequence: Synthetic polypeptide"

<400> 49

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175
```

```
Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Ala Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
```

420 425 430

His Glu Ala Leu His Asn His Tyr Thr Gln Lys Ser Leu Ser Leu Ser
435 440 445

Pro Gly Lys
450

<210> 50
<211> 1356
<212> DNA
<213> Artificial Sequence

<220>
<221> source
<223> /note="Description of Artificial Sequence: Synthetic polynucleotide"

<400> 50

caggtccagc tgcagcagag tggggccgaa ctggcaagac ccggagcaag cgtcaaaatg 60
tcatgtaaag caagcggtta tactttcact aggagcacca tgcactgggt gaaacagagg 120
cccggccagg gactggagtg gatcgggtac attaaccctt ccagcgctta caccaactat 180
aatcagaagt tcaaagacaa ggccaccctg acagctgata agtctagttc aacagcatat 240
atgcagctgt ccagcctgac ttctgaagac agtgcagtgt actattgcgc ctccccacag 300
gtccactacg attacaatgg ttttccttac tgggggcagg gcacactggt gactgtctcc 360
gccgctagca caaagggccc tagtgtgttt cctctggctc cctcttccaa atccacttct 420
ggtggcactg ctgctctggg atgcctggtg aaggattact ttcctgaacc tgtgactgtc 480
tcatggaact ctggtgctct gacttctggt gtccacactt tccctgctgt gctgcagtct 540
agtggactgt actctctgtc atctgtggtc actgtgccct cttcatctct gggaacccag 600
acctacattt gtaatgtgaa ccacaaacca tccaacacta aagtggacaa aaaagtggaa 660
cccaaatcct gtgacaaaac ccacacctgc ccaccttgtc ctgcccctga agccgccgga 720
ggaccttctg tgtttctgtt ccccccaaa ccaaaggata ccctgatgat ctctagaacc 780
cctgaggtga catgtgtggt ggtggatgtg tctcatgagg accctgaggt caaattcaac 840
tggtacgtgg atggagtgga agtccacaat gccaaaacca agcctagaga ggaacagtac 900
aattcaacct acagagtggt cagtgtgctg actgtgctgc atcaggattg gctgaatggc 960
aaggaataca agtgtaaagt ctcaaacaag gccctgcctg ctccaattga gaaaacaatc 1020
tcaaaggcca agggacagcc tagggaaccc caggtctaca ccctgccacc ttcaagagag 1080
gaaatgacca aaaaccaggt gtccctgaca tgcctggtca aaggcttcta cccttctgac 1140
attgctgtgg agtgggagtc aaatggacag cctgagaaca actacaaaac aacccccccct 1200
gtgctggatt ctgatggctc tttctttctg tactccaaac tgactgtgga caagtctaga 1260

tggcagcagg ggaatgtctt ttcttgctct gtcatgcatg aggctctgca taaccactac 1320
actcagaaat ccctgtctct gtctcccggg aaatga 1356

<210> 51
<211> 451
<212> PRT
<213> Artificial Sequence

<220>
<221> source
<223> /note="Description of Artificial Sequence: Synthetic polypeptide"

<400> 51

```
Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Ser
            20                  25                  30

Thr Met His Trp Val Lys Gln Arg Pro Gly Gln Gly Leu Glu Trp Ile
        35                  40                  45

Gly Tyr Ile Asn Pro Ser Ser Ala Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Ser Pro Gln Val His Tyr Asp Tyr Asn Gly Phe Pro Tyr Trp Gly
            100                 105                 110

Gln Gly Thr Leu Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190
```

```
Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
```

435 440 445

Pro Gly Lys
450

<210> 52
<211> 222
<212> PRT
<213> Homo sapiens

<400> 52

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Met Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly Lys
    210                 215                 220

<210> 53
<211> 222
<212> PRT
<213> Homo sapiens

<400> 53

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Glu Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly Lys
    210                 215                 220

<210> 54
<211> 222
<212> PRT
<213> Homo sapiens

<400> 54

```
Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Glu Glu Met Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Gly Leu His
        195                 200                 205

    Asn His Tyr Thr Gln Lys Ser Leu Ser Leu Ser Pro Gly Lys
        210                 215                 220
```

<210> 55
<211> 222
<212> PRT
<213> Homo sapiens

<400> 55

```
Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly Lys


          210                 215                 220
```

<210> 56
<211> 222
<212> PRT
<213> Homo sapiens

<400> 56

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Met Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Gly Leu His
        195                 200                 205

Asn His Tyr Thr Gln Lys Ser Leu Ser Leu Ser Pro Gly Lys
    210                 215                 220

<210> 57
<211> 222
<212> PRT
<213> Homo sapiens

<400> 57

```
Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Glu Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Gly Leu His
        195                 200                 205

Asn His Tyr Thr Gln Lys Ser Leu Ser Leu Ser Pro Gly Lys
    210                 215                 220
```

<210> 58
<211> 222
<212> PRT
<213> Homo sapiens

<400> 58

```
Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Gly Leu His
        195                 200                 205

Asn His Tyr Thr Gln Lys Ser Leu Ser Leu Ser Pro Gly Lys
    210                 215                 220
```

<210> 59
<211> 63
<212> PRT
<213> Artificial Sequence

<220>
<221> source
<223> /note="Description of Artificial Sequence: Synthetic polypeptide"

<400> 59

```
Gly Val Thr Leu Phe Val Ala Leu Tyr Asp Tyr Glu Ala Arg Thr Glu
1               5                   10                  15

Asp Asp Leu Ser Phe His Lys Gly Glu Lys Phe Gln Ile Leu Asn Ser
            20                  25                  30

Ser Glu Gly Asp Trp Trp Glu Ala Arg Ser Leu Thr Thr Gly Glu Thr
        35                  40                  45

Gly Tyr Ile Pro Ser Asn Tyr Val Ala Pro Val Asp Ser Ile Gln
    50                  55                  60
```

<210> 60
<211> 110
<212> PRT
<213> human

<400> 60

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110
```

<210> 61
<211> 107
<212> PRT
<213> human

<400> 61

```
Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105
```

## Description

### TECHNICAL FIELD

The disclosure provided herein relates to human antibody IgG1 constant regions (Fc regions) mutated such that they retain FcRn binding, but substantially lose the capacity to specifically bind Fcγ receptors and C1q.

### BACKGROUND

Immunoglobulins, which are glycoproteins present in the serum, tissue, or body fluid of every mammal, have the function of recognizing foreign antigens. The immunoglobulins participate through antibody binding to antigens in biophylaxis via the activation of the complement system or via the activation of effector functions such as enhancement in cellular phagocytosis, antibody-dependent cytotoxicity, and mediator release triggered by interactions with an Fc receptor (FcR) present on the effector cell surface.

Human immunoglobulins are divided into 5 different classes consisting of IgG, IgA, IgM, IgD, and IgE. IgG can further be classified into 4 subclasses consisting of IgG1, IgG2, IgG3, and IgG4, while IgA can further be classified into two subclasses consisting of IgA1 and IgA2. The basic structure of immunoglobulin comprises two homologous light chains (L chains) and 2 homologous heavy chains (H chains). The immunoglobulin classes and subclasses are determined depending on H chains.

Different types of immunoglobulins are known to have different functions. For example, complement-binding ability is high in IgM>IgG3>IgGl>IgG2 in this order, and affinity for Fcγ receptor I is high in IgG3>IgG1>IgG4>IgG2 in this order. Moreover, IgG1, IgG2, and IgG4 are capable of binding to Protein A.

Many monoclonal antibodies have undergone clinical trials in recent years and have been placed on the market for pharmaceutical applications. The majority of these monoclonal antibodies are derived from the IgG1 subclass.

Using IgG1 as the starting point, efforts have been made to generate Fc-containing molecules with diminished Fc receptor binding and effector functions but which retain FcRn binding, prolonged stability, and low immunogenicity. These types of molecules may provide improved antibody therapeutics, for example with a better safety profile.

IgG antibodies are bifunctional molecules in the sense that besides antigen-specific binding via their Fab arms, they are capable of engaging via their Fcγ domain with Fcγ receptors (FcγR) (Woof JM and DR Burton (2004). Nat Rev Immunol 4(2): 89-99). There are three types receptors for Fcγ, FcγRI - FcyRIII, with different affinities for IgG (Bruhns P, et al. (2009). Blood 113(16): 3716-3725). FcγR are expressed on various cell types which mediate Fcy-mediated immune effector functions such as antibody-dependent cell-mediated cytotoxicity (ADCC) or antibody dependent cellular phagocytosis (ADCP). The antibody Fcγ domain also binds to the complement factor C1q and thereby can activate the complement pathway, ultimately leading to complement-dependent cytotoxicity (CDC). Effector functions mediated by FcγR or C1q are believed to play a role in the activity of several therapeutic antibodies (Redman JM, et al. (2015). Mol Immunol 67(2 Pt A): 28-45.

In addition to inducing effects via FcγR expressing immune cells, engagement of FcγR forms higher order clusters of antibody which causes higher order clustering, or cross-linking, of cell-membrane antigens bound by the antibody Fab, thereby triggering downstream signaling (Stewart RH, et al. (2014). Journal of ImmunoTherapy of Cancer 2(29)). As an example, CD40-specific antibodies have been shown to activate CD40 downstream signaling in an FcγR dependent fashion (White AL, et al. (2011). J Immunol 187(4): 1754-1763).

While for many therapeutic antibody applications, it is desirable to have strong Fcγ mediated effector functions, certain applications rely on mode of actions that do not require effector functions or even necessitate inert antibodies that do not induce FcγR mediated effects. For this reason, IgG isotypes with reduced effector functions (e.g. IgG2 or IgG4) or engineered Fcγ sequences with mutations in the Fcγ-FcγR interface that reduce the affinity to FcγR are utilized in such antibodies. For example, anti-tumor antibodies that boost T cells by blocking T cell inhibitory receptors such as PD-1 or PD-L1 are preferentially inert, since a fully competent Fc region counteracts the antibody's mode of action by depletion of T cells via ADCC or CDC (Stewart RH, et al. (2014). Journal of ImmunoTherapy of Cancer 2(29)). Thus, there is a wide application for antibodies with reduced or abrogated binding to FcγR.

The field has found several solutions to the technical challenge of finding Fcγ with reduced affinity to FcγR by introduction of targeted mutations in the antibody Fcγ domain. Nose and Wigzell described that antibodies without N-linked carbohydrate at N297 did not bind to FcγR expressing cells and lacked ADCC activity (Nose M and H Wigzell (1983). Proc Natl Acad Sci U S A 80(21): 6632-6636). Tao and Morrison, and Bolt et al. performed site-directed mutagenesis at position N297, resulting in aglycosylated antibodies with reduced binding to FcγR and C1q (Tao MH and SL Morrison (1989). J Immunol 143(8): 2595-2601; Bolt S, et al. (1993). Eur J Immunol 23(2): 403-411). Several clinical-stage antibodies or Fcy-fusion proteins carry mutations at N297, for instance anti-PDL1 mAb atezolizumab, anti-GITR mAb TRX518, anti-CD3 mAb otelixizumab or the peptide-Fcy fusion protein romiplostim (Strohl WR (2009). Curr Opin Biotechnol 20(6): 685-691; Stewart RH, et al. (2014). Journal of ImmunoTherapy of Cancer 2(29)).

CD3 specific antibodies induce FcR-dependent T cell activation and cytokine release (Parren PW, et al. (1992). J Immunol 148(3): 695-701; Xu D, et al. (2000). Cell Immunol 200(1): 16-26). It was observed that a CD3-specific IgG1 antibody with a N297A mutation in the Fcγ still leads to T cell activation (WO2012143524), despite the fact the N297A has been described to have no detectable binding to FcγR expressing cells (Bolt S, et al. (1993). Eur J Immunol 23(2): 403-411). These observations suggest that in vitro T cell activation assays with CD3-specific antibodies are very sensitive to residual FcγR binding. Thus, in vitro T cell assays with CD3 antibodies represent an optimal functional assay to identify engineered Fcγ sequences with no or minimal binding affinity to FcγR.

Canfield and Morrison described that the hinge region of IgG contributes to binding to the high-affinity FcγRI (Canfield SM and SL Morrison (1991). J Exp Med 173(6): 1483-1491). Xu et al. demonstrated that the humanized anti-CD3 antibody hOKT3 containing the double mutations L234A and L235A in the lower hinge (also termed "Ala-Ala", or "LALA") demonstrated reduced C1q and FcγR binding, leading to dampened FcγR-mediated T cell activation and cytokine release *in vitro* (Xu D, et al. (2000). Cell Immunol 200(1): 16-26). This antibody, termed hOKT3γ1(Ala-Ala) or teplizumab, was subsequently investigated in clinical trials, where it was found that the introduction of the LALA mutations led to a reduced incidence of adverse cytokine release (Herold KC, et al. (2005). Diabetes 54(6): 1763-1769).

(Shields et al. (2001), J Biol Chem 276(9): 6591-6604) performed an alanine scanning mutagenesis approach on the entire antibody Fcγ to identify residues that contribute to FcγR binding. They found that mutation of D265 decreased the affinity to all Fcγ receptors. In addition, mutating position P329 was shown to reduce binding to Fcγ receptors. Idusogie et al. mapped the C1q binding site of rituximab, a chimeric IgG1 antibody, and found that mutations at D270, K322, P329 or P331 reduced binding to C1q (Idusogie EE, et al. (2000). J Immunol 164(8): 4178-4184). Wilson et al. described a combination of mutations at D265 and N297 to alanine, termed "DANA". These combined mutations were claimed to have reduced binding to FcγR, but residual binding to mouse FcγRIII was detected (Wilson NS, et al. (2011). Cancer Cell 19(1): 101-113). Gong et al. described that the "DANA" mutations exhibit partial reduction in complement activation (Gong Q, et al. (2005). J Immunol 174(2): 817-826).

Other reports described mutations of certain amino acids in the Fc part of antibodies (e.g. D265N and D265E: Shields RL, et al. (2001) J Biol Chem 276: 6591-6604; N297Q: Stavenhagen JB, et al. (2007) Cancer Res 67: 8882-8890; N297D: Sazinsky SL, et al. (2008) Proc Natl Acad Sci USA 105: 20167-20172, Kelton W, et al. (2014) Chem Biol 21: 1603-1609; P329G: Schlothauer T, et al. (2016) Protein Eng Des Sel 29: 457-466), although not all these studies related to impairment of Fc functionality.

Several Fcγ mutations were described that reduce binding to FcγR, but none of those mentioned above completely abrogate FcγR binding and C1q binding. Shields et al. have described the possibility to further reduce FcγR binding by combining individual mutations (Shields RL, et al. (2001). J Biol Chem 276(9): 6591-6604), a concept that is also underlying the "LALA" or "DANA" combination mutations described above.

However, the challenge remains to identify a combination of mutations that results in optimally reduced FcγR binding and importantly, that does not negatively impact other key properties that are of importance to a pharmaceutical product, such as manufacturability, pharmacokinetics, or antigenicity.

For example, WO 2014/108483 describes several Fcγ sequences containing combinations of mutations with reduced FcγR binding. The majority of the Fcγ-mutated antibodies had a faster clearance in mice than the corresponding unmodified IgG1 antibody. Therefore, introducing mutations in Fcγ domains is known to potentially have an impact on the pharmacokinetic properties.

The Fcγ domain also interacts with the neonatal Fc receptor, FcRn (Kuo TT and VG Aveson (2011). MAbs 3(5): 422-430). This interaction is responsible for antibody recycling, rescue from lysosomal degradation and thus for the long half-life of IgG1 antibodies. The FcRn binding site is located in the CH2-CH3 interface of Fcγ1 (Martin WL, et al. (2001). Mol Cell 7(4): 867-877). Therefore, novel engineered Fcγ domains with mutations in the CH2 domain can have impaired FcRn affinity and therefore impaired pharmacokinetic properties (Shields RL, et al. (2001). J Biol Chem 276(9): 6591-6604).

Thus, there is a need for novel engineered Fcγ sequences that have no or minimal binding affinity to FcyR and C1q but retain other properties of importance to a pharmaceutical product, such as pharmacokinetic profile or manufacturability.

## SUMMARY

The invention is set out in the appended set of claims.

Provided herein are compositions, comprising modified immunoglobulin constant domains useful in engineering of antibody or antibody-like therapeutics, such as those comprising an Fc region. Also described are related polynucleotides capable of encoding the provided modified constant domains, cells expressing the provided modified constant domains, as well as associated vectors. In addition, methods of using the provided modified constant domains are described.

The composition described is an IgG1 Fc mutant exhibiting diminished FcγR binding capacity but having conserved FcRn binding. These IgG Fc mutants enable therapeutic targeting of soluble or cell surface antigens while minimizing Fc-associated engagement of immune effector cells and complement mediated cytotoxicity. The IgG1 Fc-containing molecule comprises a CH2 domain in which amino acids at position 265, 297, and 329 indicated by the EU index as in Kabat, et al. are replaced by other amino acids.

In one embodiment, the amino acid at position 265 of the IgG1 Fc-containing molecule is replaced with alanine (A), asparagine (N) or glutamic acid (E), the amino acid at position 297 is replaced with alanine, aspartic acid (D), or glutamine (Q), and the amino acid at position 329 is replaced with alanine, glycine (G), or serine (S).

The IgG1 Fc mutant compositions can be used in indications where retention of therapeutic antibody (or Fc-fusion) half-life is conserved through interactions with FcRn, while undesired effects derived from binding and/or activation of C1q and FcγRs associated with immune cells and effector functions such as i) antibody dependent cytotoxicity (ADCC), ii) complement dependent cytotoxicity (CDC), iii) antibody dependent cellular phagocytosis (ADCP), iv) FcyR-mediated cellular activation, v) FcyR-mediated platelet activation/depletion, and/or vi) FcyR-mediated cross-linking of the bound target, are minimized or eliminated.

The IgG1 Fc mutations may be incorporated into therapeutic antibodies or Fc-fusions of binders, such as multivalent binders, targeting ligands on cells involved in cancer, neurological disorders, immune system disorders such as those related to B-cell or T-cell activation, or on cells involved in tissue repair or healing, such as fibroblasts or stem cells.

In certain embodiments, the IgG1 Fc mutant is comprised in a pharmaceutical composition. The IgG1 Fc mutant may be part of a pharmaceutically active molecule. The pharmaceutical compositions comprising the IgG1 Fc mutant or active IgG1 Fc mutant-comprising molecules are useful for the treatment of diseases or disorders, for example cancer.

Provided herein are recombinant IgG1 Fc-containing molecules having decreased affinity for C1q and to at least one Fcγ receptor (FcγR) as compared to an Fc-containing molecule with a wild type Fc domain, the recombinant IgG1 Fc-containing molecules comprising mutations at amino acid position 265, 297, and 329, wherein residue numbering is as indicated by the EU index as in Kabat, et al.

Provided herein are recombinant polypeptides comprising (a) one or more binding domains capable of binding at least one target molecule; and (b) an IgG1 Fc domain comprising mutations at amino acid position 265, 297, and 329, wherein the polypeptide is capable of binding the target molecule without triggering significant Fcy-mediated effects, such as complement dependent lysis, cell mediated destruction of the target molecule, and/or FcγR-mediated cross-linking of the bound target.

## BRIEF DESCRIPTION OF DRAWINGS

**Fig. 1:** Size exclusion chromatography profiles of mAb1 IgG1 and mAb1 DANAPA IgG1.

**Fig. 2**: Binding of anti-CD3 antibody mAb1 mutants with mutated Fc to $CD3^+$ Jurkat cells.

**Fig. 3A and 3B:** A) Induction of lymphocyte activation in human PBMC by mAb1 mutants with mutated Fcγ1 determined by CD69 surface staining. The dotted line represents the percentage CD69 positive cells obtained in the positive control wells containing CD2/CD3/CD28 activation beads. B) Induction of cytokine release in human PBMC by mAb1 mutants with mutated Fcγ1, as determined by IFNγ ELISA. The dotted line represents the level of IFNy obtained in the positive control wells containing CD2/CD3/CD28 activation beads.

**Fig. 4A-4D:** A) Schematic illustration of the AlphaScreen™ Fc receptor competition binding assay. B) Binding of Fc mutated mAb1 mutants to human FcγRI, FcyRIIA, FcyRIIB and FcγRIIIA, analyzed by AlphaScreen™ Fc receptor competition binding assay. C,D) Binding of Fc mutated mAb1 mutants to human FcγRI (C) and human FcγRIIIA (D), analyzed by surface plasmon resonance (BIAcore).

**Fig. 5:** Binding of different antibodies in DANAPA IgG1 format to human FcγRI, FcyRIIA, FcyRIIB and FcyRIIIA, analyzed by AlphaScreen™ Fc receptor competition binding assay.

**Fig. 6**: Binding of mAb1 with different substitutions at position D265, N297 and P329 to human FcγRI, analyzed by AlphaScreen™ Fc receptor competition binding assay.

**Fig. 7:** Binding of mAb1 DANAPA IgG1 to human C1q, analyzed by surface plasmon resonance (BIAcore).

**Fig. 8:** Binding of mAb1 DANAPA IgG1 to human FcRn at pH 6.0, analyzed by surface plasmon resonance (BIAcore).

**Fig. 9:** Antibody plasma concentrations in C57BL/6 mice after i.v. administration of 10 mg/kg mAb1 DANAPA IgG1 or mAb1 IgG1, respectively. Data is presented as mean ± standard deviation (n=5)

**Fig. 10:** Binding of different CD3/CD33 FynomAbs to human FcγRI, FcγRIIA, FcyRIIB and FcyRIIIA, analyzed by AlphaScreen™ Fc receptor competition binding assay.

**DETAILED DESCRIPTION OF ILLUSTRATIVE EMBODIMENTS**

In order that the application may be more completely understood, several definitions are set forth below. Such definitions are meant to encompass grammatical equivalents.

Throughout the present specification and claims, the numbering of the residues in the Fc region is that of the immunoglobulin heavy chain according to the EU index as in Kabat et al., Sequences of Proteins of Immunological Interest, 5th Ed. Public Health Service, National Institutes of Health, Bethesda, Md. (1991). The "EU index as in Kabat" herein refers to the residue numbering of the human IgG1 EU antibody. This numbering is well known to the skilled person and often used in the field.

By "polypeptide" or "protein" as used herein is meant at least two covalently attached amino acids, which includes proteins, polypeptides, oligopeptides and peptides.

By "amino acid" as used herein is meant one of the 20 naturally occurring amino acids or any non-natural analogues that may be present at a specific, defined position.

An "Fc-containing molecule having a substitution (or 'mutation', or 'replacement') at positions 265, 297 and 329", means a molecule wherein the amino acid at position 265 is different from aspartic acid (D), the amino acid at position 297 is different from asparagine (N), and the amino acid at position 329 is different from proline (P), wherein all numbering in the Fc-region is according to the EU-index in Kabat et al.

"Amino acid changes", herein include amino acid mutations such as substitution, insertion, and/or deletion in a polypeptide sequence. By "amino acid substitution" or "substitution" herein is meant the replacement of an amino acid at a particular position in a parent polypeptide sequence with another amino acid. For example, the substitution P329A refers to a mutant polypeptide, in this case an Fc mutant, in which the proline at position 329 is replaced with alanine.

In case of a combination of amino acid mutations, the preferred format is the following: D265A/N297A/P329A. That means that there are three amino acid mutations in the Fc region of the mutant as compared to its parent polypeptide: one in position 265 (aspartic acid (D) replaced with alanine (A)), one in position 297 (asparagine (N) replaced with alanine), and one in position 329 (proline (P) replaced with alanine).

The term "antibody" is used herein in the broadest sense. "Antibody" refers to any polypeptide which at least comprises (i) an Fc region and (ii) a binding polypeptide domain derived from a variable region of an immunoglobulin. Antibodies thus include, but are not limited to, full-length immunoglobulins, multi-specific antibodies, Fc-fusion protein comprising at least one variable region, synthetic antibodies (sometimes referred to herein as "antibody mimetics"), chimeric antibodies, humanized antibodies, fully human antibodies, heterodimeric antibodies, antibody-fusion proteins, antibody conjugates and fragments of each respectively. A "FynomAb" as described in more detail below also comprises an antibody.

By "full-length antibody" or by "immunoglobulin" as used herein is meant the structure that constitutes the natural biological form of an antibody, including variable and constant regions. "Full length antibody" covers monoclonal full-length antibodies, wild-type full-length antibodies, chimeric full-length antibodies, humanized full-length antibodies, fully human full-length antibodies, the list not being limitative.

In most mammals, including humans and mice, the structure of full-length antibodies is generally a tetramer. Said tetramer is composed of two identical pairs of polypeptide chains, each pair having one "light" chain (typically having a molecular weight of about 25 kDa) and one "heavy" chain (typically having a molecular weight of about 50-70 kDa). In some mammals, for example in camels and llamas, full-length antibodies may consist of only two heavy chains, each heavy chain comprising a variable domain attached to the Fc region.

The amino-terminal portion of each chain includes a variable region of about 100 to 110 or more amino acids primarily responsible for antigen recognition and comprising the so-called complementarity-determining regions (CDR).

The carboxy-terminal portion of each chain defines a constant region normally primarily responsible for effector functions.

In the case of human immunoglobulins, light chains are classified as kappa and lambda light chains. Heavy chains are classified as mu, delta, gamma, alpha, or epsilon, and define the antibody's isotype as IgM, IgD, IgG, IgA, and IgE, respectively.

As used herein, "human" antibodies include antibodies having the amino acid sequence of a human immunoglobulin and includes antibodies isolated from human immunoglobulin libraries or from animals transgenic for one or more human immunoglobulin and that do not express endogenous immunoglobulins.

By "IgG" as used herein is meant a polypeptide belonging to the class of antibodies that are substantially encoded by a recognized immunoglobulin gamma gene. In humans, IgG comprises the subclasses or isotypes IgG1, IgG2, IgG3, and IgG4. In mice, IgG comprises IgG1, IgG2a, IgG2b, IgG3. Full-length IgGs consist of two identical pairs of two immunoglobulin chains, each pair having one light and one heavy chain, each light chain comprising immunoglobulin domains VL and CL, and each heavy chain comprising immunoglobulin domains VH, Cγ1 (also called CHI), Cγ2 (also called CH2), and Cγ3 (also called CH3). In the context of human IgG1, "CHI" refers to positions 118-215, CH2 domain refers to positions 231-340 and CH3 domain refers to positions 341-447 according to the EU index as in Kabat. IgG1 also comprises a hinge domain which refers to positions 216-230 in the case of IgG1.

By "Fc" or "Fc region", as used herein is meant the constant region of a full-length immunoglobulin excluding the first constant region immunoglobulin domain. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, Fc comprises immunoglobulin domains CH2, CH3 and the lower hinge region between CH1 and CH2. The Fc region of IgG1 comprises the domain from amino acid C226 to the carboxyl terminus end, wherein the numbering is according to the EU index as in Kabat. For example, the "Fc" or "Fc region" may include, without being limited to, Fc region of IgG1 comprising any one of the sequences SEQ ID NO: 43 and 52-58 (each of which are examples of human wild-type IgG1 Fc amino acid sequences), or comprising a sequence that is at least 80%, at least 85%, preferably at least 90%, at least 91%, at least 92%, at least 93%, at least 94%, more preferably at least 95%, at least 96%, at least 97%, at least 98%, or at least 99%, identical to SEQ ID NO: 43 or to any of SEQ ID NO: 52-58. In preferred embodiments, an Fc region according to the invention from position 226 (Kabat numbering) onwards comprises a sequence that is at least 80%, at least 85%, preferably at least 90%, at least 91%, at least 92%, at least 93%, at least 94%, more preferably at least 95%, at least 96%, at least 97%, or at least 98%, identical to SEQ ID NO: 43, and wherein the amino acid at position 265 is different from aspartic acid (D), the amino acid at position 297 is different from asparagine (N), and the amino acid at position 329 is different from proline (P). The analogous domains for other IgG sub-classes can be determined from amino acid sequence alignment of heavy chains or heavy chain fragments of said IgG sub-classes with that of human IgG1.

A "CH2 domain" as used herein is preferably of an Fc region of human IgG1, and comprises an amino acid sequence at least 80%, 85%, 90%, preferably at least 95%, at least 96%, at least 97%, at least 98%, at least 99%, or 100%, identical to SEQ ID NO: 60. A "CH3 domain" of an Fc region of human IgG1 as described herein comprises an amino acid sequence at least 80%, 85%, 90%, preferably at least 95%, at least 98%, or 100%, identical to SEQ ID NO: 61.

By "Fc-containing molecule" as used herein is meant a polypeptide that comprises an Fc region. Fc-containing molecules include, but are not limited to, antibodies, Fc fusions, isolated Fcs, Fc-conjugates, antibody fusions, FynomAbs, and the like.

By "wild-type" or "WT" herein is meant an amino acid sequence or a nucleotide sequence that is found in nature i.e. that is naturally-occurring, including allelic variations. A WT protein, polypeptide, antibody, immunoglobulin, IgG, etc. have an amino acid sequence or a nucleotide sequence that has not been intentionally modified by molecular biological techniques such as mutagenesis. For example, "wild-type Fc regions" may include, without being limited to, Fc region of IgG1 comprising the sequence SEQ ID NO: 43, which is an example of a human wild-type IgG1 Fc amino acid sequence, or Fc region of IgG comprising any one of the sequences of SEQ ID NOs: 52-58, each of which are also examples of human wild-type IgG1 Fc amino acid sequences.

The terms "Fc receptor" or "FcR" are used to describe a receptor that binds to an Fc region (e.g., the Fc region of an antibody).

The terms "Fc gamma receptor", "Fcγ receptor" or "FcγR" refer to human receptors which bind the Fc region of IgG antibodies. As used herein, FcγR includes FcγRI (CD64), FcγRII (CD32), FcγRIII (CD16) subclasses including their allelic mutants and alternatively spliced forms of these receptors.

These FcγRs are also defined as either activating receptors (FcyRI, FcyRIIa/c, FcyRIIIa/b) or inhibitory receptor (FcγRIIb) as they elicit or inhibit immune functions.

FcγRI family is composed of three genes (FCGRIA, FCGRIB and FCGRIC) but only the product of FCGRIA has been identified as full-length surface receptor. The said product, namely FcγRI, is expressed by dendritic cells (DC), macrophages and also activated neutrophils.

FcγRII family is composed of three genes (FCGR2A, FCGR2B and FCGR2C) which encode the FcyRIIa, FcyRIIb and FcyRIIc proteins. FcγRIIa is expressed on monocytes, certain dendritic cells and neutrophils. FcγRIIc is expressed on natural killer (NK) cells. FcγRIIb is the broadly expressed FcγR. FcγRIIb is virtually present on all leukocytes with exception of NK cells and T cells.

FcγRIII family is composed of two genes FCGR3A and FCGR3B which encode FcyRIIIa and FcγRIIIb. The FcγRIIIa protein is expressed as a transmembrane protein on monocytes, tissue specific macrophages, dendritic cells, γδ T cells, and natural killer cells. FcyRIIIb is a GPI-anchored receptor expressed on the surface of neutrophils and basophils.

Two alleles of the gene encoding FcγRIIa generate 2 mutants differing at position 131 (low-responder FcγRIIaR131 and high-responder FcγRIIaH131). Similarly, two alleles of the gene encoding FcyRIIIa generate 2 mutants differing at position 158 (low-responder FcγRIIIaF158 and high-responder FcγRIIIaV158).

Noticeably, NK cells, which are believed to be the crucial mediators of antibody-dependent cell-cytotoxicity, only express FcγRIIIa and FcγRIIc and none of the other FcγRs, in particular, the inhibitory FcyRIIb.

Each FcγR protein has differential ligand binding preferences with respect to IgG subclasses and distinct affinities for IgG subclasses.

Activating FcγRs trigger various immune responses such as phagocytosis, respiratory burst and cytokine production (TNF-α, IL-6) by antigen presenting cells (APC), antibody-dependent cellular cytotoxicity (ADCC) and degranulation by neutrophils and NK cells. Activating FcγRs also play an important role in the clearance of immune complex. On the other hand, the inhibitory receptor FcγRIIb is a critical regulatory element in B-cell homeostasis. It controls the threshold and the extent of cell activation.

Fc gamma receptors and their functions are reviewed in Nimmerjahn and Ravetch, Nature Reviews Immunology, 2008, 8, 34-47.

As used herein, "C1q" is a hexavalent molecule with a molecular weight of approximately 460,000 and a structure likened to a bouquet of tulips in which six collagenous "stalks" are connected to six globular head regions. C1q forms with the two serine proteases, C1r and C1s, the complex C1 which is the first component of the complement cascade pathway.

C1q and its function are reviewed e.g. in Kishore et al., Immunopharmacology, 2000, 49:159-170 and Sjöberg et al. Trends Immunol. 2009 30(2):83-90.

By "FcRn" or "neonatal Fc Receptor" as used herein is meant a protein that binds the IgG antibody Fc region and is encoded at least in part by an FCRN gene. As is known in the art, the functional FcRn protein comprises two polypeptides, often referred to as the heavy chain and light chain. The light chain is beta-2-microglobulin and the heavy chain is encoded by the FCRN gene. FcRn or FcRn protein refers to the complex of α-chain with beta-2-microglobulin. In human, the gene coding for FcRn is called FCGRT. FcRn is involved in the transfer of passive humoral immunity from a mother to her fetus and also in the control of the clearance of IgGs.

FcRn and its function is reviewed e.g. in Roopenian, Nature Reviews Immunology, 2007, 7, 715-725.

A molecule "retains binding to FcRn" as used herein when it binds to FcRn with a KD that is lower than 5-fold, preferably lower than 4-fold, more preferably lower than 3-fold, still more preferably lower than 2-fold the KD, of the parental Fc-containing molecule without the amino acid substitution (e.g. wild-type IgG1), as measured using surface plasmon resonance (SPR), wherein the KD is measured at pH 6.0. In certain embodiments the KD is about 1 to 2-fold, e.g. about 1.5-fold the KD of the parental molecule, or about the same as (i.e. 1-fold) the KD of the parental molecule, and in certain embodiments the KD can also be lower than the KD of the parental molecule.

"Reduced binding" refers to reduced binding of the Fc-containing molecules of the invention having at least one amino acid substitution in the Fc region described herein, for instance to C1q and/or to FcyR receptor when compared to the binding of the parental Fc-containing molecule without the amino acid substitution. "Reduced binding" may be at least 20-fold, at least about 50-fold, at least about 75-fold, or at least about 100-fold reduced binding. Binding of Fc-containing molecules can be assayed using a variety of techniques known in the art, including but not limited to surface plasmon resonance (SPR). SPR measurements can be performed using a BIAcore® instrument. In practice, Fc-containing molecules exhibiting "reduced binding" to a particular FcyR refer to Fc-containing molecules that have significantly reduced or abrogated effector function mediated by the particular FcyR.

"Recombinant" as used herein, includes antibodies and other proteins that are prepared, expressed, created or isolated by recombinant means.

"Vector" means a polynucleotide capable of being duplicated within a biological system or that can be moved between such systems. Vector polynucleotides typically contain elements, such as origins of replication, polyadenylation signal or selection markers, that function to facilitate the duplication or maintenance of these polynucleotides in a biological system. Examples of such biological systems may include a cell, virus, animal, plant, and reconstituted biological systems utilizing biological components capable of duplicating a vector. The polynucleotide comprising a vector may be DNA or RNA molecules or a hybrid of these.

"Polynucleotide" means a molecule comprising a chain of nucleotides covalently linked by a sugar-phosphate backbone or other equivalent covalent chemistry. Double and single-stranded DNA and RNA are typical examples of polynucleotides.

## FC MUTANTS WITH DECREASED BINDING TO C1Q AND FCγRS

The present disclosure is a demonstration for the first time of combined substitutions in positions 265, 297, and 329 of the IgG1 constant regions (Fc), according to the EU index as in Kabat. The directed selection of multiple residue substitutions unexpectedly provided a functional Fc domain for use in antibody engineering and for use as a fusion polypeptide as well as the possibility of providing a therapeutic entity which is devoid of measurable effector function.

The disclosure thus provides a recombinant IgG1 Fc-containing molecule, comprising a CH2 domain in which amino acid D at position 265, amino acid N at position 297, and amino acid P at position 329 indicated by the EU index as in Kabat are replaced by other amino acids.

Preferred IgG1 Fc-containing molecules include those comprising an amino acid substitution at position 265, 297 and 329. As discussed below, such polypeptides may have one or more additional deletions, additions, or substitutions within the Fc region. Thus, disclosed are IgG1 Fc-containing molecules having an amino acid substitution at at position 265 (i.e. having an amino acid different from D at this position), 297 (i.e. having an amino acid different from N at this position) and 329 (i.e. having an amino acid different from P at this position) and at the same time the Fc-regions from position 226 (Kabat numbering) onwards are at least 80%, at least 85%, preferably at least 90%, at least 91%, at least 92%, at least 93%, at least 94%, more preferably at least 95%, at least 96%, at least 97%, or at least 98% identical to SEQ ID NO: 43.

The term "percent (%) sequence identity" or "% identity" describes the number of matches ("hits") of identical amino acids of two or more aligned amino acid sequences as compared to the number of amino acid residues making up the overall length of the amino acid sequences. In other terms, using an alignment, for two or more sequences the percentage of amino acid residues that are the same (e.g. 90%, 95%, 97% or 98% identity) may be determined, when the sequences are compared and aligned for maximum correspondence as measured using a sequence comparison algorithm as known in the art, or when manually aligned and visually inspected. The sequences which are compared to determine sequence identity may thus differ by substitution(s), addition(s) or deletion(s) of amino acids. Suitable programs for aligning protein sequences are known to the skilled person. The percentage sequence identity of protein sequences can, for example, be determined with programs such as CLUSTALW, Clustal Omega, FASTA or BLAST, e.g using the NCBI BLAST algorithm (Altschul SF, et al (1997), Nucleic Acids Res. 25:3389-3402).

For example, for amino acid sequences, sequence identity and/or similarity can be determined by using standard techniques known in the art, including, but not limited to, the local sequence identity algorithm of Smith and Waterman, 1981, Adv. Appl. Math. 2:482, the sequence identity alignment algorithm of Needleman and Wunsch, 1970, J. Mol. Biol. 48:443, the search for similarity method of Pearson and Lipman, 1988, Proc. Nat. Acad. Sci. U.S.A. 85:2444, computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux et al, 1984, Nucl. Acid Res. 12:387-395, preferably using the default settings, or by inspection. In certain embodiments, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.

Another example of a useful algorithm is PILEUP. PILEUP creates a multiple sequence alignment from a group of related sequences using progressive, pairwise alignments. It can also plot a tree showing the clustering relationships used to create the alignment. Useful PILEUP parameters including a default gap weight of 3.00, a default gap length weight of 0.10, and weighted end gaps.

Another example of a useful algorithm is the BLAST algorithm, described in: Altschul et al, 1990, J. Mol. Biol. 215:403-410; Altschul et al, 1997, Nucleic Acids Res. 25:3389-3402; and Karin et al, 1993, Proc. Natl. Acad. Sci. U.S.A. 90:5873-5787. A particularly useful BLAST program is the WU-BLAST-2 program which was obtained from Altschul et al, 1996, Methods in Enzymology 266:460-480. WU-BLAST-2 uses several search parameters, most of which are set to the default values.

An additional useful algorithm is gapped BLAST as reported by Altschul et al, 1993, Nucl. Acids Res. 25:3389-3402.

The multi-substituted IgG1 mutants were selected on the basis of their relative affinities for human FcRs (FcyRI, FcγRIIa, FcγRIIb, FcγRIIIa and FcRn) assessed by AlphaScreen™ competition assays and SPR/Biacore analyses. These mutants were further tested and ranked in the appropriate cellular systems for their ability to induce cytokine release by PBMCs. In the set of experimental data provided herein, the IgG1 Fc mutants were compared to wild-type IgG1 Fc-containing molecules. Further analyses of these mutants in several *in vitro* bioassays demonstrated minimal to undetectable levels of activity and greatly ablated binding affinity for FcγRs. Based on these screens, IgG1 Fc mutants, comprising substitutions at all three amino acid positions 265, 297 and 329 combined, were surprisingly identified to have no or minimal detectable affinity for FcγRs and are virtually or completely devoid of activity in the various aforementioned effector/immunostimulatory bioassays. The IgG1 Fc mutants of the invention may be considered a truly "silenced" Fc in having no or minimal ability to bind FcγRs, mediate effector functions, or engage Fc-mediated cytokine release.

Based on the present invention, substitutions at amino acid positions 265, 297 and 329 can optionally be combined with other amino acid mutations, or the substitutions can be used in another IgG isotype to achieve similar or selective silencing of effector functions as taught herein and combined with what is known in the art. This combination of mutations at positions 265, 297 and 329 surprisingly led to significantly improved silencing compared to previously described Fc mutation N297A, or Fc double mutation L234A/L235A, each of which have been used in clinical-stage therapeutic antibodies/Fc-containing proteins for which minimal residual FcγR interaction is desired (Herold KC, et al.

(2005). Diabetes 54(6): 1763-1769).

The D265, N297 and P329 triple mutant according to the present invention exhibits a reduced binding to the first complement component C1q as compared to its wild-type counterpart. In other words, the affinity of the mutant for C1q is lower than that of the wild-type.

The D265, N297 and P329 triple mutant according to the present invention also exhibits an affinity for at least one Fcγ receptor lower than that of its parent polypeptide. As used herein, Fcγ receptors include FcγRI, FcγRII and FcγRIII receptors. Preferably, the at least one FcγR is selected from the group consisting of FcγRI, FcγRIIa, FcγRIIb, FcyRIIIa.

The D265, N297 and P329 triple mutant exhibits a reduced binding to both C1q and Fcγ receptors as compared to its wild-type counterpart.

In certain embodiments, the mutant IgG1 Fc-containing molecule exhibits a reduced binding to C1q, FcγRI, FcγRIIa, FcγRIIb, and FcγRIIIa as compared to its wild-type counterpart.

The binding for C1q or for anyone of Fc receptors can be evaluated by well-known methods of the prior art such as AlphaScreen™ and Surface Plamon Resonance (SPR).

For example, the bond strength of a mutant of the invention for a protein of interest (such as C1q or a FcγR) may be compared to that of its wild-type counterpart by calculating the ratio of their specific $IC_{50}$ values obtained by AlphaScreen™ competition assay as described in Example 4. AlphaScreen™, used in high throughput screening, is a homogenous assay technology which allows detection of molecular events such as binding. Coated "Donor" and "Acceptor" beads are the basis of the assay technology. As a bead based assay, AlphaScreen™ works through the interaction of the beads in close proximity, resulting in a cascade of chemical reactions that act to produce a greatly amplified signal. Direct or indirect, e.g., competitive binding, measurements can be applied for assessing relative affinities and avidities among and between proteins.

As an alternative, the binding of the mutant IgG1 Fc-containing molecule and that of its wild-type counterpart for a protein of interest (e.g., C1q and/or an FcγR) may be compared through the determination of $EC_{50}$ by an appropriate ELISA assay. The $EC_{50}$ refers to the concentration of the mutant which provides a signal representing 50% of the saturation of the curve relating to the percentage of bound protein of interest versus the log of the concentration of the mutant. Generally, a mutant IgG1 Fc-containing molecule is considered to display a reduced binding to a protein of interest (such as C1q and/or an FcγR) as compared to its wild-type counterpart if its $EC_{50}$ is at least 1.5-fold higher than that of its wild-type counterpart.

The binding affinity of the mutant IgG1 Fc-containing molecule to a protein of interest (e.g., C1q and/or a FcγR) may also be assessed by SPR through the determination of the constant of dissociation (Kd). Generally, a mutant IgG1 Fc-containing molecule is considered to display a reduced binding to a protein of interest (e.g. C1q and/or a FcγR) as compared to its wild-type counterpart if its Kd is at least 1.5-fold higher than that of its polypeptide parent.

The affinity of the mutant for C1q or for an FcγR may be so weak that the specific signal by AlphaScreen™ assay and even the Kd by SPR or the $EC_{50}$ by ELISA assay cannot be accurately determined since the binding signal is in the background noise or under the threshold of detection. In such a case, the mutant IgG1 Fc-containing molecule is considered not to bind the C1q and/or respective FcγR.

For example, the triple mutant IgG1 Fc-containing molecule according to the invention may not bind to at least one FcγR and exhibits a reduced or no binding to C1q.Such a mutant IgG1 Fc-containing molecule is clearly illustrated in the examples of the present application.

In some embodiments, the mutant IgG1 Fc-containing molecule of the invention does not bind to at least one protein selected from C1q and Fcγ receptors.

The Applicant showed that the introduction of mutations at D265, N297 and P329 are sufficient to significantly impair the binding to C1q and to Fcγ receptors. In other words, no mutation other than those at D265, N297 and P329 needs to be introduced within the IgG1 Fc region of the IgG1 wild-type counterpart in order to obtain a mutant IgG1 Fc-containing molecule with appropriate reduced binding to C1q and/or Fcγ receptors. Nevertheless, it would optionally be possible to add further mutations to the Fc-containing molecule of the invention if so desired, e.g. to alter other functionalities of the molecule.

Without to be bound by any theory, the Applicant believes that the amino acid substitutions provided by the present invention do not significantly cause major structural rearrangement in the IgG1 Fc region so that in some cases, the other functions which are not mediated by the binding to C1q and FcγRs are not significantly altered as compared to those of the polypeptide parent. Noticeably, the Applicant showed that the introduction of substitution mutations at positions D265, N297 and P329 in the IgG1 Fc region does not significantly impair their affinity for neonatal Fc Receptor (FcRn). For example, the dissociation constant, $K_D$, for mAb1, comprising D265A, N297A and P329A IgG1 Fc substitutions (DANAPA), is 500 nM and 470 nM for its wild-type counterpart (see Example 8). In other words, the wild-type IgG1 Fc-containing molecule and mutant IgG1 Fc-containing molecule according to the present invention display close binding property for FcRn.

As mentioned hereabove, the Fc region of the wild-type may be selected from the group consisting of wild-type Fc regions of human IgGs, fragments and mutants thereof.

As indicated hereabove, the Fc region of the invention may comprise amino acid substitutions of at least three amino acids in the IgG1 Fc. For reminder, wild-type Fc regions include, without being limited to, the Fc region of human IgG1 having SEQ ID NO: 43. Allelic variants of human Fc regions are known and can also be used as the parent molecule to introduce the combination of mutations according to the invention. Allelic variants of human IgG1 Fc differ from each other at position 356 (Glutamic acid (E) or Aspartic acid (D)), and/or at position 358 (Methionine (M) or Leucine (L)) and/or at position 431 (Alanine (A) or Glycine (G)). Allelic variants include naturally occurring allelic variants as well as non-natural allelic variants. Non-natural allelic variants contain residues which do occur in naturally occurring allelic variants but in combinations which are not found in nature. Jefferis et al. provide an overview on human IgG allelic variants which allows a skilled person to derive naturally occurring and non-natural allelic variants of Fc sequences (Jefferis R and M-P Lefranc (2009) mAbs 1: 1-7). In certain embodiments, the parent molecule for the introduction of the combination of mutations according to the invention (i.e. mutations at positions 265, 297 and 329 according to Kabat numbering) therefore is a molecule comprising a human IgG1 Fc sequence chosen from the group consisting of SEQ ID NOs: 43, 52, 53, 54, 55, 56, 57, and 58. The invention in specific embodiments thus provides recombinant IgG1 Fc-containing polypeptides comprising an amino acid sequence according to any one of SEQ ID Nos: 43, 52, 53, 54, 55, 56, 57, and 58, characterized in that: (i) the amino acid D at position 265 has been replaced by another amino acid, (ii) the amino acid N at position 297 has been replaced by another amino acid, and (iii) the amino acid P at position 329 has been replaced by another amino acid, wherein the numbering is indicated by the EU index as in Kabat.

An Fc region according to the invention as compared to a wild-type or parent Fc region has a combination of mutations, such that amino acid residues at positions 265, 297 and 329 are different from D, N and P, respectively, wherein numbering is according to the EU index in Kabat et al. In certain embodiments, the amino acid residue at position 265 is A, N or E. In certain embodiments, the amino acid residue at position 297 is A, D or Q. In certain embodiments, the amino acid residue at position 329 is A, G or S. The skilled person will appreciate that other amino acids can be substituted on these positions (e.g. R, C, Q, G, H, I, L, K, M, F, P, S, T, W, Y, or V at position 265; R, C, E, G, H, I, L, K, M, F, P, S, T, W, Y, or V at position 297; R, C, Q, N, H, I, L, K, M, F, E, D, T, W, Y, or V at position 329) and resulting Fc variants having the indicated amino acids at positions 265, 297 and 329 can be tested by routine methods for having substantially the same properties in binding to Fc receptors and C1q as the embodiments exemplified in the working examples herein, and such variants are included in the present invention.

In some embodiments, the amino acid substitutions of the IgG1 Fc-containing molecule comprise amino acid substitutions D265A, N297A, P329A.

In some other embodiments, the amino acid substitutions of the IgG1 Fc-containing molecule comprise amino acid substitutions D265N, N297D, P329G.

In some other embodiments, the amino acid substitutions of the IgG1 Fc-containing molecule comprise amino acid substitutions D265E, N297Q, P329S.

In a specific embodiment, a mutant IgG1 Fc-containing molecule, which mutant exhibits reduced binding to the protein C1q and to at least one receptor FcγR as compared to the wild-type IgG1 Fc-containing molecule is characterized in that:

1. amino acid at position 265 is replaced with alanine, asparagine or glutamic acid,
2. amino acid at position 297 is replaced with alanine, aspartate, or glutamine, and
3. amino acid at position 329 is replaced with alanine, glycine, or serine, wherein the numbering of amino acids in indicated by the EU index as in Kabat.

In some embodiments, a method of making a recombinant IgG1 Fc-containing molecule, comprising a CH2 domain in which amino acids at position 265, 297, and 329 indicated by the EU index as in Kabat are replaced by other amino acids than D, N and P respectively, comprises the steps of:

(a) providing a nucleic acid encoding a parent IgG1 Fc-containing molecule,
(b) modifying the nucleic acid provided in step (a) so as to obtain a nucleic acid encoding a recombinant IgG1 Fc-containing molecule wherein the amino acids at at least one of positions 265, 297, and 329 are replaced such that in the resulting encoded Fc-containing molecule the amino acids on these positions are different from D (position 265), N (position 297) and P (position 329), and
(c) expressing the nucleic acid obtaining in step (b) in a host cell and recovering the said mutant.

Of course, if the parent molecule already contains a different amino acid than D at position 265, N at position 297, or P at position 329, only the other one or two of these three positions still needs to be modified to create an Fc containing molecule according to the invention.

Such steps may be performed by conventional practices of molecular biology. For carrying out a method of making a recombinant IgG1 Fc-containing molecule of the invention, the one skilled in the art may refer to well-known procedures described in the prior art which may be found e.g. in Molecular Cloning - A Laboratory Manual, 3rd Ed. (Maniatis, Cold Spring Harbor Laboratory Press, New York, 2001), The condensed protocols from Molecular cloning: a laboratory manual (Sambrook, Russell, CSHL Press, 2006), and Current Protocols in Molecular Biology (John Wiley & Sons, 2004).

The nucleic acid of the wild-type IgG1 Fc-containing molecule may be commercial or may be obtained by classical procedure of molecular biology or chemical synthesis. The nucleic acid encoding the mutant IgG1 Fc-containing molecule as mentioned in step (b) may be achieved by chemical synthesis, or by modifying the nucleic acid of the parent polypeptide using a variety of methods known in the prior art. These methods include, but are not limited to site-directed mutagenesis, random mutagenesis, PCR mutagenesis and cassette mutagenesis.

The nucleic acid encoding the mutant IgG1 Fc-containing molecule may be incorporated into an expression vector for its expression in a host cell.

Expression vectors typically include a protein encoding sequence operably linked, that is, placed in a functional relationship, with control or regulatory sequences such as a promoter, as well as optionally including selectable markers, any fusion partners, and/or additional elements. The mutant IgG1 Fc-containing molecule of the present invention may be produced by culturing a host cell transformed with nucleic acid, preferably an expression vector, containing nucleic acid encoding the mutant IgG1 Fc-containing molecule, under the appropriate conditions to induce or cause expression of the protein. A wide variety of appropriate host cell lines may be used, including but not limited to mammalian cells, bacteria, insect cells, and yeast.

For example, a variety of mammalian cell lines that can be used are described in the ATCC cell line catalog, available from the American Type Culture Collection. Host cells may be, but not limited to, YB2/0 (YB2/3HL.P2.GII.IGAg.20 cell, deposit to the American Type Culture Collection, ATCC n°CRL-1662), SP2/0, YE2/0, 1R983F, Namalwa, PER.C6, CHO cell lines, particularly CHO-K-1, CHO-Lecl0, CHO-Lecl, CHO-Lecl3, CHO Pro-5, CHO dhfr-, Wil-2, Jurkat, Vero, Molt-4, COS-7, HEK293, BHK, Vero, MDCK, immortalized amniotic cell lines (CAP), EB66, KGH6, NSO, SP2/0-Ag 14, P3X63Ag8.653, C127, JC, LA7, ZR-45-30, hTERT, NM2C5, UACC-812 and the like. The methods of introducing exogenous nucleic acid into host cells are well known in the art, and may vary with the host cell used.

The host cell may optionally belong to a transgenic non-human animal or to a transgenic plant. In this case, the mutant IgG1 Fc-containing molecule is thus obtained from a transgenic organism.

A transgenic non-human animal can be obtained by directly injecting a desired gene into a fertilized egg (Gordon et al., 1980 Proc Natl Acad Sci U S A.;77:7380-4). The transgenic non-human animals include mouse, rabbit, rat, goat, cow, cattle or fowl, and the like. A transgenic non-human animal having a desired gene can be obtained by introducing the desired gene into an embryonic stem cell and preparing the animal by an aggregation chimera method or injection chimera method (Manipulating the Mouse Embryo, A Laboratory Manual, second edition, Cold Spring Harbor Laboratory Press (1994); Gene Targeting, A Practical Approach, IRL Press at Oxford University Press (1993)). Examples of the embryonic stem cell include embryonic stem cells of mouse (Evans and Kaufman, 1981, Nature; 292:154-156), rat, goat, rabbit, monkey, fowl, cattle and the like. In addition, a transgenic non-human animal can also be prepared using a clonal technique in which a nucleus into which a desired gene is introduced is transplanted into an enucleated egg (Ryan et al., 1997 Science; 278: 873 - 876; Cibelli et al., 1998 Science, 280: 1256-1258). The mutant IgG1 Fc-containing molecule can be produced by introducing DNA encoding the mutant IgG1 Fc-containing molecule into an animal prepared by the above method to thereby form and accumulate the mutant molecule in the animal, and then collecting the mutant from the animal. The mutant IgG1 Fc-containing molecule may be made to be formed and accumulated in the milk, egg or the like of the animal.

As used above, an IgG1 Fc-containing molecule may be a naturally occurring polypeptide (wild-type polypeptide), a mutant or an engineered version of a naturally occurring polypeptide, or a synthetic polypeptide.

In some embodiments, an IgG1 Fc-containing molecule is selected from the group consisting of IgG1 Fc-fusion protein, IgG1 Fc-conjugate, and antibodies.

As used herein, Fc-fusion protein and Fc-conjugate consist of an Fc region linked to a partner. The Fc region can be linked to its partner with or without a spacer, also referred to as linker.

Suitable linkers are at the skilled person's disposal. A linker can for instance be selected from the group consisting of alkyl with 1 to 30 carbon atoms, polyethylene glycol with 1 to 20 ethylene moieties, polyalanine with 1 to 20 residues, caproic acid, substituted or unsubstituted poly-p-phenylene and triazol. Preference is given to peptidic linkers, more specifically to oligopeptides having a length from 1 to 30 amino acids. Preferred length ranges are from 5 to 15 amino acids.

Particularly preferred are linkers which are peptides which consist of at least 50%, at least 60%, at least 70%, at least 80%, at least 90% or 100% of small amino acids such as glycine, serine and alanine. Particularly preferred are linkers consisting of glycines and serines only. A non-limiting example of a suitable linker is a (G4S)3 linker (SEQ ID NO: 40).

According to the present invention, an Fc fusion protein is a protein that comprises a protein, a polypeptide or a small peptide covalently linked to an Fc region. An Fc fusion protein optionally comprises a peptide linker as described above. Virtually any protein or small peptide may be linked to Fc regions to generate an Fc fusion. Protein fusion partners may include, but are not limited to, the target-binding region of a receptor, an adhesion molecule, a ligand, an enzyme, a cytokine, a chemokine, or some other protein or protein domain. Some non-limiting examples of Fc fusion proteins include alefacept, abatacept, belatacept, rilonacept, etanercept, romiplostim, and ablifercept.

In particular the Fc-fusion protein can be an immunoadhesin, i.e. antibody-like protein which combines the binding domain of a heterologous "adhesion" protein (i.e receptor, ligand or enzyme) with a fragment of immunoglobulin constant domain (i.e. an Fc region) (see for a review about immunoadhesins, Ashkenazi A, Chamow SM. 1997, Curr Opin Immunol. 9(2): 195-200).

Small peptides may include, but are not limited to, any therapeutic agent that directs the Fc fusion to a therapeutic target.

According to the present invention, an Fc conjugate may in certain embodiments result from the chemical coupling of an Fc region with a conjugate partner and optionally comprises a spacer linking the Fc region to the conjugate partner. The conjugate partner can be proteinaceous or non-proteinaceous. The coupling reaction generally uses functional groups on the Fc region and on the conjugate partner.

Suitable conjugate partners include, but are not limited to, therapeutic polypeptides, labels (for example of labels, see further below), drugs, cytotoxic agents, cytotoxic drugs (e.g., chemotherapeutic agents), toxins and active fragments of such toxins. Suitable toxins and their corresponding fragments include, but are not limited to, diptheria A chain, exotoxin A chain, ricin A chain, abrin A chain, and the like. A cytotoxic agent may be any radionuclide which can be directly conjugated to the IgG1 Fc mutant or sequestrated by a chelating agent which is covalently attached to the IgG1 Fc mutant. In additional embodiments, the conjugate partners can be selected from the group consisting of calicheamicin, auristatins, geldanamycin, maytansine, and duocarmycins and analogs.

In one embodiment, the IgG1 Fc-containing molecule comprises a "Fynomer". Fynomers are small 7-kDa globular proteins derived from the SH3 domain of the human Fyn kinase (Fyn SH3, aa 83-145 of Fyn kinase: GVTLFVALYDYEARTEDDLSFHKGEKFQILNSSEGDWWEARSLTTGETGYIPS NYVAPVDSIQ (SEQ ID NO: 59). In SEQ ID NO: 59 as shown above the sequences of the RT and the src loop are underlined and double-underlined, respectively, and such molecules can be engineered to bind with antibody-like affinity and specificity to virtually any target of choice through random mutation of two loops (RT- and src-loop) on the surface of the Fyn SH3 domain, optionally combined with mutations of other selected positions in the Fyn SH3 domain (see, e.g. WO 2008/022759). Fyn SH3-derived polypeptides or Fynomers are well known in the art and have been described e.g. in Grabulovski et al. (2007) JBC, 282, p. 3196-3204; WO 2008/022759; Bertschinger et al (2007) Protein Eng Des Sel 20(2):57-68; and Gebauer and Skerra (2009) Curr Opinion in Chemical Biology 13:245-255. The term "Fyn SH3-derived polypeptide", used interchangeably herein with the term "Fynomer", refers to a non-immunoglobulin-derived binding polypeptide (e.g. a so-called scaffold as described in Gebauer and Skerra (2009) Curr Opinion in Chemical Biology 13:245-255) derived from the human Fyn SH3 domain. Fynomers can be genetically fused to other molecules such as antibodies, to create so-called FynomAbs that can be engineered to have dual specificity (e.g. Silacci et al, 2016, mAbs 8:1, 141-149; Brack et al, 2014, Mol Cancer Ther 13(8): p. 2030-9; WO 2014/044758 A1; WO 2014/170063 A1; WO 2015/141862 A1).

As mentioned, the term "antibody" is used herein in the broadest sense. According to the present invention, "antibody" refers to any polypeptide which at least comprises (i) an Fc region and (ii) a binding polypeptide domain derived from a variable domain of an immunoglobulin. The said binding polypeptide domain is able to bind specifically one given target antigen or a group of target antigens. A binding polypeptide domain which derives from a variable region of an immunoglobulin comprises at least one or more CDRs. Herein, antibodies include, but are not limited to, full-length antibodies, multi-specific antibodies, Fc-fusion protein comprising at least one variable region or synthetic antibodies (sometimes referred to herein as "antibody mimetics"), antibody-fusion proteins, antibody conjugates and fragments of each respectively. FynomAbs according to the invention also comprise antibodies with an Fc region. The invention thus also provides FynomAbs, i.e. one or more copies of a Fynomer coupled to an antibody, that comprise an Fc region with the mutations according to the invention, i.e. having an amino acid different from D at position 265, an amino acid different from N at position 297, and an amino acid different from P at position 329, wherein numbering is according to the EU index as in Kabat et al. The Fynomer can be covalently linked via a linker peptide to the antibody, or may be directly fused to the antibody. The Fynomer in certain embodiments may be located downstream of the C-terminus of the heavy chain of the antibody, or upstream of the N-terminus of the heavy chain of the antibody, or downstream of the C-terminus of the light chain of the antibody, or upstream of the N-terminus of the light chain of the antibody. Preferably, two copies of the Fynomer are coupled to the antibody, one of each to a corresponding terminus in two chains of the antibody, e.g. one copy at the N-terminus of the light chain of the first half of the antibody and one copy at the N-terminus of the light chain of the second half of the antibody (a "half' of an antibody meaning herein a heavy chain and a light chain that together comprise a binding region), or one copy at the N-terminus of the heavy chain of the first half of the antibody and one copy at the N-terminus of the heavy chain of the second half of the antibody, or one copy at the C-terminus of the light chain of the first half of the antibody and one copy at the C-terminus of the light chain of the second half of the antibody, or one copy at the C-terminus of the heavy chain of the first half of the antibody and one copy at the C-terminus of the heavy chain of the second half of the antibody (see e.g. Brack et al, 2014, Mol Cancer Ther 13: 2030-2039, and Fig 8 of WO 2013/135588, for examples of different positions of Fynomers at the four termini of an IgG antibody). Such fusions can be generated by genetic engineering, cloning nucleic acid encoding the Fynomer part and the respective antibody chain in frame to form a single fusion molecule. Co-expression with the other chain of the antibody (e.g. the light chain in case the Fynomer is fused to the heavy chain, or the heavy chain in case the Fynomer is fused to the light chain) within a cell will lead to expression of functional Fynomabs. The Fynomer part may bind to a different target molecule than the antibody part (for non-limiting examples see e.g. Fynomabs described in Silacci et al, 2016, mAbs 8:1, 141-149; WO 2014/044758 A1; WO 2014/170063 A1; WO 2015/141862 A1) or the Fynomer part may bind to a different epitope on the same target molecule as the antibody part (for non-limiting examples see Fynomabs described in Brack et al, 2014, Mol Cancer Ther 13(8): p. 2030-9; WO 2013/135588).

By Fc-fusion protein comprising at least one variable region is meant an engineered protein comprising (i) an Fc region and (ii) a binding polypeptide domain derived from a variable domain of an immunoglobulin. Of particular interest are antibodies that comprise (a) an IgG1 Fc mutant of the invention, and (b) one of the following binding polypeptide domains derived from a variable region of an immunoglobulin (i.e. which comprise at least one CDR) : (i) the Fab fragment consisting of VL, VH, CL and CH1 domains, (ii) the Fd fragment consisting of the VH and CH1 domains, (iii) the Fv fragment consisting of the VL and VH domains of a single antibody; (iv) isolated CDR regions, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site, (vii) bispecific single chain Fv and (viii) "diabodies" or "triabodies", multivalent or multispecific fragments constructed by gene fusion, this list not being limitative.

By "full length antibody" herein is meant an antibody having the natural-occurring biological form of an antibody, including variable and constant regions. A full-length antibody may be a wild-type antibody, a mutant of a wild-type antibody (e.g. comprising pre-existing modifications), an engineered version of a wild-type antibody (e.g. for example a chimeric, a humanized antibody or a fully human antibody, see further below), this list not being limitative. As well-known, the structure of a full-length antibody is generally a tetramer except for some mammals such as llamas and camels in which some immunoglobulins are dimers.

The scaffold components of the full-length antibody may be a mixture from different species. Such antibody mutant may be a chimeric antibody and/or a humanized antibody. In general, both "chimeric antibodies" and "humanized antibodies" refer to antibodies that combine regions from more than one species. For example, "chimeric antibodies" traditionally comprise variable region(s) from a non-human animal, generally the mouse (or rat, in some cases) and the constant region(s) from a human. For the most part, humanized antibodies are chimeric antibodies that contain minimal sequence derived from non-human immunoglobulin. Generally, in a humanized antibody, the entire antibody, except the CDRs, is encoded by a polynucleotide of human origin or is identical to a human antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The method for preparing such antibodies are well-known and are described in, e.g., WO 92/11018; Jones, 1986, Nature 321:522-525 ; Verhoeyen et al., 1988, Science 239:1534-1536, Tsurushita & Vasquez, 2004, Humanization of Monoclonal Antibodies, Molecular Biology of B Cells, 533-545, Elsevier Science (USA)).

As used herein, "fully human antibody" or "complete human antibody" refers to an antibody entirely comprising sequences originating from human genes. In some cases this may be human antibodies that have the gene sequence of an antibody derived from a human chromosome with the modifications outlined herein. Alternatively, the components of the antibody may be human but not be derived from a single gene. Thus, for example, human CDRs from one antibody can be combined with sequences, such as scaffold sequences, from one or more human antibodies. For example, a variety of germline sequences can be combined to form a human antibody or human scaffold.

Full-length antibodies comprising covalent modifications are also included within the scope of this invention. Such modifications include, but are not limited to, glycosylations, labeling and conjugation.

Labeling refers to the coupling of a detectable label with the full-length antibody. As use herein, a label includes, without being limited to, : a) isotopic labels, which may be radioactive or heavy isotopes; b) magnetic labels (e.g., magnetic particles); c) redox active moieties; d) optical dyes such as chromophores, phosphors and fluorophores; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

Conjugation refers to the coupling of the full-length antibody with a polypeptide or a non-peptide molecule such as a target-binding region of a receptor, an adhesion molecule, a ligand, an enzyme, a cytokine, a chemokine, a drug, a cytotoxic agent (e.g., chemotherapeutic agents) or a toxin.

In certain embodiments, an IgG1 Fc-containing molecule is selected from the group consisting of chimeric immunoglobulins, humanized immunoglobulins, fully-human immunoglobulins, immunoglobulins being preferably selected among IgGs and optionally conjugated or labelled.

The properties of the mutant IgG1 Fc-containing molecule can be generally deduced from those of the wild-type IgG1 Fc-containing molecule except in terms of binding to C1q and Fcγ receptors since the binding of the mutant to C1q and FcγRs are controlled by the amino acid modifications at position 265, 297, and 329. Apart from these highly relevant differences, there are some minor differences in properties of the Fc-containing molecules of the invention and their corresponding wild-types, for instance a slight drop in thermostability due to a lack of N-linked glycosylation.

A further object of the invention is an isolated nucleic acid encoding a mutant IgG1 Fc-containing molecule as defined hereabove. The invention also relates to a vector comprising a nucleic acid encoding the mutant IgG1 Fc-containing molecule and to a host cell comprising the said vector. In a preferred embodiment, the nucleic acid encoding the said vector has been stably integrated in the genome of the host cell. Also disclosed is a non-human transgenic animal comprising the said nucleic acid or the said vector stably integrated within its genome.

## USES OF THE METHOD AND THE MUTANTS ACCORDING TO THE INVENTION

The Applicant showed that the substitution of amino acids 265, 297, and 329 of the IgG1 Fc region drastically impairs the affinity of the Fc mutant for C1q and for FcγRs such as FcγRI, FcyRIIa, FcyRIIb and FcyRIIa. The decrease in the affinity for these effector molecules is so pronounced that in some cases, the binding of the Fc mutant to C1q and/or to certain FcγRs cannot be observed *in vitro* by conventional AlphaScreen™/SPR assays. The binding of the IgG1 Fc region to C1q is essential for the induction of CDC *in vivo.* In the same way, the binding of the IgG1 Fc region to FcyRIIa and FcγRIIIa is a key step for the induction of ADCC and ADCP *in vivo.* Binding to FcγR can induce clustering of the cognate receptor, which may provide an agonistic signal through that receptor to the target cell.

Consequently, due to their poor affinity for C1q, the mutant IgG1 Fc-containing molecules of the invention are anticipated to have no CDC activity or to induce a significantly lower CDC response *in vivo* as compared to their wild-type counterparts (i.e., IgG1 Fc-containing molecules comprising an IgG1 Fc region with amino acids D at position 265, N at position 297, and P at position 329, wherein numbering is with reference to the EU index as in Kabat). In the same way, due to their poor affinity for certain FcγRs (in particular FcyRIIa and FcγRIIIa), the mutants of the invention are anticipated to have no ADCC activity or to induce a significantly lower ADCC response *in vivo* as compared to their wild-type counterparts. In the same way, the mutants of the invention are anticipated to not induce receptor clustering or agonism via FcγR engagement *in vivo.* The same result is also expected for *in vitro* CDC assays, ADCC assays and receptor clustering assays.

Due to their effector activity profiles, the mutants of the invention may find use in a wide range of scientific fields. In particular, the mutants of the invention may be used as research reagents, diagnostic agents or therapeutics.

For example, the mutants may be labeled with a fluorophore or with an isotope such as indium-111 or technetium-99m and be used for *in vivo* imaging since in such an application, the activation of ADCC or CDC is not required.

When used as therapeutics, the mutant may be used to convey a therapeutic agent such as radionuclides, toxins, cytokines or enzymes to a target cell for example a cancerous cell. In this case, the mutant may be a conjugate between an antibody and the cytotoxic agent and its therapeutic activity relies on the cytotoxic agent (e.g. Gilliland et al., PNAS, 1980, 77, 4539-4543).

The IgG1 Fc-containing molecule of the invention may also function as a blocking or neutralizing agent of a target molecule. It may also agonize, antagonize or inhibit a target molecule.

The IgG1 Fc-containing molecule of the invention may be used to target receptors without inducing receptor clustering or agonism via FcγR.

The target molecule may be of any kind and includes both exogenous and endogenous molecules. Target molecules (also called antigens when the polypeptide mutant is or comprises an antibody) include without being limited, viral, bacterial and fungal proteins, prions, toxins, enzymes, membrane receptors, drugs and soluble proteins.

Membrane receptors include, without being limited to, RhD antigen, CD3, CD4, CD19, CD20, CD22, CD25, CD28, CD32B, CD33, CD38, CD40, CD44, CD52, CD71 (transferrin receptor), CD80, CD86, CTLA-4, CD147, CD160, CD224, growth factor receptors like those belonging to the ErbB family of receptors ErbB1, ErbB2, ErbB3, ErbB4 (EGFR, HER2/neu, HER3, HER4), PD1, VEGF-R1, VEGF-R2, IGF-R1, PIGF-R, MHC class I and MHC class II molecules, e.g. HLA-DR, type I interferon receptor, interleukin receptors like IL-1R, IL-2R alpha, IL-2R beta and IL-2R gamma, IL-6R, hormone receptors like Müllerian inhibitory substance type II receptor, LDL receptor, NKp44L, chemokine receptors like CXCR4, CCR5, TNFR, CD137, integrins, adhesion molecules like CD2, ICAM, EpCAM, G-protein-coupled receptor, etc.

Other potential target proteins include tumour markers like GD2, GD3, CA125, MUC-1, MUC-16, carcinoembrionic antigen (CEA), Tn, glycoprotein 72, PSMA, HMW-MAA other proteins such as BDCA-2 specific for DC cells, glucagon-like peptides (e.g., GLP-1, etc.), enzymes (e.g., glucocerebrosidase, iduronate-2-sulfatase, alphagalactosidase-A, agalsidase alpha and beta, alpha-L-iduronidase, butyrylcholinesterase, chitinase, glutamate decarboxylase, imiglucerase, lipase, uricase, platelet-activating factor acetylhydrolase, neutral endopeptidase, myeloperoxidase, etc.),

interleukin and cytokine binding proteins (e.g., IL-18 bp, TNF-binding protein, etc.), macrophage activating factor, macrophage peptide, B cell factor, T cell factor, protein A, allergy inhibitor, cell necrosis glycoproteins, immunotoxin, lymphotoxin, tumor necrosis factor, tumor suppressors, etc.

Soluble proteins include, without being limited to, cytokines such as for instance IL-1 beta, IL-2, IL-6, IL-12, IL-23, TGF beta, TNF alpha, IFN gamma, chemokines, growth factors like VEGF, G-CSF, GM-CSF, EGF, PIGF, PDGF, IGF, hormones and inhibitory antibody such as a FVIII inhibitory, metastasis growth factor, alpha-1 antitrypsin, albumin, alpha-lactalbumin, apolipoprotein-E, erythropoietin, highly glycosylated erythropoietin, angiopoietins, hemoglobin, thrombin, anti-thrombin III, thrombin receptor activating peptide, thrombomodulin, factor VII, factor VIIa, factor VIII, factor IX, factor XIII, plasminogen activating factor, fibrin-binding peptide, urokinase, streptokinase, hirudin, protein C, C-reactive protein, B cell activating factor receptor, receptor antagonists (e.g., IL1-Ra), complement proteins, C1, C2, C3, C4, C5, C6, C7, C8, C9, factor H, factor I, factor P, other proteins such as CSAP, CD137-ligand, lectins, sialylated proteins.

In some exemplary and non-limiting embodiments, the IgG1 Fc-containing molecule may be selected from anti-CD3, anti-HER2, and anti-PD1 antibodies or molecules comprising such antibodies.

In certain embodiments, the IgG1 Fc-containing molecule comprises an anti-CD3 antibody. In certain embodiments, the molecule of the invention comprises an antibody that binds to CD3, as well as another binding moiety such as a Fynomer binding to another target, i.e. it has bispecific binding activities. Such molecules can be agonistic mAbs used for treating cancer, and are for instance described in more detail in the examples herein.

In some embodiments, the mutant IgG1 Fc-containing molecule is or comprises a neutralizing antibody directed to a target molecule selected from the group of membrane receptors, human soluble proteins, toxins, viral, bacterial and fungal proteins.

Because of its low binding to C1q and some FcγRs, the mutant of the invention is particularly appropriate to be used for the treatment of conditions in which the recruitment of the immune system through ADCC or CDC, or where clustering of the cognate receptor or agonism via FcγR, is not crucial for the therapeutic efficiency.

In some cases, the administration of the mutant IgG1 Fc-containing molecule of the invention is anticipated to induce less side-effect and less IgG-mediated cytotoxicity than most of the antibodies and immunoadhesins which do not comprise mutations at amino acid position 265, 297, and 329 in their IgG1 Fc region.

Also disclosed is thus the use of the mutant IgG1 Fc-containing molecule of the invention for preventing or treating a pathological condition wherein FcR-mediated effects including the induction of ADCC and/or CDC responses, or the clustering of the cognate receptor via FcγR, is not desirable.

The induction of ADCC and CDC responses is not desirable when the therapeutic efficacy of the mutant does not require effector-cell activation or CDC activation. Such a mutant includes for example blocking or neutralizing antibodies.

Pathological conditions which treatment or prevention do not require the induction of CDC and ADCC, include without being limited to, graft rejection, autoimmune diseases, inflammatory diseases.

The induction of receptor clustering via FcγR is not desirable when the therapeutic efficacy of the mutant does not require FcγR mediated receptor clustering for therapeutic efficacy. Such mutants include for instance CD3/tumor antigen bispecific molecules, which require clustering of the CD3 receptor in a strictly tumor antigen dependent manner, but not in an FcyR-dependent manner.

In certain embodiments, the invention provides a FynomAb according to the invention (i.e. comprising an IgG1 Fc-region with a CH2 domain wherein the amino acid at position 265 is not D, the amino acid at position 297 is not N, and the amino acid at position 329 is not P, wherein numbering is according to the EU index as in Kabat) having an antibody part binding to CD3 and a Fynomer part binding to CD33.

Also disclosed is the use of a mutant of the invention for preparing a pharmaceutical composition.

A further object of the invention is to provide pharmaceutical compositions comprising the said mutant. When the mutant IgG1 Fc-containing molecule is an antibody, the mutant may be present in the form of monoclonal or polyclonal antibodies. The pharmaceutical compositions are prepared by mixing the polypeptide mutant having the desired degree of purity with optional physiologically acceptable carrier, excipients or stabilizers in the form of lyophilised formulations or aqueous solutions.

The pharmaceutical composition of the invention may be formulated according to standard methods such as those described in Remington: The Science and Practice of Pharmacy (Lippincott Williams & Wilkins; Twenty first Edition, 2005).

Pharmaceutically acceptable excipients that may be used are, in particular, described in the Handbook of Pharmaceuticals Excipients, American Pharmaceutical Association (Pharmaceutical Press; 6th revised edition, 2009).

In order to treat a patient in need, a therapeutically effective dose of the mutant IgG1 Fc-containing molecule of the invention may be administered. By "therapeutically effective dose" herein is meant a dose that produces the effects for which it is administered. The exact dose will depend on the purpose of the treatment, and will be ascertainable by one skilled in the art using known techniques. Dosages may range from 0.0001 to 100 mg/kg of body weight or greater, for example 0.001, 0.01, 0.1, 1.0, 10, or 50 mg/kg of body weight, with 0.001 to 10 mg/kg being preferred. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art.

Administration of the pharmaceutical composition comprising a mutant IgG1 Fc-containing molecule of the invention may be done in a variety of ways, including, but not limited to, orally, subcutaneously, intravenously, parenterally, intranasally, intraortically, intraocularly, rectally, vaginally, transdermally, topically (e.g., gels), intraperitoneally, intramuscularly, intrapulmonary.

The mutant IgG1 Fc-containing molecules described herein may optionally be administered with other therapeutics concomitantly, i.e., the therapeutics described herein may optionally be co-administered with other therapies or therapeutics, including for example, small molecules, other biologicals, radiation therapy, surgery, etc.

## EXAMPLES

The following examples are provided to supplement the prior disclosure and to provide a better understanding of the subject matter described herein. These examples should not be considered to limit the described subject matter. It is understood that the examples and embodiments described herein are for illustrative purposes only and that various modifications or changes in light thereof will be apparent to persons skilled in the art.

## EXAMPLE 1. EXPRESSION AND PURIFICATION OF FC MUTATED

## ANTIBODIES

Several antibodies based on mAb1, a human IgG1 antibody specific to human CD3, were produced with different mutations in the CH2 domain. The mutations were:

- i) N297A,
- ii) D265 plus P329A (DAPA),
- iii) D265 plus N297A plus P329A (DANAPA), and
- iv) L234A plus L235A (LALA)

(EU numbering according to Kabat (Kabat, E. A. (1991). Sequences of proteins of immunological interest, Bethesda, MD : U.S. Dept. of Health and Human Services, Public Health Service, National Institutes of Health, 1991).

For expression of antibodies, a leader sequence is typically present, which is cleaved off and no longer present in the secreted product. An example of a leader sequence used for expression in the examples described herein is provided in SEQ ID NO: 42, and an example of a nucleotide sequence encoding such is provided in SEQ ID NO: 41.

Expression vectors encoding the antibodies with the different Fc mutations were transiently transfected into FreeStyle CHO-S cells and expressed in serum-free/animal component-free media for 6 days. The anti-CD3 antibodies were purified from the supernatants by Protein A affinity chromatography (GE-Healthcare cat no 89928) with an ÄKTA Purifier instrument (GE Healthcare) and dialyzed against PBS. Concentrations were determined by absorbance measurement at 280 nm.

SEC was performed using a SEC-5 column (Agilent, 5 μm particle size, 300Å) on an Agilent HPLC 1260 system. 10 μl purified protein was loaded on the column and elution was recorded by OD280 measurement.

The Fc mutated antibody mutants could be purified with good yields and high purity by single-step protein A affinity chromatography. Yields are listed in Table 1. As found by SEC, all proteins were approximately 95 % monomeric. The SEC profiles of mAb1 IgG1 and mAb1 DANAPA IgG1 are shown in Fig. 1.

These results demonstrate the DANAPA triple mutation inserted into a human IgG1 sequence retains good expression and monodispersity, which both are key criteria for a pharmaceutical product.

Table 1. Protein Yields of mAb1 mutants

| Clone ID | Fc mutations | Heavy Chain SEQ ID NO: | Light Chain SEQ ID NO: | Purification yield (mg/l) |
|---|---|---|---|---|
| mAb1 IgG1 | none (wild-type) | 2 | 4 | 98 |
| mAb1 N297A IgG1 | N297A | 49 | 4 | 97 |
| mAb1 DAPA IgG1 | D265A, P329A | 47 | 4 | 66 |

(continued)

| Clone ID | Fc mutations | Heavy Chain SEQ ID NO: | Light Chain SEQ ID NO: | Purification yield (mg/l) |
|---|---|---|---|---|
| mAb1 DANAPA IgG1 | D265A, N297A, P329A | 45 | 4 | 62 - 132 |
| mAb1 LALA IgG1 | L234A, L235A | 51 | 4 | 88 |

## EXAMPLE 2: FC MUTATED ANTIBODIES BIND TO CD3-EXPRESSING CELLS WITH IDENTICAL AFFINITY AS THE UNMODIFIED ANTIBODY

Different Fc mutated mAb1 were titrated on $CD3^+$ Jurkat cells (ATCC® TIB-152™) to assess the binding affinity to human CD3. Serial dilutions of Fc mutated antibodies between 50 nM and 0.13 pM concentration were added to Jurkat cells, and bound antibody was detected with an anti-human IgG - Alexa488 conjugated antibody. The mean fluorescent intensity (MFI) determined on a cytometer was plotted against the antibody concentration on a logarithmic scale.

The binding curves obtained on $CD3^+$ Jurkat cells are shown in Fig. 2.

The Fc mutated antibody mutants bound to CD3 with identical affinity, indicating that the Fc mutations do not have any impact on target cell binding.

## EXAMPLE 3: MAB1 DANAPA IGG1 DOES NOT INDUCE LYMPHOCYTE ACTIVATION

In order to investigate the effect of Fc mutated mAb1 on immune cell activation, freshly isolated human PBMC were incubated in the presence of Fc mutated mAb1. Immune cell activation was detected by i) CD69 surface staining after 14 h incubation, or by ii) quantification of IFNγ in the supernatant after 3 days incubation. Human PBMC were isolated from buffy coat preparations collected by Blutspende Bern, Switzerland, one day before PBMC isolation. PBMC were isolated by density centrifugation, using Pancoll tubes (Pan-BioTech) according to the manufacturer's instructions. After PBMC isolation, residual red blood cells were lysed with 1× RBC lysis buffer (Miltenyi).

100'000 freshly isolated PBMC were mixed with various Fc mutants of mAb1 at serial dilutions (concentrations between 300 nM and 0.15 pM) in a total volume of 200 μl RPMI1640 supplemented with 10% heat-inactivated FBS in the wells of a 96-well U-bottom plate. As positive control, PBMC were incubated in the presence of anti-CD2/CD3/CD28 activation MACSibeads contained in the human T cell activation/expansion kit purchased from Miltenyi.

CD69 surface expression was determined after 14 h incubation. The contents of the assay wells were mixed, and 100 μl of each well was transferred into a 96-well U-bottom plate for subsequent CD69 staining. Cells were pelleted and resuspended in 40 μl anti-CD69-FITC conjugated antibody (BD Biosciences) in FACS buffer containing 1% FBS and 0.2% sodium azide. After 45 min incubation on ice, unbound antibody was washed off, samples were fixed in 50 μl 1.8% formalin for 15 min on ice, and analyzed on a Guava easeCyte 8HT flow cytometer (Millipore). The percentage CD69 positive lymphocytes were plotted against the antibody concentration on a logarithmic scale.

IFNγ levels in the supernatant were determined by sandwich ELISA after 3 days incubation, using the BD OptEIA human IFNγ ELISA set (BD biosciences) according to the manufacturer's instructions. IFNγ concentrations were plotted against the antibody concentration on a logarithmic scale.

Unexpectedly, mAb1 DANAPA IgG1 was the only construct that did not induce lymphocyte activation, as demonstrated by the lack of induction of CD69 expression on PBMC (Fig. 3A), and of IFNy in the culture supernatant (Fig. 3B). In contrast, all other mutants which contain single or combined Fc mutations previously reported to reduce FcR binding, still induced significant lymphocyte activation. Importantly, the DANAPA Fc sequence led to better silencing than the N297A Fc sequence or the LALA Fc sequence, both silenced Fc sequences used in several clinical-stage therapeutic Fc containing proteins for which minimal FcR interaction is desired. These results suggest that the DANAPA Fc sequence confers a strongly reduced potential to induce T cell activation and cytokine release in human PBMC assays.

## EXAMPLE 4: DANAPA IGG1 SHOWS MINIMAL BINDING TO HUMAN FCγ RECEPTORS

Binding to FcγRI (CD64), FcyRIIA (CD32A), FcyRIIB (CD32B) and FcγRIIIA (CD16A) was characterized by AlphaScreen™ competition assay (Vafa, O., G. L. Gilliland, R. J. Brezski, B. Strake, T. Wilkinson, E. R. Lacy, B. Scallon, A. Teplyakov, T. J. Malia and W. R. Strohl (2014). Methods 65(1): 114-126). The assay is schematically illustrated in Fig. 4A. A biotinylated control antibody is captured on Streptavidin Donor beads; His-tagged Fcγ receptors are captured on $Ni^{2+}$ Acceptor beads; serial dilutions of unlabelled antibodies with Fc of interest are applied as competitors. This format produces a reduction in the signal when receptor binding of the competitors takes place.

B21M, a human IgG1 control antibody specific to respiratory syncytial virus and believed not to bind specifically to any targets in healthy mammals (Vafa, O., G. L. Gilliland, R. J. Brezski, B. Strake, T. Wilkinson, E. R. Lacy, B. Scallon, A. Teplyakov, T. J. Malia and W. R. Strohl (2014). Methods 65(1): 114-126), was labeled with biotin (SureLINK Chromophoric Biotin Labeling Kit, KPL). 0.2 μg/ml biotinylated B21M IgG1 control antibody, Fc mutated test antibodies (400 μg/ml, and eight serial 3-fold dilutions thereof), His-tagged human Fcγ receptors (R&D, carrier-free formulation), $Ni^{2+}$-acceptor beads (Perkin Elmer, 1:250 diluted), and Streptavidin donor beads (Perkin Elmer, 1:250 diluted) were mixed in assay buffer (PBS, 0.05% BSA, 0.01% Tween 20, pH 7.2) in the order indicated above. The human Fcγ receptors were used at the following concentrations: FcγRI and FcyRIIIA at 200 ng/ml; FcyRIIA at 10 ng/ml; FcyRIIB at 14 ng/ml.

For the binding assessment on FcγRI, biotinylated B21M LALA IgG1 was used instead of B21M IgG1 (heavy chain SEQ ID NO: 18; light chain SEQ ID NO: 32) in order to increase the sensitivity of the assay. B21M LALA IgG1 (heavy chain SEQ ID NO: 30; light chain SEQ ID NO: 32) carries two Alanine substitutions at L234 and L235 (see also Example 1) which reduce the binding affinity to FcγRI.

After 30 min incubation, the plates were analyzed in an EnVision plate reader. %Max signal was obtained from raw EnVision data by normalization to the minimal and maximal signal, using the following equation:

$$\%Max = (Exp - Min) / (Max - Min) * 100$$

where

Exp = EnVision raw well signal
Min = Minimum signal obtained at highest competitor concentration across all tested competitors on a plate.
Max = Maximum signal, i.e. typically in the absence of competitor.

The %Max values were plotted in GraphPad Prism as mean ± standard deviation (n=3) on the y-axis, and log (inhibitor) on the x-axis. Data was fitted by nonlinear regression, using a four parameter Log (inhibitor) vs. response model with variable Hill slope,

To confirm the results of the AlphaScreen™ competition assay, binding of Fc mutated mAb1 mutants to the high-affinity FcγRI (CD64) and to the low-affinity FcyRIIIA (CD16A) was analyzed by surface plasmon resonance (SPR). A BIAcore CM5 chip was coated with 1400 RU of human recombinant FcyRIIIA (158F; R&D Systems) or with 1500 RU of human recombinant FcγRI (Sino Biological) using the BIAcore amine coupling kit (GE healthcare). Serial two-fold dilutions of Fc mutated mAb1 at concentrations between 2000 nM and 31 nM were prepared and injected at 30 μl/min in PBS pH 7.4 supplemented with 0.05% Tween-20 over the FcR coated chip surfaces and over an uncoated reference surface. The chip surface was regenerated with 10 mM NaOH between injections. The obtained binding curves were reference substracted, then buffer substracted, and the resulting double-referenced curves were evaluated using the BIAcore evaluation software, using either a 1:1 Langmuir kinetic model to obtain kinetic association and dissociation constants, $k_{on}$ and $k_{off}$, from which the thermodynamic dissociation constant $K_D$ was calculated as $k_{off}$ / $k_{on}$, or using a steady-state affinity model to directly obtain the thermodynamic dissociation constant, $K_D$.

The results of the AlphaScreen™ competition assay are shown in Fig. 4B and in Table 2. mAb1 DANAPA IgG1 showed minimal competition on FcγRI ($IC_{50}$ > 1000 nM) that is more than 400-fold reduced as compared to unmodified IgG1, indicating that this Fc sequence has minimal residual FcγRI binding activity. Unexpectedly, the DANAPA Fc showed strikingly reduced binding to FcγRI compared to the LALA Fc or the N297A Fc sequences used in clinical-stage antibodies for which minimal FcR interaction is desired. mAb1 LALA IgG1, mAb1 N297A IgG1 and mAb1 DAPA IgG1 showed reduced but still more than 37-fold stronger binding to human FcγRI than mAb1 DANAPA IgG1 ($IC_{50}$ = 27 nM, 24 nM and 18 nM).

No binding to any other human FcγR was found for mAb1 DANAPA IgG1, mAb1 DAPA IgG1, and mAb1 N297A IgG1. mAb1 LALA IgG1 was observed to bind to FcyRIIIA and very weakly to FcyRIIB.

The results of the BIAcore binding assays are shown in Fig. 4C and Table 3 (FcγRI binding), and in Fig. 4D and Table 4 (FcγRIIIA binding). Unexpectedly, mAb1 DANAPA IgG1 shows completely abrogated binding to FcγRI. mAb1 DAPA IgG1, mAb1 N297A IgG1 and mAb1 LALA IgG1 retain residual binding activity to FcγRI, albeit with reduced affinity as compared to mAb1 IgG1. mAb1 DANAPA IgG1 shows no binding to human FcγRIIIA. Similarly, mAb1 DAPA IgG1 and mAb1 N297A IgG1 show no binding, whereas mAb1 LALA IgG1 shows residual binding to FcγRIIIA, albeit with reduced activity as compared to mAb1 IgG1.

Conclusively, these results demonstrate that the DANAPA Fc sequence has strikingly reduced binding to human FcγRI, FcyRIIA, FcyRIIB and FcyRIIIA. The degree to which the DANAPA Fc sequence has reduced FcR binding activity is far superior compared to other single or combined Fc mutations previously known to lead to reduced FcγR binding activity. These results further suggest that the main difference between DANAPA Fc and the other Fc's tested here lies in the strikingly reduced binding to FcγRI.

**Table 2 - $IC_{50}$ values for competition binding to FcyRI**

| | mAb1 IgG1 | mAb1 DAPA IgG1 | mAb1 N297A IgG1 | mAb1 LALA IgG1 | mAb1 DANAPA IgG1 |
|---|---|---|---|---|---|
| $IC_{50}$ (nM) | 2.27 | 17.74 | 23.89 | 27.12 | >1000 |
| $IC_{50}$ relative to IgG1 | 1 | 7.8 | 10.5 | 11.9 | >400 |

**Table 3 - Parameters for binding to FcyRI (BIAcore)**

| Analyte | $k_{on}$ ($M^{-1}$ $s^{-1}$) | $k_{off}$ ($s^{-1}$) | $K_D$ (nM) | $K_D$ (nM) steady-state |
|---|---|---|---|---|
| mAb1 IgG1 | 6.49E+04 | 7.29E-03 | 112 | 243 |
| mAb1 DANAPA IgG1 | n.b. | n.b. | n.b. | n.b. |
| mAb1 N297A IgG1 | 1.52E+05 | 1.12E-01 | 737 | 1512 |
| mAb1 DAPA IgG1 | 1.03E+05 | 5.95E-02 | 575 | 1186 |
| mAb1 LALA IgG1 | n.d. | n.d. | n.d. | 1554 |
| n.b.: no binding observed (data was analyzed)<br>n.d.: not determined (binding observed, but kinetic data was not analyzed) | | | | |

**Table 4 - Parameters for binding to FcyRIIIA (BIAcore)**

| Analyte | $k_{on}$ ($M^{-1}$ $s^{-1}$) | $k_{off}$ ($s^{-1}$) | $K_D$ (nM) | $K_D$ (nM) steady-state |
|---|---|---|---|---|
| mAb1 IgG1 | 3.18E+04 | 2.14E-02 | 674 | 956 |
| mAb1 DANAPA IgG1 | n.b. | n.b. | n.b. | n.b. |
| mAb1 N297A IgG1 | n.b. | n.b. | n.b. | n.b. |
| mAb1 DAPA IgG1 | n.b. | n.b. | n.b. | n.b. |
| mAb1 LALA IgG1 | 1.42E+04 | 1.11E-01 | 7782 | 13828 |
| n.b.: no binding observed (data was not analyzed) | | | | |

## EXAMPLE 5: DANAPA IGG1 FC SHOWS REDUCED FCγR BINDING IN THE CONTEXT OF DIFFERENT ANTIBODY SEQUENCES

In addition to mAb1 DANAPA IgG1 presented in the previous examples above, three antibodies with different Fab sequences and different cognate targets were generated in DANAPA IgG1 format as described in Example 1 for mAb1: an anti-CD3 antibody mAb2 (heavy chain SEQ ID NO: 14; light chain SEQ ID NO: 16), an anti-HER2 antibody (heavy chain SEQ ID NO: 10; light chain SEQ ID NO: 12), and an anti-PD1 antibody (heavy chain SEQ ID NO: 6; light chain SEQ ID NO: 8). FcγR binding activity was compared to mAb1 DANAPA IgG1 in an AlphaScreen™ competition assay as described in Example 4.

The results are shown in Fig. 5. The CD3-specific antibody mAb2, the HER2-specific antibody and the PD-1 specific antibodies in DANAPA IgG1 format all show minimal binding to FcγRI ($IC_{50}$ = 600 nM or higher), and no binding to the other tested FcR. These data show that the DANAPA IgG1 sequence strongly reduces FcR binding irrespective of Fab sequence or cognate target and potentially confers minimal FcγR binding to virtually any antibody.

## EXAMPLE 6: MAB1 WITH SUBSTITUTIONS AT D265, N297, AND P329

## SHOW REDUCED BINDING TO HUMAN FCγR1

In order to determine whether the strongly reduced ability of DANAPA Fc to bind to Fc receptors can be mirrored by substituting the same set of residues (i.e. D265, N297 and P329) with amino acids different than alanine, mAb1 with the following substitutions were generated as described in Example 1:

a) D265N, N297D, P329G (referred to as DNNDPG) (heavy chain SEQ ID NO: 18; light chain SEQ ID NO: 4)
b) D265E, N297Q, P329S (referred to as DENQPS) (heavy chain SEQ ID NO: 20; light chain SEQ ID NO: 4)

The FcγRI binding activity was compared to mAb1 DANAPA IgG1 in an AlphaScreen™ competition assay as described in Example 4.

The results are shown in Fig. 6. All three antibodies show minimal binding to FcγRI, the interaction which apparently is most challenging to abrogate by Fc engineering (see Example 4). These results indicate that FcγRI interaction can be reduced by substituting the three residues, D265, N297, and P329, with different amino acid residues and not solely by substitutions with alanine.

## EXAMPLE 7: DANAPA IGG1 ABROGATES $C1_Q$ BINDING

Binding of C1q to the antibody Fc is the initial step in the induction of antibody-mediated complement activation and subsequent complement mediated target cell lysis. mAb1 DANAPA IgG1 binding to human C1q was measured in an SPR binding assay on a BIAcore T100 instrument. Antibodies were coated onto a CM5 chip via amine coupling at coating density of 5000 RU Human C1q (EMD Millipore) was injected in running buffer (PBS pH7.4, 0.05% TWEEN-20) at 200 nM and three-fold serial dilutions thereof at a flow rate of 30 μl/min. Binding was recorded, and $K_D$ was determined by curve fitting using the BIAcore software using a steady-state affinity model.

The results of the experiment are shown in Fig. 7. mAb1 IgG1 showed strong binding to C1q with an apparent $K_D$ of 30 nM, which is in agreement with published affinity values for this interaction and validates the assay set-up (Moore GL, et al. (2010), Mabs 2(2): 181-189).

mAb1 DANAPA IgG1 did not show any detectable binding to C1q.

Noteworthily, a similar Fcγ1 sequence, the DANA Fcγ1, which combines the D265A and the N297A mutations but lacks the P329A mutation present in DANAPA Fcγ1, has been described in literature to show residual C1q binding (Gong, Q, et al. (2005), J Immunol 174(2): 817-826).

In contrast to DANA Fcγ1, mAb1 DANAPA IgG1 strikingly demonstrated complete loss of binding to C1q.

## EXAMPLE 8: DANAPA MUTATIONS DO NOT IMPAIR BINDING TO HUMAN FCRN

The interaction of IgG Fc with FcRn plays an important role in antibody turnover (Kuo TT and VG Aveson (2011), MAbs 3(5): 422-430). IgG that have been taken up by cells via pinocytosis engage with the FcRn receptor in the acidic environment of the endosomes. FcRn recycles the IgG back to the cell surface where the antibody dissociates from FcRn at neutral or basic pH and thus is rescued from lysosomal degradation. This mechanism provides an explanation for the long serum half-life of IgG. Therefore, in order to have a long circulation half-life, it is important that antibodies with substitutions in the Fc retain full binding to FcRn at acidic pH and readily dissociate at neutral pH.

While human FcγR bind to residues in the lower hinge and to the CH2 domain of IgG antibodies (Woof JM and DR Burton (2004), Nat Rev Immunol 4(2): 89-99), human FcRn interacts with several residues in the CH2-CH3 interface (Martin WL, et al. (2001), Mol Cell 7(4): 867-877). Therefore, mutations introduced with the aim to reduce FcR binding may have an impact on the Fc - FcRn interaction. For instance, Shields et al. observed that some mutations in the lower hinge or the CH2 domain that led to reduced FcγR binding also resulted in reduced FcRn binding (e.g. E233P, Q295A). Therefore, it is of particular importance to assess the impact of the DANAPA mutations on FcRn binding.

Binding of DANAPA IgG1 to FcRn was analyzed by surface plasmon resonance (SPR). A BIAcore CM5 chip was coated with 600 RU of human recombinant FcRn (Sino Biological) using the BIAcore amine coupling kit (GE healthcare). Serial two-fold dilutions of DANAPA Fc mutated mAb1 and of unmutated mAb1 IgG1 at concentrations between 2000 nM and 31 nM were prepared and injected at 30 μl/min in PBS pH 6.0 supplemented with 0.05% Tween-20 over the FcRn-coated and over an uncoated reference surface. Between injections, the chip surface was regenerated with PBS pH 7.4. The obtained binding curves were reference substracted, then buffer substracted, and the resulting double-referenced curves were evaluated using the BIAcore evaluation software, using a steady-state affinity model to obtain the thermodynamic dissociation constant, $K_D$.

The results of the binding assay to human FcRn are shown in Fig. 8. The dissociation constant, $K_D$, was 500 nM for mAb1 DANAPA IgG1, and 470 nM for mAb1 IgG1, indicating that there is no difference in binding to human FcRn. mAb1 DANAPA IgG1 shows rapid dissociation at neutral pH with essentially identical dissociation kinetics as mAb1 IgG1. These results suggest that mAb1 DANAPA IgG1 retains IgG1-like binding to FcRn despite abrogated binding to FcγR.

## EXAMPLE 9: MAB1 DANAPA IGG1 EXHIBITS IGG1-LIKE PHARMACOKINETIC PROFILE

Good pharmacokinetics properties, i.e. a long half-life in the circulation, is one of the key criteria that an antibody-based pharmaceutical product must meet. Engineering of antibody Fc sequences may have unexpected effects on the pharmacokinetic profile. For example, antibodies with an Fc sequence containing five mutations to reduce Fc receptor binding ("LFLEDANQPS" [note: the last P to S mutation being at position 331 according to Kabat numbering, i.e. at a position differing from the mutants in the instant disclosure]) had a 3- to 5-fold increased clearance compared to a wild-type IgG1, resulting in a shorter terminal half-life than the corresponding wild-type IgG1 (WO2014108483).

The pharmacokinetic profile of mAb1 DANAPA IgGI in C57BL/6 mice (Charles River) was investigated and compared to mAb1 IgG1. Five C57BL/6 mice were injected i.v. with 10 mg/kg mAb1 DANAPA IgGI or mAb1 IgG1. After 10 min, 6, 24, 48, 96, 120, 144, 168, 192 and 216 hours, blood was collected into EDTA coated microvettes (Sarstedt), centrifuged for 10 min at 9300 g and the serum levels of mAb1 DANAPA IgGI and of mAb1 IgGI were determined by an Fc specific sandwich ELISA. Transparent maxisorp microtiter plates (Nunc) were coated with 440-fold diluted Fc-specific anti-human IgG1 capture antibody (12134, Sigma). After blocking with 2 % BSA (Sigma) in PBS, 40 $\mu$l of PBS and 10 $\mu$l of plasma at appropriate dilutions were applied. After incubation for 1 h, wells were washed with PBS, and bound mAb1 DANAPA IgGI or mAb1 IgGI was detected with 10'000-fold diluted Fc-specific HRP conjugated anti-human IgG1 detection antibody (A0170, Sigma). The assay was developed with QuantaRed fluorogenic substrate (Pierce) and the fluorescence intensity was measured after 2 to 4 min at 544 nm (excitation) and 590 nm (emission). The plasma levels of mAb1 DANAPA IgGI and mAb1 IgGI were determined using a standard curve of the respective antibodies. Antibody exposure in the plasma is presented in a semi-logarithmic plot over a period of 216 hours.

The pharmacokinetic profiles of mAb1 DANAPA IgGI and of mAb1 IgGI are shown in Fig. 9. Importantly and unpredictably before the instant invention, mAb1 DANAPA IgG1 has pharmacokinetic properties that are essentially identical to mAb1 IgGI.

## EXAMPLE 10: CD33 X CD3 BISPECIFIC FYNOMABS WITH DANAPA IGG1 FC SHOW REDUCED FCγR BINDING

Upon interaction with Fc receptor expressing cells, Fc-containing bispecific T cell engaging molecules that have a first binding site specific to a tumor antigen and a second binding site specific for T cells may induce tumor-independent T cell activation and cytokine release (off-tumor effect) (see WO2012143524). In order to mitigate this risk, such molecules can be equipped with engineered Fc sequences that have minimal intrinsic FcγR affinity.

CD3/CD33 bispecific FynomAbs were generated by fusion of the CD33-specific Fynomer G1 (SEQ ID NO: 36) or the CD33-specific Fynomer D5 (SEQ ID NO: 38) to the C-terminus of the light chain of a humanized CD3 specific antibody mAb2 (heavy chain SEQ ID NO: 14; light chain SEQ ID NO: 16), using a flexible $(G_4S)_3$ linker (SEQ ID NO: 40). FcγR binding of these FynomAbs was determined in an AlphaScreen™ competition assay as described in Example 4.

FcγR binding of the CD3/CD33 FynomAbs was compared to COVA467 (heavy chain SEQ ID NO: 26; light chain SEQ ID NO: 28), a previously described CD3/CD33 FynomAb with a LALA IgG1 Fc (i.e. an IgG1 Fc having the L234A and L235A mutations) that was generated by fusing the CD33-specific Fynomer B3 to the C-terminus of the CD3 specific antibody mAb3 light chain (see WO2014170063). B21M IgG1 served as positive control (see Example 4).

The results are shown in Fig. 10. Whereas COVA467 showed residual binding to human FcγRIIIA and FcγRI ($IC_{50}$ = 390 nM and 29 nM, respectively), the two FynomAbs with the DANAPA IgGI Fc did not show any binding to FcyRIIIA and strongly reduced binding to FcγRI, compared to COVA467 ($IC_{50}$ = 206 nM or 800 nM, respectively). No significant binding to FcγRII A and B was found for the constructs. These results demonstrate that the CD3/CD33 bispecific FynomAbs with a DANAPA IgG1 Fc show reduced FcγR binding as compared to COVA467. Therefore, they have a reduced potential to induce undesired off-tumor T cell activation and cytokine release and represent improved mutants of CD3/CD33 bispecific FynomAbs.

**Table 5 - Sequences**

| SEQ ID NO: | Description | Sequence |
|---|---|---|
| 1 | mAb1 IgG1 HC (DNA) | CAGGTCCAGCTGCAGCAGAGTGGGGCCGAACTGGCAAGACCC<br>GGAGCAAGCGTCAAAATGTCATGTAAAGCAAGCGGTTATACT<br>TTCACTAGGAGCACCATGCACTGGGTGAAACAGAGGCCCGGC<br>CAGGGACTGGAGTGGATCGGGTACATTAACCCTTCCAGCGCT<br>TACACCAACTATAATCAGAAGTTCAAAGACAAGGCCACCCTG<br>ACAGCTGATAAGTCTAGTTCAACAGCATATATGCAGCTGTCC<br>AGCCTGACTTCTGAAGACAGTGCAGTGTACTATTGCGCCTCC<br>CCACAGGTCCACTACGATTACAATGGTTTTCCTTACTGGGGG<br>CAGGGCACACTGGTGACTGTCTCCGCCGCTAGCACAAAGGGC<br>CCTAGTGTGTTTCCTCTGGCTCCCTCTTCCAAATCCACTTCT<br>GGTGGCACTGCTGCTCTGGGATGCCTGGTGAAGGATTACTTT<br>CCTGAACCTGTGACTGTCTCATGGAACTCTGGTGCTCTGACT<br>TCTGGTGTCCACACTTTCCCTGCTGTGCTGCAGTCTAGTGGA<br>CTGTACTCTCTGTCATCTGTGGTCACTGTGCCCTCTTCATCT<br>CTGGGAACCCAGACCTACATTTGTAATGTGAACCACAAACCA<br>TCCAACACTAAAGTGGACAAAAAAGTGGAACCCAAATCCTGT<br>GACAAAACCCACACCTGCCCACCTTGTCCTGCCCCTGAACTG<br>CTGGGAGGACCTTCTGTGTTTCTGTTCCCCCCCAAACCAAAG<br>GATACCCTGATGATCTCTAGAACCCCTGAGGTGACATGTGTG<br>GTGGTGGATGTGTCTCATGAGGACCCTGAGGTCAAATTCAAC<br>TGGTACGTGGATGGAGTGGAAGTCCACAATGCCAAAACCAAG<br>CCTAGAGAGGAACAGTACAATTCAACCTACAGAGTTGTCAGT<br>GTGCTGACTGTGCTGCATCAGGATTGGCTGAATGGCAAGGAA<br>TACAAGTGTAAAGTCTCAAACAAGGCCCTGCCTGCTCCAATT<br>GAGAAAACAATCTCAAAGGCCAAGGGACAGCCTAGGGAACCC<br>CAGGTCTACACCCTGCCACCTTCAAGAGAGGAAATGACCAAA<br>AACCAGGTGTCCCTGACATGCCTGGTCAAAGGCTTCTACCCT<br>TCTGACATTGCTGTGGAGTGGGAGTCAAATGGACAGCCTGAG<br>AACAACTACAAAACAACCCCCCCTGTGCTGGATTCTGATGGC<br>TCTTTCTTTCTGTACTCCAAACTGACTGTGGACAAGTCTAGA<br>TGGCAGCAGGGGAATGTCTTTTCTTGCTCTGTCATGCATGAG<br>GCTCTGCATAACCACTACACTCAGAAATCCCTGTCTCTGTCT<br>CCCGGGAAATGA |
| 2 | mAb1 IgG1 HC (protein) | QVQLQQSGAELARPGASVKMSCKASGYTFTRSTMHWVKQRPG<br>QGLEWIGYINPSSAYTNYNQKFKDKATLTADKSSSTAYMQLS<br>SLTSEDSAVYYCASPQVHYDYNGFPYWGQGTLVTVSAASTKG<br>PSVFPLAPSSKSTSGGTAALGCLVKDYFPEPVTVSWNSGALT<br>SGVHTFPAVLQSSGLYSLSSVVTVPSSSLGTQTYICNVNHKP<br>SNTKVDKKVEPKSCDKTHTCPPCPAPELLGGPSVFLFPPKPK<br>DTLMISRTPEVTCVVVDVSHEDPEVKFNWYVDGVEVHNAKTK<br>PREEQYNSTYRVVSVLTVLHQDWLNGKEYKCKVSNKALPAPI<br>EKTISKAKGQPREPQVYTLPPSREEMTKNQVSLTCLVKGFYP<br>SDIAVEWESNGQPENNYKTTPPVLDSDGSFFLYSKLTVDKSR<br>WQQGNVFSCSVMHEALHNHYTQKSLSLSPGK |
| 3 | mAb1, mAb1 DANAPA, mAb1 DAPA, mAb1 N297A, | CAGGTGGTGCTGACCCAGAGCCCTGCTATTATGTCCGCATTC<br>CCCGGTGAAAAAGTGACTATGACTTGTTCCGCTTCTTCCTCC<br>GTCTCCTACATGAACTGGTATCAGCAGAAGTCAGGAACATCT<br>CCCAAAAGGTGGATCTACGACTCCAGCAAGCTGGCATCCGGC |

(continued)

| SEQ ID NO: | Description | Sequence |
|---|---|---|
| | mAb1 DNNDPG, mAb1 DENQPS, and mAb1 LALA-IgG1 LC (DNA) | GTGCCTGCACGATTCTCAGGCTCCGGAAGCGGGACCTCTTAT AGTCTGACAATTTCTAGTATGGAGACTGAAGATGCCGCTACC TACTATTGCCAGCAGTGGTCAAGAAACCCTCCAACATTCGGG GGGGGACTAAACTGCAGATTACTCGTACGGTCGCGGCGCCT TCTGTGTTCATTTTCCCCCCATCTGATGAACAGCTGAAATCT GGCACTGCTTCTGTGGTCTGTCTGCTGAACAACTTCTACCCT AGAGAGGCCAAAGTCCAGTGGAAAGTGGACAATGCTCTGCAG AGTGGGAATTCCCAGGAATCTGTCACTGAGCAGGACTCTAAG GATAGCACATACTCCCTGTCCTCTACTCTGACACTGAGCAAG GCTGATTACGAGAAACACAAAGTGTACGCCTGTGAAGTCACA CATCAGGGGCTGTCTAGTCCTGTGACCAAATCCTTCAATAGG GGAGAGTGCTGA |
| 4 | mAb1, mAb1 DANAPA, mAb1 DAPA, mAb1 N297A, mAb1 DNNDPG, mAb1 DENQPS, and mAb1 LALA-IgG1 LC (protein) | QVVLTQSPAIMSAFPGEKVTMTCSASSSVSYMNWYQQKSGTS PKRWIYDSSKLASGVPARFSGSGSGTSYSLTISSMETEDAAT YYCQQWSRNPPTFGGGTKLQITRTVAAPSVFIFPPSDEQLKS GTASVVCLLNNFYPREAKVQWKVDNALQSGNSQESVTEQDSK DSTYSLSSTLTLSKADYEKHKVYACEVTHQGLSSPVTKSFNR GEC |
| 5 | Anti-PD1 mAb DANAPA-IgG1 HC (DNA) | CAGGTGCAGCTCCAGCAGAGTGGCGCAGAGCTGGTGAAGCCC GGAGCCTCAGTCAAGATGTCCTGCAAGGCCTTCGGCTACACT TTTACCACATATCCTATCGAGTGGATGAAGCAGAACCACGGG AAAAGCCTGGAATGGATTGGTAACTTCCATCCATACAATGAC GATACCAAGTATAATGAGAAGTTTAAAGGCAAGGCAAAACTG ACAGTGGAGAAATCCAGCACTACCGTCTACCTGGAACTGTCC AGGCTGACATCTGACGATAGTGCCGTGTACTATTGTGCTCGG GAAAACTACGGAAGCCACGGCGGATTCGTCTATTGGGGGCAG GGTACACTGGTGACTGTCTCTGCCGCTAGCACAAAGGGCCCT AGTGTGTTTCCTCTGGCTCCCTCTTCCAAATCCACTTCTGGT GGCACTGCTGCTCTGGGATGCCTGGTGAAGGATTACTTTCCT GAACCTGTGACTGTCTCATGGAACTCTGGTGCTCTGACTTCT GGTGTCCACACTTTCCCTGCTGTGCTGCAGTCTAGTGGACTG TACTCTCTGTCATCTGTGGTCACTGTGCCCTCTTCATCTCTG GGAACCCAGACCTACATTTGTAATGTGAACCACAAACCATCC AACACTAAAGTGGACAAAAAAGTGGAACCCAAATCCTGTGAC AAAACCCACACCTGCCCACCTTGTCCTGCCCCTGAACTGCTG GGAGGACCTTCTGTGTTTCTGTTCCCCCCCAAACCAAAGGAT ACCCTGATGATCTCTAGAACCCCTGAGGTGACATGTGTGGTG GTGGCTGTGTCTCATGAGGACCCTGAGGTCAAATTCAACTGG TACGTGGATGGAGTGGAAGTCCACAATGCCAAAACCAAGCCT AGAGAGGAACAGTACGCTTCAACCTACAGAGTTGTCAGTGTG CTGACTGTGCTGCATCAGGATTGGCTGAATGGCAAGGAATAC AAGTGTAAAGTCTCAAACAAGGCCCTGGCTGCTCCAATTGAG AAAACAATCTCAAAGGCCAAGGGACAGCCTAGGGAACCCCAG GTCTACACCCTGCCACCTTCAAGAGAGGAAATGACCAAAAAC CAGGTGTCCCTGACATGCCTGGTCAAAGGCTTCTACCCTTCT GACATTGCTGTGGAGTGGGAGTCAAATGGACAGCCTGAGAAC AACTACAAAACAACCCCCCCTGTGCTGGATTCTGATGGCTCT TTCTTTCTGTACTCCAAACTGACTGTGGACAAGTCTAGATGG CAGCAGGGGAATGTCTTTTCTTGCTCTGTCATGCATGAGGCT |

(continued)

| SEQ ID NO: | Description | Sequence |
|---|---|---|
| | | CTGCATAACCACTACACTCAGAAATCCCTGTCTCTGTCTCCC GGGAAATGA |
| 6 | Anti-PD1 mAb DANAPA IgG1 HC (protein) | QVQLQQSGAELVKPGASVKMSCKAFGYTFTTYPIEWMKQNHG KSLEWIGNFHPYNDDTKYNEKFKGKAKLTVEKSSTTVYLELS RLTSDDSAVYYCARENYGSHGGFVYWGQGTLVTVSAASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVAVSHEDPEVKFNWYVDGVEVHNAKTKP REEQYASTYRVVSVLTVLHQDWLNGKEYKCKVSNKALAAPIE KTISKAKGQPREPQVYTLPPSREEMTKNQVSLTCLVKGFYPS DIAVEWESNGQPENNYKTTPPVLDSDGSFFLYSKLTVDKSRW QQGNVFSCSVMHEALHNHYTQKSLSLSPGK |
| 7 | Anti-PD1 mAb DANAPA IgG1 LC (DNA) | GAGAACGTGCTGACCCAGTCCCCCGCAATCATGTCTGCCAGT CCTGGAGAAAAGGTCACCATGACATGCAGGGCATCCAGCTCT GTCATCAGTTCATACCTGCACTGGTATCAGCAGAAGAGCGGA GCTTCTCCAAAACTGTGGATCTACTCAACCTCCAACCTGGCA AGCGGGGTGCCCGACCGGTTCAGCGGCTCTGGAAGTGGGACT TCATATAGTCTGACCATCTCGTCGGTCGAGGCCGAAGATGCC GCTACATACTATTGTCAGCAGTACAATGGCTATCCCCTGACA TTTGGTGCTGGTACCAAACTCGAGATTAAGCGTACGGTCGCG GCGCCTTCTGTGTTCATTTTCCCCCCATCTGATGAACAGCTG AAATCTGGCACTGCTTCTGTGGTCTGTCTGCTGAACAACTTC TACCCTAGAGAGGCCAAAGTCCAGTGGAAAGTGGACAATGCT CTGCAGAGTGGGAATTCCCAGGAATCTGTCACTGAGCAGGAC TCTAAGGATAGCACATACTCCCTGTCCTCTACTCTGACACTG AGCAAGGCTGATTACGAGAAACACAAAGTGTACGCCTGTGAA GTCACACATCAGGGGCTGTCTAGTCCTGTGACCAAATCCTTC AATAGGGGAGAGTGCTGA |
| 8 | Anti-PD1 mAb DANAPA IgG1 LC (protein) | ENVLTQSPAIMSASPGEKVTMTCRASSSVISSYLHWYQQKSG ASPKLWIYSTSNLASGVPDRFSGSGSGTSYSLTISSVEAEDA ATYYCQQYNGYPLTFGAGTKLEIKRTVAAPSVFIFPPSDEQL KSGTASVVCLLNNFYPREAKVQWKVDNALQSGNSQESVTEQD SKDSTYSLSSTLTLSKADYEKHKVYACEVTHQGLSSPVTKSF NRGEC |

(continued)

| SEQ ID NO: | Description | Sequence |
|---|---|---|
| 9 | Anti-HER2 mAb DANAPA IgG1 HC (DNA) | GAGGTTCAGCTGGTGGAGTCTGGCGGTGGCCTGGTGCAGCCA<br>GGGGGCTCACTCCGTTTGTCCTGTGCAGCTTCTGGCTTCAAC<br>ATTAAAGACACCTATATACACTGGGTGCGTCAGGCCCCGGGT<br>AAGGGCCTGGAATGGGTTGCAAGGATTTATCCTACGAATGGT<br>TATACTAGATATGCCGATAGCGTCAAGGGCCGTTTCACTATA<br>AGCGCAGACACATCCAAAAACACAGCCTACCTGCAGATGAAC<br>AGCCTGCGTGCTGAGGACACTGCCGTCTATTATTGTTCTAGA<br>TGGGGAGGGGACGGCTTCTATGCTATGGACTACTGGGGTCAA<br>GGAACCCTGGTCACCGTCTCCTCGGCTAGCACAAAGGGCCCT<br>AGTGTGTTTCCTCTGGCTCCCTCTTCCAAATCCACTTCTGGT<br>GGCACTGCTGCTCTGGGATGCCTGGTGAAGGATTACTTTCCT<br>GAACCTGTGACTGTCTCATGGAACTCTGGTGCTCTGACTTCT<br>GGTGTCCACACTTTCCCTGCTGTGCTGCAGTCTAGTGGACTG<br>TACTCTCTGTCATCTGTGGTCACTGTGCCCTCTTCATCTCTG<br>GGAACCCAGACCTACATTTGTAATGTGAACCACAAACCATCC<br>AACACTAAAGTGGACAAAAAAGTGGAACCCAAATCCTGTGAC<br>AAAACCCACACCTGCCCACCTTGTCCTGCCCCTGAACTGCTG<br>GGAGGACCTTCTGTGTTTCTGTTCCCCCCCAAACCAAAGGAT<br>ACCCTGATGATCTCTAGAACCCCTGAGGTGACATGTGTGGTG<br>GTGGCTGTGTCTCATGAGGACCCTGAGGTCAAATTCAACTGG<br>TACGTGGATGGAGTGGAAGTCCACAATGCCAAAACCAAGCCT<br>AGAGAGGAACAGTACGCTTCAACCTACAGAGTTGTCAGTGTG<br>CTGACTGTGCTGCATCAGGATTGGCTGAATGGCAAGGAATAC<br>AAGTGTAAAGTCTCAAACAAGGCCCTGGCTGCTCCAATTGAG<br>AAAACAATCTCAAAGGCCAAGGGACAGCCTAGGGAACCCCAG<br>GTCTACACCCTGCCACCTTCAAGAGAGGAAATGACCAAAAAC<br>CAGGTGTCCCTGACATGCCTGGTCAAAGGCTTCTACCCTTCT<br>GACATTGCTGTGGAGTGGGAGTCAAATGGACAGCCTGAGAAC<br>AACTACAAAACAACCCCCCCTGTGCTGGATTCTGATGGCTCT<br>TTCTTTCTGTACTCCAAACTGACTGTGGACAAGTCTAGATGG<br>CAGCAGGGGAATGTCTTTTCTTGCTCTGTCATGCATGAGGCT<br>CTGCATAACCACTACACTCAGAAATCCCTGTCTCTGTCTCCC<br>GGGAAATGA |
| 10 | Anti-HER2 mAb DANAPA IgG1 HC (protein) | EVQLVESGGGLVQPGGSLRLSCAASGFNIKDTYIHWVRQAPG<br>KGLEWVARIYPTNGYTRYADSVKGRFTISADTSKNTAYLQMN<br>SLRAEDTAVYYCSRWGGDGFYAMDYWGQGTLVTVSSASTKGP<br>SVFPLAPSSKSTSGGTAALGCLVKDYFPEPVTVSWNSGALTS<br>GVHTFPAVLQSSGLYSLSSVVTVPSSSLGTQTYICNVNHKPS<br>NTKVDKKVEPKSCDKTHTCPPCPAPELLGGPSVFLFPPKPKD<br>TLMISRTPEVTCVVVAVSHEDPEVKFNWYVDGVEVHNAKTKP<br>REEQYASTYRVVSVLTVLHQDWLNGKEYKCKVSNKALAAPIE<br>KTISKAKGQPREPQVYTLPPSREEMTKNQVSLTCLVKGFYPS<br>DIAVEWESNGQPENNYKTTPPVLDSDGSFFLYSKLTVDKSRW<br>QQGNVFSCSVMHEALHNHYTQKSLSLSPGK |

(continued)

| SEQ ID NO: | Description | Sequence |
|---|---|---|
| 11 | Anti-HER2 mAb DANAPA IgG1 LC (DNA) | GATATCCAGATGACCCAGTCCCCGAGCTCCCTGTCCGCCTCT GTGGGCGATAGGGTCACCATCACCTGCCGTGCCAGTCAGGAT GTGAATACTGCTGTAGCCTGGTATCAACAGAAACCAGGAAAA GCTCCGAAACTACTGATTTACTCGGCATCCTTCCTCTACTCT GGAGTCCCTTCTCGCTTCTCTGGGTCCAGATCTGGGACGGAT TTCACTCTGACCATCAGCAGTCTGCAGCCGGAAGACTTCGCA ACTTATTACTGTCAGCAACATTATACTACTCCTCCCACGTTC GGACAGGGGACCAAGGTGGAGATCAAACGTACGGTCGCGGCG CCTTCTGTGTTCATTTTCCCCCCATCTGATGAACAGCTGAAA TCTGGCACTGCTTCTGTGGTCTGTCTGCTGAACAACTTCTAC CCTAGAGAGGCCAAAGTCCAGTGGAAAGTGGACAATGCTCTG CAGAGTGGGAATTCCCAGGAATCTGTCACTGAGCAGGACTCT AAGGATAGCACATACTCCCTGTCCTCTACTCTGACACTGAGC AAGGCTGATTACGAGAAACACAAAGTGTACGCCTGTGAAGTC ACACATCAGGGGCTGTCTAGTCCTGTGACCAAATCCTTCAAT AGGGGAGAGTGCTGA |
| 12 | Anti-HER2 mAb DANAPA IgG1 LC (protein) | DIQMTQSPSSLSASVGDRVTITCRASQDVNTAVAWYQQKPGK APKLLIYSASFLYSGVPSRFSGSRSGTDFTLTISSLQPEDFA TYYCQQHYTTPPTFGQGTKVEIKRTVAAPSVFIFPPSDEQLK SGTASVVCLLNNFYPREAKVQWKVDNALQSGNSQESVTEQDS KDSTYSLSSTLTLSKADYEKHKVYACEVTHQGLSSPVTKSFN RGEC |
| 13 | mAb2 DANAPA, mAb2 D5 C-LC DANAPA, and mAb2 G1 C-LC DANAPA-IgG1 HC (DNA) | GAGGTGCAGCTGGTCGAGTCTGGAGGAGGATTGGTGCAGCCT GGAGGGTCATTGAAACTCTCATGTGCAGCCTCTGGATTCACC TTCAATACCTACGCCATGAACTGGGTCCGCCAGGCTCCAGGA AAGGGTTTGGAATGGGTTGCTCGCATAAGAAGTAAATATAAT AATTATGCAACATATTATGCCGATTCAGTGAAAGACAGGTTC ACCATCTCCAGAGATGATTCAAAAAACACTGCCTATCTACAA ATGAACAGCTTGAAAACTGAGGACACTGCCGTGTACTACTGT |

(continued)

| SEQ ID NO: | Description | Sequence |
|---|---|---|
| | | GTGAGACATGGGAACTTCGGTGATAGCTACGTTTCCTGGTTT GCTTACTGGGGCCAAGGGACTCTGGTCACCGTCTCGAGCGCT AGCACAAAGGGCCCTAGTGTGTTTCCTCTGGCTCCCTCTTCC AAATCCACTTCTGGTGGCACTGCTGCTCTGGGATGCCTGGTG AAGGATTACTTTCCTGAACCTGTGACTGTCTCATGGAACTCT GGTGCTCTGACTTCTGGTGTCCACACTTTCCCTGCTGTGCTG CAGTCTAGTGGACTGTACTCTCTGTCATCTGTGGTCACTGTG CCCTCTTCATCTCTGGGAACCCAGACCTACATTTGTAATGTG AACCACAAACCATCCAACACTAAAGTGGACAAAAAAGTGGAA CCCAAATCCTGTGACAAAACCCACACCTGCCCACCTTGTCCT GCCCCTGAACTGCTGGGAGGACCTTCTGTGTTTCTGTTCCCC CCCAAACCAAAGGATACCCTGATGATCTCTAGAACCCCTGAG GTGACATGTGTGGTGGTGGCTGTGTCTCATGAGGACCCTGAG GTCAAATTCAACTGGTACGTGGATGGAGTGGAAGTCCACAAT GCCAAAACCAAGCCTAGAGAGGAACAGTACGCTTCAACCTAC AGAGTTGTCAGTGTGCTGACTGTGCTGCATCAGGATTGGCTG AATGGCAAGGAATACAAGTGTAAAGTCTCAAACAAGGCCCTG GCTGCTCCAATTGAGAAAACAATCTCAAAGGCCAAGGGACAG CCTAGGGAACCCCAGGTCTACACCCTGCCACCTTCAAGAGAG GAAATGACCAAAAACCAGGTGTCCCTGACATGCCTGGTCAAA GGCTTCTACCCTTCTGACATTGCTGTGGAGTGGGAGTCAAAT GGACAGCCTGAGAACAACTACAAAACAACCCCCCCTGTGCTG GATTCTGATGGCTCTTTCTTTCTGTACTCCAAACTGACTGTG GACAAGTCTAGATGGCAGCAGGGGAATGTCTTTTCTTGCTCT GTCATGCATGAGGCTCTGCATAACCACTACACTCAGAAATCC CTGTCTCTGTCTCCCGGGAAATGA |
| 14 | mAb2 DANAPA, mAb2 D5 C-LC DANAPA, and mAb2 G1 C-LC DANAPA-IgG1 HC (protein) | EVQLVESGGGLVQPGGSLKLSCAASGFTFNTYAMNWVRQAPG KGLEWVARIRSKYNNYATYYADSVKDRFTISRDDSKNTAYLQ MNSLKTEDTAVYYCVRHGNFGDSYVSWFAYWGQGTLVTVSSA STKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFLFP PKPKDTLMISRTPEVTCVVVAVSHEDPEVKFNWYVDGVEVHN AKTKPREEQYASTYRVVSVLTVLHQDWLNGKEYKCKVSNKAL AAPIEKTISKAKGQPREPQVYTLPPSREEMTKNQVSLTCLVK GFYPSDIAVEWESNGQPENNYKTTPPVLDSDGSFFLYSKLTV DKSRWQQGNVFSCSVMHEALHNHYTQKSLSLSPGK |

(continued)

| SEQ ID NO: | Description | Sequence |
|---|---|---|
| 15 | mAb2 DANAPA IgG1 LC (DNA) | CAGACCGTTGTGACTCAGGAACCTTCACTCACCGTATCACCT<br>GGTGGAACAGTCACACTCACTTGTCGCTCGTCGACTGGGGCT<br>GTTACAACTAGCAACTATGCCAACTGGGTCCAACAAAAACCG<br>GGTCAGGCACCCCGTGGTCTAATAGGTGGTACCAACAAGCGC<br>GCACCAGGTACTCCTGCCAGATTCTCAGGCTCCCTGCTTGGA<br>GGCAAGGCTGCCCTCACCCTCTCGGGGGTACAGCCAGAGGAT<br>GAGGCAGAATATTACTGTGCTCTATGGTACAGCAACCTCTGG<br>GTGTTCGGTGGAGGAACCAAACTGACTGTCCTAGGCCAGCCT<br>AAAGCGGCGCCATCCGTCACCCTGTTCCCTCCCTCATCCGAG<br>GAACTGCAGGCCAATAAGGCTACACTGGTCTGTCTGATTAGC<br>GACTTCTACCCTGGGGCCGTGACTGTGGCTTGGAAAGCCGAT<br>TCTTCTCCCGTGAAAGCTGGAGTGGAAACAACCACCCCCTCT<br>AAACAGAGCAACAACAAATACGCTGCCTCTTCATACCTGTCC<br>CTGACCCCTGAACAGTGGAAATCTCACCGGTCTTACTCATGC<br>CAGGTGACACACGAGGGATCAACTGTGGAGAAAACCGTGGCT<br>CCTACCGAATGTTCATGA |
| 16 | mAb2 DANAPA IgG1 LC (protein) | QTVVTQEPSLTVSPGGTVTLTCRSSTGAVTTSNYANWVQQKP<br><br>GQAPRGLIGGTNKRAPGTPARFSGSLLGGKAALTLSGVQPED<br>EAEYYCALWYSNLWVFGGGTKLTVLGQPKAAPSVTLFPPSSE<br>ELQANKATLVCLISDFYPGAVTVAWKADSSPVKAGVETTTPS<br>KQSNNKYAASSYLSLTPEQWKSHRSYSCQVTHEGSTVEKTVA<br>PTECS |

(continued)

| SEQ ID NO: | Description | Sequence |
|---|---|---|
| 17 | mAb1 DNNDPG IgG1 HC (DNA) | CAGGTCCAGCTGCAGCAGAGTGGGGCCGAACTGGCAAGACCC GGAGCAAGCGTCAAAATGTCATGTAAAGCAAGCGGTTATACT TTCACTAGGAGCACCATGCACTGGGTGAAACAGAGGCCCGGC CAGGGACTGGAGTGGATCGGGTACATTAACCCTTCCAGCGCT TACACCAACTATAATCAGAAGTTCAAAGACAAGGCCACCCTG ACAGCTGATAAGTCTAGTTCAACAGCATATATGCAGCTGTCC AGCCTGACTTCTGAAGACAGTGCAGTGTACTATTGCGCCTCC CCACAGGTCCACTACGATTACAATGGTTTTCCTTACTGGGGG CAGGGCACACTGGTGACTGTCTCCGCCGCTAGCACAAAGGGC CCTAGTGTGTTTCCTCTGGCTCCCTCTTCCAAATCCACTTCT GGTGGCACTGCTGCTCTGGGATGCCTGGTGAAGGATTACTTT CCTGAACCTGTGACTGTCTCATGGAACTCTGGTGCTCTGACT TCTGGTGTCCACACTTTCCCTGCTGTGCTGCAGTCTAGTGGA CTGTACTCTCTGTCATCTGTGGTCACTGTGCCCTCTTCATCT CTGGGAACCCAGACCTACATTTGTAATGTGAACCACAAACCA TCCAACACTAAAGTGGACAAAAAAGTGGAACCCAAATCCTGT GACAAAACCCACACCTGCCCACCTTGTCCTGCCCCTGAACTG CTGGGAGGACCTTCTGTGTTTCTGTTCCCCCCCAAACCAAAG GATACCCTGATGATCTCTAGAACCCCTGAGGTGACATGTGTG GTGGTGAATGTGTCTCATGAGGACCCTGAGGTCAAATTCAAC TGGTACGTGGATGGAGTGGAAGTCCACAATGCCAAAACCAAG CCTAGAGAGGAACAGTACGATTCAACCTACAGAGTTGTCAGT GTGCTGACTGTGCTGCATCAGGATTGGCTGAATGGCAAGGAA TACAAGTGTAAAGTCTCAAACAAGGCCCTGGGTGCTCCAATT GAGAAAACAATCTCAAAGGCCAAGGGACAGCCTAGGGAACCC CAGGTCTACACCCTGCCACCTTCAAGAGAGGAAATGACCAAA AACCAGGTGTCCCTGACATGCCTGGTCAAAGGCTTCTACCCT TCTGACATTGCTGTGGAGTGGGAGTCAAATGGACAGCCTGAG AACAACTACAAAACAACCCCCCCTGTGCTGGATTCTGATGGC TCTTTCTTTCTGTACTCCAAACTGACTGTGGACAAGTCTAGA TGGCAGCAGGGGAATGTCTTTTCTTGCTCTGTCATGCATGAG GCTCTGCATAACCACTACACTCAGAAATCCCTGTCTCTGTCT CCCGGGAAATGA |
| 18 | mAb1 DNNDPG IgG1 HC (protein) | QVQLQQSGAELARPGASVKMSCKASGYTFTRSTMHWVKQRPG QGLEWIGYINPSSAYTNYNQKFKDKATLTADKSSSTAYMQLS SLTSEDSAVYYCASPQVHYDYNGFPYWGQGTLVTVSAASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVNVSHEDPEVKFNWYVDGVEVHNAKTK PREEQYDSTYRVVSVLTVLHQDWLNGKEYKCKVSNKALGAPI EKTISKAKGQPREPQVYTLPPSREEMTKNQVSLTCLVKGFYP SDIAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 19 | mAb1 DENQPS IgG1 HC (DNA) | CAGGTCCAGCTGCAGCAGAGTGGGGCCGAACTGGCAAGACCC GGAGCAAGCGTCAAAATGTCATGTAAAGCAAGCGGTTATACT TTCACTAGGAGCACCATGCACTGGGTGAAACAGAGGCCCGGC CAGGGACTGGAGTGGATCGGGTACATTAACCCTTCCAGCGCT TACACCAACTATAATCAGAAGTTCAAAGACAAGGCCACCCTG ACAGCTGATAAGTCTAGTTCAACAGCATATATGCAGCTGTCC AGCCTGACTTCTGAAGACAGTGCAGTGTACTATTGCGCCTCC CCACAGGTCCACTACGATTACAATGGTTTTCCTTACTGGGGG CAGGGCACACTGGTGACTGTCTCCGCCGCTAGCACAAAGGGC CCTAGTGTGTTTCCTCTGGCTCCCTCTTCCAAATCCACTTCT GGTGGCACTGCTGCTCTGGGATGCCTGGTGAAGGATTACTTT CCTGAACCTGTGACTGTCTCATGGAACTCTGGTGCTCTGACT TCTGGTGTCCACACTTTCCCTGCTGTGCTGCAGTCTAGTGGA CTGTACTCTCTGTCATCTGTGGTCACTGTGCCCTCTTCATCT CTGGGAACCCAGACCTACATTTGTAATGTGAACCACAAACCA TCCAACACTAAAGTGGACAAAAAAGTGGAACCCAAATCCTGT GACAAAACCCACACCTGCCCACCTTGTCCTGCCCCTGAACTG CTGGGAGGACCTTCTGTGTTTCTGTTCCCCCCCAAACCAAAG GATACCCTGATGATCTCTAGAACCCCTGAGGTGACATGTGTG GTGGTGGAGGTGTCTCATGAGGACCCTGAGGTCAAATTCAAC TGGTACGTGGATGGAGTGGAAGTCCACAATGCCAAAACCAAG CCTAGAGAGGAACAGTACCAATCAACCTACAGAGTTGTCAGT GTGCTGACTGTGCTGCATCAGGATTGGCTGAATGGCAAGGAA TACAAGTGTAAAGTCTCAAACAAGGCCCTGTCTGCTCCAATT GAGAAAACAATCTCAAAGGCCAAGGGACAGCCTAGGGAACCC CAGGTCTACACCCTGCCACCTTCAAGAGAGGAAATGACCAAA AACCAGGTGTCCCTGACATGCCTGGTCAAAGGCTTCTACCCT TCTGACATTGCTGTGGAGTGGGAGTCAAATGGACAGCCTGAG AACAACTACAAAACAACCCCCCCTGTGCTGGATTCTGATGGC TCTTTCTTTCTGTACTCCAAACTGACTGTGGACAAGTCTAGA TGGCAGCAGGGGAATGTCTTTTCTTGCTCTGTCATGCATGAG GCTCTGCATAACCACTACACTCAGAAATCCCTGTCTCTGTCT CCCGGGAAATGA |
| 20 | mAb1 DENQPS IgG1 HC (protein) | QVQLQQSGAELARPGASVKMSCKASGYTFTRSTMHWVKQRPG QGLEWIGYINPSSAYTNYNQKFKDKATLTADKSSSTAYMQLS SLTSEDSAVYYCASPQVHYDYNGFPYWGQGTLVTVSAASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVEVSHEDPEVKFNWYVDGVEVHNAKTK PREEQYQSTYRVVSVLTVLHQDWLNGKEYKCKVSNKALSAPI EKTISKAKGQPREPQVYTLPPSREEMTKNQVSLTCLVKGFYP SDIAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 21 | mAb2 G1 C-LC DANAPA IgG1 LC (DNA) | CAGACCGTTGTGACTCAGGAACCTTCACTCACCGTATCACCT<br>GGTGGAACAGTCACACTCACTTGTCGCTCGTCGACTGGGGCT<br>GTTACAACTAGCAACTATGCCAACTGGGTCCAACAAAAACCG<br>GGTCAGGCACCCCGTGGTCTAATAGGTGGTACCAACAAGCGC<br>GCACCAGGTACTCCTGCCAGATTCTCAGGCTCCCTGCTTGGA<br>GGCAAGGCTGCCCTCACCCTCTCGGGGGTACAGCCAGAGGAT<br>GAGGCAGAATATTACTGTGCTCTATGGTACAGCAACCTCTGG<br>GTGTTCGGTGGAGGAACCAAACTGACTGTCCTAGGCCAGCCT<br>AAAGCGGCGCCATCCGTCACCCTGTTCCCTCCCTCATCCGAG<br>GAACTGCAGGCCAATAAGGCTACACTGGTCTGTCTGATTAGC<br>GACTTCTACCCTGGGGCCGTGACTGTGGCTTGGAAAGCCGAT<br>TCTTCTCCCGTGAAAGCTGGAGTGGAAACAACCACCCCCTCT<br>AAACAGAGCAACAACAAATACGCTGCCTCTTCATACCTGTCC<br>CTGACCCCTGAACAGTGGAAATCTCACCGGTCTTACTCATGC<br>CAGGTGACACACGAGGGATCAACTGTGGAGAAAACCGTGGCT<br>CCTACCGAATGTTCAGGCGGTGGAGGATCCGGGGGTGGGGGA<br>AGCGGCGGAGGAGGTAGCGGCGTGACTCTGTTCGTCGCTCTG<br>TACGACTATGAGGCCCTGGGGGCTCACGAACTGTCCTTCCAT<br>AAGGGCGAGAAATTTCAGATCCTGTCCCCCAGGAGCGAGGGA<br>CCTTTTTGGGAAGCACACTCTCTGACCACAGGCGAAACCGGA<br>TGGATTCCCTCTAACTACGTGGCCCCCGTCGATAGTATTCAG<br>TGA |
| 22 | mAb2 G1 C-LC DANAPA IgG1 LC (protein) | QTVVTQEPSLTVSPGGTVTLTCRSSTGAVTTSNYANWVQQKP<br>GQAPRGLIGGTNKRAPGTPARFSGSLLGGKAALTLSGVQPED<br>EAEYYCALWYSNLWVFGGGTKLTVLGQPKAAPSVTLFPPSSE<br>ELQANKATLVCLISDFYPGAVTVAWKADSSPVKAGVETTTPS<br>KQSNNKYAASSYLSLTPEQWKSHRSYSCQVTHEGSTVEKTVA<br>PTECSGGGGSGGGGSGGGGSGVTLFVALYDYEALGAHELSFH<br>KGEKFQILSPRSEGPFWEAHSLTTGETGWIPSNYVAPVDSIQ |

(continued)

| SEQ ID NO: | Description | Sequence |
|---|---|---|
| 23 | mAb2 D5 C-LC DANAPA IgG1 LC (DNA) | CAGACCGTTGTGACTCAGGAACCTTCACTCACCGTATCACCT GGTGGAACAGTCACACTCACTTGTCGCTCGTCGACTGGGGCT GTTACAACTAGCAACTATGCCAACTGGGTCCAACAAAAACCG GGTCAGGCACCCCGTGGTCTAATAGGTGGTACCAACAAGCGC GCACCAGGTACTCCTGCCAGATTCTCAGGCTCCCTGCTTGGA GGCAAGGCTGCCCTCACCCTCTCGGGGGTACAGCCAGAGGAT GAGGCAGAATATTACTGTGCTCTATGGTACAGCAACCTCTGG GTGTTCGGTGGAGGAACCAAACTGACTGTCCTAGGCCAGCCT AAAGCGGCGCCATCCGTCACCCTGTTCCCTCCCTCATCCGAG GAACTGCAGGCCAATAAGGCTACACTGGTCTGTCTGATTAGC GACTTCTACCCTGGGGCCGTGACTGTGGCTTGGAAAGCCGAT TCTTCTCCCGTGAAAGCTGGAGTGGAAACAACCACCCCCTCT AAACAGAGCAACAACAAATACGCTGCCTCTTCATACCTGTCC CTGACCCCTGAACAGTGGAAATCTCACCGGTCTTACTCATGC CAGGTGACACACGAGGGATCAACTGTGGAGAAAACCGTGGCT CCTACCGAATGTTCAGGCGGTGGAGGATCCGGGGGTGGGGGA AGCGGCGGAGGAGGTAGCGGCGTGACTCTGTTCGTCGCTCTG TACGACTATGAGGCCCTGGGGGCTCACGAACTGTCCTTCCAT AAGGGCGAGAAATTTCAGATCCTGTCCAGCCTGGCAGTGGGA CCATTTTGGGAGGCCCACTCTCTGACCACAGGCGAAACCGGA TGGATTCCCTCTAACTACGTGGCACCTGTCGATAGTATTCAG TGA |
| 24 | mAb2 D5 C-LC DANAPA IgG1 LC (protein) | QTVVTQEPSLTVSPGGTVTLTCRSSTGAVTTSNYANWVQQKP GQAPRGLIGGTNKRAPGTPARFSGSLLGGKAALTLSGVQPED EAEYYCALWYSNLWVFGGGTKLTVLGQPKAAPSVTLFPPSSE ELQANKATLVCLISDFYPGAVTVAWKADSSPVKAGVETTTPS KQSNNKYAASSYLSLTPEQWKSHRSYSCQVTHEGSTVEKTVA PTECSGGGGSGGGGSGGGGSGVTLFVALYDYEALGAHELSFH KGEKFQILSSLAVGPFWEAHSLTTGETGWIPSNYVAPVDSIQ |
| 25 | mAb3 B3 C-LC LALA IgG1 (COVA467) HC (DNA) | CAGGTGCAGCTGGTGCAGTCTGGCGGCGGAGTGGTGCAGCCT GGAAGATCCCTGCGGCTGTCCTGCAAGGCCTCCGGCTACACC TTCACCCGGTACACCATGCACTGGGTGCGACAGGCCCCTGGC AAGGGCCTGGAATGGATCGGCTACATCAACCCCTCCCGGGGC TACACCAACTACAACCAGAAAGTGAAGGACCGGTTCACCATC TCCCGGGACAACTCCAAGAACACCGCCTTTCTGCAGATGGAC AGCCTGCGGCCTGAGGATACCGGCGTGTACTTCTGCGCCCGG TACTACGACGACCACTACTGCCTGGACTACTGGGGCCAGGGC ACCCCTGTGACAGTGTCCTCTGCTAGCACAAAGGGCCCTAGT GTGTTTCCTCTGGCTCCCTCTTCCAAATCCACTTCTGGTGGC ACTGCTGCTCTGGGATGCCTGGTGAAGGATTACTTTCCTGAA |

(continued)

| SEQ ID NO: | Description | Sequence |
|---|---|---|
| | | CCTGTGACTGTCTCATGGAACTCTGGTGCTCTGACTTCTGGT GTCCACACTTTCCCTGCTGTGCTGCAGTCTAGTGGACTGTAC TCTCTGTCATCTGTGGTCACTGTGCCCTCTTCATCTCTGGGA ACCCAGACCTACATTTGTAATGTGAACCACAAACCATCCAAC ACTAAAGTGGACAAAAAAGTGGAACCCAAATCCTGTGACAAA ACCCACACCTGCCCACCTTGTCCTGCCCCTGAAGCCGCCGGA GGACCTTCTGTGTTTCTGTTCCCCCCCAAACCAAAGGATACC CTGATGATCTCTAGAACCCCTGAGGTGACATGTGTGGTGGTG GATGTGTCTCATGAGGACCCTGAGGTCAAATTCAACTGGTAC GTGGATGGAGTGGAAGTCCACAATGCCAAAACCAAGCCTAGA GAGGAACAGTACAATTCAACCTACAGAGTGGTCAGTGTGCTG ACTGTGCTGCATCAGGATTGGCTGAATGGCAAGGAATACAAG TGTAAAGTCTCAAACAAGGCCCTGCCTGCTCCAATTGAGAAA ACAATCTCAAAGGCCAAGGGACAGCCTAGGGAACCCCAGGTC TACACCCTGCCACCTTCAAGAGAGGAAATGACCAAAAACCAG GTGTCCCTGACATGCCTGGTCAAAGGCTTCTACCCTTCTGAC ATTGCTGTGGAGTGGGAGTCAAATGGACAGCCTGAGAACAAC TACAAAACAACCCCCCCTGTGCTGGATTCTGATGGCTCTTTC TTTCTGTACTCCAAACTGACTGTGGACAAGTCTAGATGGCAG CAGGGGAATGTCTTTTCTTGCTCTGTCATGCATGAGGCTCTG CATAACCACTACACTCAGAAATCCCTGTCTCTGTCTCCCGGG AAATGA |
| 26 | mAb3 B3 C-LC LALA IgG1 (COVA467) HC (protein) | QVQLVQSGGGVVQPGRSLRLSCKASGYTFTRYTMHWVRQAPG KGLEWIGYINPSRGYTNYNQKVKDRFTISRDNSKNTAFLQMD SLRPEDTGVYFCARYYDDHYCLDYWGQGTPVTVSSASTKGPS VFPLAPSSKSTSGGTAALGCLVKDYFPEPVTVSWNSGALTSG VHTFPAVLQSSGLYSLSSVVTVPSSSLGTQTYICNVNHKPSN TKVDKKVEPKSCDKTHTCPPCPAPEAAGGPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEK TISKAKGQPREPQVYTLPPSREEMTKNQVSLTCLVKGFYPSD IAVEWESNGQPENNYKTTPPVLDSDGSFFLYSKLTVDKSRWQ QGNVFSCSVMHEALHNHYTQKSLSLSPGK |

(continued)

| SEQ ID NO: | Description | Sequence |
|---|---|---|
| 27 | mAb3 B3 C-LC LALA IgG1 (COVA467) LC (DNA) | GACATCCAGATGACCCAGTCCCCCTCCAGCCTGTCTGCCTCT GTGGGCGACAGAGTGACAATTACCTGCTCCGCCTCCTCCTCC GTGTCCTACATGAACTGGTATCAGCAGACCCCCGGCAAGGCC CCCAAGCGGTGGATCTACGACACCTCCAAGCTGGCCTCTGGC GTGCCCTCCAGATTCTCCGGCTCTGGCTCTGGCACCGACTAT ACCTTCACCATCAGCTCCCTGCAGCCCGAGGATATCGCCACC TACTACTGCCAGCAGTGGTCCTCCAACCCCTTCACCTTTGGC CAGGGCACCAAGCTGCAGATCACCCGTACGGTCGCGGCGCCT TCTGTGTTCATTTTCCCCCCATCTGATGAACAGCTGAAATCT GGCACTGCTTCTGTGGTCTGTCTGCTGAACAACTTCTACCCT AGAGAGGCCAAAGTCCAGTGGAAAGTGGACAATGCTCTGCAG AGTGGGAATTCCCAGGAATCTGTCACTGAGCAGGACTCTAAG GATAGCACATACTCCCTGTCCTCTACTCTGACACTGAGCAAG GCTGATTACGAGAAACACAAAGTGTACGCCTGTGAAGTCACA CATCAGGGGCTGTCTAGTCCTGTGACCAAATCCTTCAATAGG GGAGAGTGCGGCGGTGGAGGATCCGGGGGTGGGGGAAGCGGC GGAGGAGGTAGCGGCGTGACCCTGTTTGTGGCCCTGTACGAC TACGAGGCCCTGGGCGCTCACGAGCTGTCTTTCCACAAGGGC GAGAAGTTCCAGATCCTGAACTCCTCCGAGGGCCCCTTCTGG GAGGCTCACTCTCTGACAACCGGCGAGACAGGCTGGATTCCC TCCAACTATGTGGCCCCCGTGGACTCCATCCAGTGA |
| 28 | mAb3 B3 C-LC LALA IgG1 (COVA467) LC (protein) | DIQMTQSPSSLSASVGDRVTITCSASSSVSYMNWYQQTPGKA PKRWIYDTSKLASGVPSRFSGSGSGTDYTFTISSLQPEDIAT YYCQQWSSNPFTFGQGTKLQITRTVAAPSVFIFPPSDEQLKS GTASVVCLLNNFYPREAKVQWKVDNALQSGNSQESVTEQDSK DSTYSLSSTLTLSKADYEKHKVYACEVTHQGLSSPVTKSFNR GECGGGGSGGGGSGGGGSGVTLFVALYDYEALGAHELSFHKG EKFQILNSSEGPFWEAHSLTTGETGWIPSNYVAPVDSIQ |

(continued)

| SEQ ID NO: | Description | Sequence |
|---|---|---|
| 29 | B21M IgG1 HC (DNA) | CAGATCACCCTGAAGGAGTCCGGGCCCACACTGGTGAAACCT ACTCAGACCCTGACACTGACTTGCACCTTCTCCGGTTTTTCT CTGAGTACCTCGGGCATGGGAGTGAGCTGGATCAGGCAGCCC CCTGGCAAGGCACTGGAATGGCTGGCCCACATCTACTGGGAC GATGACAAGAGGTACAACCCTTCACTGAAATCCCGGCTGACA ATTACTAAGGATACCAGCAAAAACCAGGTGGTCCTGACCATG ACAAATATGGACCCCGTGGACACTGCTACCTACTATTGTGCA AGACTGTACGGCTTCACCTATGGATTTGCTTACTGGGGGCAG GGCACCCTGGTCACAGTCTCGAGCGCTAGCACAAAGGGCCCT AGTGTGTTTCCTCTGGCTCCCTCTTCCAAATCCACTTCTGGT GGCACTGCTGCTCTGGGATGCCTGGTGAAGGATTACTTTCCT GAACCTGTGACTGTCTCATGGAACTCTGGTGCTCTGACTTCT GGTGTCCACACTTTCCCTGCTGTGCTGCAGTCTAGTGGACTG TACTCTCTGTCATCTGTGGTCACTGTGCCCTCTTCATCTCTG GGAACCCAGACCTACATTTGTAATGTGAACCACAAACCATCC AACACTAAAGTGGACAAAAAAGTGGAACCCAAATCCTGTGAC AAAACCCACACCTGCCCACCTTGTCCTGCCCCTGAACTGCTG GGAGGACCTTCTGTGTTTCTGTTCCCCCCCAAACCAAAGGAT ACCCTGATGATCTCTAGAACCCCTGAGGTGACATGTGTGGTG GTGGATGTGTCTCATGAGGACCCTGAGGTCAAATTCAACTGG TACGTGGATGGAGTGGAAGTCCACAATGCCAAAACCAAGCCT AGAGAGGAACAGTACAATTCAACCTACAGAGTTGTCAGTGTG CTGACTGTGCTGCATCAGGATTGGCTGAATGGCAAGGAATAC AAGTGTAAAGTCTCAAACAAGGCCCTGCCTGCTCCAATTGAG AAAACAATCTCAAAGGCCAAGGGACAGCCTAGGGAACCCCAG GTCTACACCCTGCCACCTTCAAGAGAGGAAATGACCAAAAAC CAGGTGTCCCTGACATGCCTGGTCAAAGGCTTCTACCCTTCT GACATTGCTGTGGAGTGGGAGTCAAATGGACAGCCTGAGAAC AACTACAAAACAACCCCCCCTGTGCTGGATTCTGATGGCTCT TTCTTTCTGTACTCCAAACTGACTGTGGACAAGTCTAGATGG CAGCAGGGGAATGTCTTTTCTTGCTCTGTCATGCATGAGGCT CTGCATAACCACTACACTCAGAAATCCCTGTCTCTGTCTCCC GGGAAATGA |
| 30 | B21M IgG1 HC (protein) | QITLKESGPTLVKPTQTLTLTCTFSGFSLSTSGMGVSWIRQP PGKALEWLAHIYWDDDKRYNPSLKSRLTITKDTSKNQVVLTM TNMDPVDTATYYCARLYGFTYGFAYWGQGTLVTVSSASTKGP SVFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPKD TLMISRTPEVTCVVVDVSHEDPEVKFNWYVDGVEVHNAKTKP REEQYNSTYRVVSVLTVLHQDWLNGKEYKCKVSNKALPAPIE KTISKAKGQPREPQVYTLPPSREEMTKNQVSLTCLVKGFYPS DIAVEWESNGQPENNYKTTPPVLDSDGSFFLYSKLTVDKSRW QQGNVFSCSVMHEALHNHYTQKSLSLSPGK |
| 31 | B21M IgG1 LC (DNA) | GACATCGTGATGACACAGAGCCCAGATTCTCTGGCCGTCAGC CTGGGCGAAAGGGCCACTATCAACTGCCGGGCCTCCCAGTCT GTGGACTACAATGGAATTTCTTACATGCACTGGTATCAGCAG |

(continued)

| SEQ ID NO: | Description | Sequence |
|---|---|---|
| | | AAGCCTGGCCAGCCCCCTAAACTGCTGATCTATGCCGCTTCA AACCCTGAGTCCGGCGTGCCAGACCGATTCAGTGGCTCAGGC TCCGGGACCGATTTTACCCTGACAATTTCCAGCCTGCAAGCT GAGGACGTGGCAGTCTACTATTGCCAGCAGATCATTGAAGAT CCCTGGACATTCGGTCAGGGCACTAAGGTGGAGATCAAACGT ACGGTCGCGGCGCCTTCTGTGTTCATTTTCCCCCCATCTGAT GAACAGCTGAAATCTGGCACTGCTTCTGTGGTCTGTCTGCTG AACAACTTCTACCCTAGAGAGGCCAAAGTCCAGTGGAAAGTG GACAATGCTCTGCAGAGTGGGAATTCCCAGGAATCTGTCACT GAGCAGGACTCTAAGGATAGCACATACTCCCTGTCCTCTACT CTGACACTGAGCAAGGCTGATTACGAGAAACACAAAGTGTAC GCCTGTGAAGTCACACATCAGGGGCTGTCTAGTCCTGTGACC AAATCCTTCAATAGGGGAGAGTGCTGA |
| 32 | B21M and B21M LALA-IgG1 LC (protein) | DIVMTQSPDSLAVSLGERATINCRASQSVDYNGISYMHWYQQ KPGQPPKLLIYAASNPESGVPDRFSGSGSGTDFTLTISSLQA EDVAVYYCQQIIEDPWTFGQGTKVEIKRTVAAPSVFIFPPSD EQLKSGTASVVCLLNNFYPREAKVQWKVDNALQSGNSQESVT EQDSKDSTYSLSSTLTLSKADYEKHKVYACEVTHQGLSSPVT KSFNRGEC |

(continued)

| SEQ ID NO: | Description | Sequence |
|---|---|---|
| 33 | B21M LALA IgG1 HC (DNA) | CAGATCACCCTGAAGGAGTCCGGGCCCACACTGGTGAAACCT<br>ACTCAGACCCTGACACTGACTTGCACCTTCTCCGGTTTTTCT<br>CTGAGTACCTCGGGCATGGGAGTGAGCTGGATCAGGCAGCCC<br>CCTGGCAAGGCACTGGAATGGCTGGCCCACATCTACTGGGAC<br>GATGACAAGAGGTACAACCCTTCACTGAAATCCCGGCTGACA<br>ATTACTAAGGATACCAGCAAAAACCAGGTGGTCCTGACCATG<br>ACAAATATGGACCCCGTGGACACTGCTACCTACTATTGTGCA<br>AGACTGTACGGCTTCACCTATGGATTTGCTTACTGGGGGCAG<br>GGCACCCTGGTCACAGTCTCGAGCGCTAGCACAAAGGGCCCT<br>AGTGTGTTTCCTCTGGCTCCCTCTTCCAAATCCACTTCTGGT<br>GGCACTGCTGCTCTGGGATGCCTGGTGAAGGATTACTTTCCT<br>GAACCTGTGACTGTCTCATGGAACTCTGGTGCTCTGACTTCT<br>GGTGTCCACACTTTCCCTGCTGTGCTGCAGTCTAGTGGACTG<br>TACTCTCTGTCATCTGTGGTCACTGTGCCCTCTTCATCTCTG<br>GGAACCCAGACCTACATTTGTAATGTGAACCACAAACCATCC<br>AACACTAAAGTGGACAAAAAAGTGGAACCCAAATCCTGTGAC<br>AAAACCCACACCTGCCCACCTTGTCCTGCCCCTGAAGCCGCC<br>GGAGGACCTTCTGTGTTTCTGTTCCCCCCCAAACCAAAGGAT<br>ACCCTGATGATCTCTAGAACCCCTGAGGTGACATGTGTGGTG<br>GTGGATGTGTCTCATGAGGACCCTGAGGTCAAATTCAACTGG<br>TACGTGGATGGAGTGGAAGTCCACAATGCCAAAACCAAGCCT<br>AGAGAGGAACAGTACAATTCAACCTACAGAGTGGTCAGTGTG<br>CTGACTGTGCTGCATCAGGATTGGCTGAATGGCAAGGAATAC<br>AAGTGTAAAGTCTCAAACAAGGCCCTGCCTGCTCCAATTGAG<br>AAAACAATCTCAAAGGCCAAGGGACAGCCTAGGGAACCCCAG<br>GTCTACACCCTGCCACCTTCAAGAGAGGAAATGACCAAAAAC<br>CAGGTGTCCCTGACATGCCTGGTCAAAGGCTTCTACCCTTCT<br>GACATTGCTGTGGAGTGGGAGTCAAATGGACAGCCTGAGAAC<br>AACTACAAAACAACCCCCCCTGTGCTGGATTCTGATGGCTCT<br>TTCTTTCTGTACTCCAAACTGACTGTGGACAAGTCTAGATGG<br>CAGCAGGGGAATGTCTTTTCTTGCTCTGTCATGCATGAGGCT<br>CTGCATAACCACTACACTCAGAAATCCCTGTCTCTGTCTCCC<br>GGGAAATGA |
| 34 | B21M LALA IgG1 HC (protein) | QITLKESGPTLVKPTQTLTLTCTFSGFSLSTSGMGVSWIRQP<br>PGKALEWLAHIYWDDDKRYNPSLKSRLTITKDTSKNQVVLTM<br>TNMDPVDTATYYCARLYGFTYGFAYWGQGTLVTVSSASTKGP<br>SVFPLAPSSKSTSGGTAALGCLVKDYFPEPVTVSWNSGALTS<br>GVHTFPAVLQSSGLYSLSSVVTVPSSSLGTQTYICNVNHKPS<br>NTKVDKKVEPKSCDKTHTCPPCPAPEAAGGPSVFLFPPKPKD<br>TLMISRTPEVTCVVVDVSHEDPEVKFNWYVDGVEVHNAKTKP<br>REEQYNSTYRVVSVLTVLHQDWLNGKEYKCKVSNKALPAPIE<br>KTISKAKGQPREPQVYTLPPSREEMTKNQVSLTCLVKGFYPS<br>DIAVEWESNGQPENNYKTTPPVLDSDGSFFLYSKLTVDKSRW<br>OOGNVFSCSVMHEALHNHYTOKSLSLSPGK |

(continued)

| SEQ ID NO: | Description | Sequence |
|---|---|---|
| 35 | Fynomer G1 (DNA) | GGCGTGACTCTGTTCGTCGCTCTGTACGACTATGAGGCCCTG GGGGCTCACGAACTGTCCTTCCATAAGGGCGAGAAATTTCAG ATCCTGTCCCCCAGGAGCGAGGGACCTTTTTGGGAAGCACAC TCTCTGACCACAGGCGAAACCGGATGGATTCCCTCTAACTAC GTGGCCCCCGTCGATAGTATTCAGTGA |
| 36 | Fynomer G1 (protein) | GVTLFVALYDYEALGAHELSFHKGEKFQILSPRSEGPFWEAH SLTTGETGWIPSNYVAPVDSIQ |
| 37 | Fynomer D5 (DNA) | GGCGTGACTCTGTTCGTCGCTCTGTACGACTATGAGGCCCTG GGGGCTCACGAACTGTCCTTCCATAAGGGCGAGAAATTTCAG ATCCTGTCCAGCCTGGCAGTGGGACCATTTTGGGAGGCCCAC TCTCTGACCACAGGCGAAACCGGATGGATTCCCTCTAACTAC GTGGCACCTGTCGATAGTATTCAGTGA |
| 38 | Fynomer D5 (protein) | GVTLFVALYDYEALGAHELSFHKGEKFQILSSLAVGPFWEAH SLTTGETGWIPSNYVAPVDSIQ |
| 39 | (G4S)3 linker (DNA) | GGCGGTGGAGGATCCGGGGGTGGGGGAAGCGGCGGAGGAGGT AGC |
| 40 | (G4S) 3 linker (protein) | GGGGSGGGGSGGGGS |
| 41 | Leader sequence (DNA) | ATGAATTTTGGACTGAGGCTGATTTTCCTGGTGCTGACCCTG AAAGGCGTCCAGTGT |
| 42 | Leader Sequence (protein) | MNFGLRLIFLVLTLKGVQC |
| 43 | Wild-type human IgG1 Fc (starting at C226, according to Kabat numbering, positions 265, 297 and 329 in underlined italics) (protein) | CPPCPAPELLGGPSVFLFPPKPKDTLMISRTPEVTCVVV*D*VS HEDPEVKFNWYVDGVEVHNAKTKPREEQY*N*STYRVVSVLTVL HQDWLNGKEYKCKVSNKAL*P*APIEKTISKAKGQPREPQVYTL PPSREEMTKNQVSLTCLVKGFYPSDIAVEWESNGQPENNYKT TPPVLDSDGSFFLYSKLTVDKSRWQQGNVFSCSVMHEALHNH YTQKSLSLSPGK |
| 44 | mAb1 DANAPA IgG1 HC (DNA) | CAGGTCCAGCTGCAGCAGAGTGGGGCCGAACTGGCAAGACCC GGAGCAAGCGTCAAAATGTCATGTAAAGCAAGCGGTTATACT TTCACTAGGAGCACCATGCACTGGGTGAAACAGAGGCCCGGC CAGGGACTGGAGTGGATCGGGTACATTAACCCTTCCAGCGCT TACACCAACTATAATCAGAAGTTCAAAGACAAGGCCACCCTG |

(continued)

| SEQ ID NO: | Description | Sequence |
|---|---|---|
| | | ACAGCTGATAAGTCTAGTTCAACAGCATATATGCAGCTGTCC AGCCTGACTTCTGAAGACAGTGCAGTGTACTATTGCGCCTCC CCACAGGTCCACTACGATTACAATGGTTTTCCTTACTGGGGG CAGGGCACACTGGTGACTGTCTCCGCCGCTAGCACAAAGGGC CCTAGTGTGTTTCCTCTGGCTCCCTCTTCCAAATCCACTTCT GGTGGCACTGCTGCTCTGGGATGCCTGGTGAAGGATTACTTT CCTGAACCTGTGACTGTCTCATGGAACTCTGGTGCTCTGACT TCTGGTGTCCACACTTTCCCTGCTGTGCTGCAGTCTAGTGGA CTGTACTCTCTGTCATCTGTGGTCACTGTGCCCTCTTCATCT CTGGGAACCCAGACCTACATTTGTAATGTGAACCACAAACCA TCCAACACTAAAGTGGACAAAAAAGTGGAACCCAAATCCTGT GACAAAACCCACACCTGCCCACCTTGTCCTGCCCCTGAACTG CTGGGAGGACCTTCTGTGTTTCTGTTCCCCCCCAAACCAAAG GATACCCTGATGATCTCTAGAACCCCTGAGGTGACATGTGTG GTGGTGGCTGTGTCTCATGAGGACCCTGAGGTCAAATTCAAC TGGTACGTGGATGGAGTGGAAGTCCACAATGCCAAAACCAAG CCTAGAGAGGAACAGTACGCTTCAACCTACAGAGTTGTCAGT GTGCTGACTGTGCTGCATCAGGATTGGCTGAATGGCAAGGAA TACAAGTGTAAAGTCTCAAACAAGGCCCTGGCTGCTCCAATT GAGAAAACAATCTCAAAGGCCAAGGGACAGCCTAGGGAACCC CAGGTCTACACCCTGCCACCTTCAAGAGAGGAAATGACCAAA AACCAGGTGTCCCTGACATGCCTGGTCAAAGGCTTCTACCCT TCTGACATTGCTGTGGAGTGGGAGTCAAATGGACAGCCTGAG AACAACTACAAAACAACCCCCCCTGTGCTGGATTCTGATGGC TCTTTCTTTCTGTACTCCAAACTGACTGTGGACAAGTCTAGA TGGCAGCAGGGGAATGTCTTTTCTTGCTCTGTCATGCATGAG GCTCTGCATAACCACTACACTCAGAAATCCCTGTCTCTGTCT CCCGGGAAATGA |
| 45 | mAb1 DANAPA IgG1 HC (protein) | QVQLQQSGAELARPGASVKMSCKASGYTFTRSTMHWVKQRPG QGLEWIGYINPSSAYTNYNQKFKDKATLTADKSSSTAYMQLS SLTSEDSAVYYCASPQVHYDYNGFPYWGQGTLVTVSAASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVAVSHEDPEVKFNWYVDGVEVHNAKTK PREEQYASTYRVVSVLTVLHQDWLNGKEYKCKVSNKALAAPI EKTISKAKGQPREPQVYTLPPSREEMTKNQVSLTCLVKGFYP SDIAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 46 | mAb1 DAPA IgG1 HC (DNA) | CAGGTCCAGCTGCAGCAGAGTGGGGCCGAACTGGCAAGACCC GGAGCAAGCGTCAAAATGTCATGTAAAGCAAGCGGTTATACT TTCACTAGGAGCACCATGCACTGGGTGAAACAGAGGCCCGGC CAGGGACTGGAGTGGATCGGGTACATTAACCCTTCCAGCGCT TACACCAACTATAATCAGAAGTTCAAAGACAAGGCCACCCTG ACAGCTGATAAGTCTAGTTCAACAGCATATATGCAGCTGTCC AGCCTGACTTCTGAAGACAGTGCAGTGTACTATTGCGCCTCC CCACAGGTCCACTACGATTACAATGGTTTTCCTTACTGGGGG CAGGGCACACTGGTGACTGTCTCCGCCGCTAGCACAAAGGGC CCTAGTGTGTTTCCTCTGGCTCCCTCTTCCAAATCCACTTCT GGTGGCACTGCTGCTCTGGGATGCCTGGTGAAGGATTACTTT CCTGAACCTGTGACTGTCTCATGGAACTCTGGTGCTCTGACT TCTGGTGTCCACACTTTCCCTGCTGTGCTGCAGTCTAGTGGA CTGTACTCTCTGTCATCTGTGGTCACTGTGCCCTCTTCATCT CTGGGAACCCAGACCTACATTTGTAATGTGAACCACAAACCA TCCAACACTAAAGTGGACAAAAAAGTGGAACCCAAATCCTGT GACAAAACCCACACCTGCCCACCTTGTCCTGCCCCTGAACTG CTGGGAGGACCTTCTGTGTTTCTGTTCCCCCCCAAACCAAAG GATACCCTGATGATCTCTAGAACCCCTGAGGTGACATGTGTG GTGGTGGCTGTGTCTCATGAGGACCCTGAGGTCAAATTCAAC TGGTACGTGGATGGAGTGGAAGTCCACAATGCCAAAACCAAG CCTAGAGAGGAACAGTACAATTCAACCTACAGAGTTGTCAGT GTGCTGACTGTGCTGCATCAGGATTGGCTGAATGGCAAGGAA TACAAGTGTAAAGTCTCAAACAAGGCCCTGGCTGCTCCAATT GAGAAAACAATCTCAAAGGCCAAGGGACAGCCTAGGGAACCC CAGGTCTACACCCTGCCACCTTCAAGAGAGGAAATGACCAAA AACCAGGTGTCCCTGACATGCCTGGTCAAAGGCTTCTACCCT TCTGACATTGCTGTGGAGTGGGAGTCAAATGGACAGCCTGAG AACAACTACAAAACAACCCCCCCTGTGCTGGATTCTGATGGC TCTTTCTTTCTGTACTCCAAACTGACTGTGGACAAGTCTAGA TGGCAGCAGGGGAATGTCTTTTCTTGCTCTGTCATGCATGAG GCTCTGCATAACCACTACACTCAGAAATCCCTGTCTCTGTCT CCCGGGAAATGA |
| 47 | mAb1 DAPA IgG1 HC (protein) | QVQLQQSGAELARPGASVKMSCKASGYTFTRSTMHWVKQRPG QGLEWIGYINPSSAYTNYNQKFKDKATLTADKSSSTAYMQLS SLTSEDSAVYYCASPQVHYDYNGFPYWGQGTLVTVSAASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVAVSHEDPEVKFNWYVDGVEVHNAKTK PREEQYNSTYRVVSVLTVLHQDWLNGKEYKCKVSNKALAAPI EKTISKAKGQPREPQVYTLPPSREEMTKNQVSLTCLVKGFYP SDIAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 48 | mAb1 N297A IgG1 HC (DNA) | CAGGTCCAGCTGCAGCAGAGTGGGGCCGAACTGGCAAGACCC GGAGCAAGCGTCAAAATGTCATGTAAAGCAAGCGGTTATACT TTCACTAGGAGCACCATGCACTGGGTGAAACAGAGGCCCGGC CAGGGACTGGAGTGGATCGGGTACATTAACCCTTCCAGCGCT TACACCAACTATAATCAGAAGTTCAAAGACAAGGCCACCCTG ACAGCTGATAAGTCTAGTTCAACAGCATATATGCAGCTGTCC AGCCTGACTTCTGAAGACAGTGCAGTGTACTATTGCGCCTCC CCACAGGTCCACTACGATTACAATGGTTTTCCTTACTGGGGG CAGGGCACACTGGTGACTGTCTCCGCCGCTAGCACAAAGGGC CCTAGTGTGTTTCCTCTGGCTCCCTCTTCCAAATCCACTTCT GGTGGCACTGCTGCTCTGGGATGCCTGGTGAAGGATTACTTT CCTGAACCTGTGACTGTCTCATGGAACTCTGGTGCTCTGACT TCTGGTGTCCACACTTTCCCTGCTGTGCTGCAGTCTAGTGGA CTGTACTCTCTGTCATCTGTGGTCACTGTGCCCTCTTCATCT CTGGGAACCCAGACCTACATTTGTAATGTGAACCACAAACCA TCCAACACTAAAGTGGACAAAAAAGTGGAACCCAAATCCTGT GACAAAACCCACACCTGCCCACCTTGTCCTGCCCCTGAACTG CTGGGAGGACCTTCTGTGTTTCTGTTCCCCCCCAAACCAAAG GATACCCTGATGATCTCTAGAACCCCTGAGGTGACATGTGTG GTGGTGGATGTGTCTCATGAGGACCCTGAGGTCAAATTCAAC TGGTACGTGGATGGAGTGGAAGTCCACAATGCCAAAACCAAG CCTAGAGAGGAACAGTACGCTTCAACCTACAGAGTTGTCAGT GTGCTGACTGTGCTGCATCAGGATTGGCTGAATGGCAAGGAA TACAAGTGTAAAGTCTCAAACAAGGCCCTGCCTGCTCCAATT GAGAAAACAATCTCAAAGGCCAAGGGACAGCCTAGGGAACCC CAGGTCTACACCCTGCCACCTTCAAGAGAGGAAATGACCAAA AACCAGGTGTCCCTGACATGCCTGGTCAAAGGCTTCTACCCT TCTGACATTGCTGTGGAGTGGGAGTCAAATGGACAGCCTGAG AACAACTACAAAACAACCCCCCCTGTGCTGGATTCTGATGGC TCTTTCTTTCTGTACTCCAAACTGACTGTGGACAAGTCTAGA TGGCAGCAGGGGAATGTCTTTTCTTGCTCTGTCATGCATGAG GCTCTGCATAACCACTACACTCAGAAATCCCTGTCTCTGTCT CCCGGGAAATGA |
| 49 | mAb1 N297A IgG1 HC (protein) | QVQLQQSGAELARPGASVKMSCKASGYTFTRSTMHWVKQRPG QGLEWIGYINPSSAYTNYNQKFKDKATLTADKSSSTAYMQLS SLTSEDSAVYYCASPQVHYDYNGFPYWGQGTLVTVSAASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVDVSHEDPEVKFNWYVDGVEVHNAKTK PREEQYASTYRVVSVLTVLHQDWLNGKEYKCKVSNKALPAPI EKTISKAKGQPREPQVYTLPPSREEMTKNQVSLTCLVKGFYP SDIAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 50 | mAb1 LALA IgG1 HC (DNA) | CAGGTCCAGCTGCAGCAGAGTGGGGCCGAACTGGCAAGACCC GGAGCAAGCGTCAAAATGTCATGTAAAGCAAGCGGTTATACT TTCACTAGGAGCACCATGCACTGGGTGAAACAGAGGCCCGGC CAGGGACTGGAGTGGATCGGGTACATTAACCCTTCCAGCGCT TACACCAACTATAATCAGAAGTTCAAAGACAAGGCCACCCTG ACAGCTGATAAGTCTAGTTCAACAGCATATATGCAGCTGTCC AGCCTGACTTCTGAAGACAGTGCAGTGTACTATTGCGCCTCC CCACAGGTCCACTACGATTACAATGGTTTTCCTTACTGGGGG CAGGGCACACTGGTGACTGTCTCCGCCGCTAGCACAAAGGGC CCTAGTGTGTTTCCTCTGGCTCCCTCTTCCAAATCCACTTCT GGTGGCACTGCTGCTCTGGGATGCCTGGTGAAGGATTACTTT CCTGAACCTGTGACTGTCTCATGGAACTCTGGTGCTCTGACT TCTGGTGTCCACACTTTCCCTGCTGTGCTGCAGTCTAGTGGA CTGTACTCTCTGTCATCTGTGGTCACTGTGCCCTCTTCATCT CTGGGAACCCAGACCTACATTTGTAATGTGAACCACAAACCA TCCAACACTAAAGTGGACAAAAAAGTGGAACCCAAATCCTGT GACAAAACCCACACCTGCCCACCTTGTCCTGCCCCTGAAGCC GCCGGAGGACCTTCTGTGTTTCTGTTCCCCCCCAAACCAAAG GATACCCTGATGATCTCTAGAACCCCTGAGGTGACATGTGTG GTGGTGGATGTGTCTCATGAGGACCCTGAGGTCAAATTCAAC TGGTACGTGGATGGAGTGGAAGTCCACAATGCCAAAACCAAG CCTAGAGAGGAACAGTACAATTCAACCTACAGAGTGGTCAGT GTGCTGACTGTGCTGCATCAGGATTGGCTGAATGGCAAGGAA TACAAGTGTAAAGTCTCAAACAAGGCCCTGCCTGCTCCAATT GAGAAAACAATCTCAAAGGCCAAGGGACAGCCTAGGGAACCC CAGGTCTACACCCTGCCACCTTCAAGAGAGGAAATGACCAAA AACCAGGTGTCCCTGACATGCCTGGTCAAAGGCTTCTACCCT TCTGACATTGCTGTGGAGTGGGAGTCAAATGGACAGCCTGAG AACAACTACAAAACAACCCCCCCTGTGCTGGATTCTGATGGC TCTTTCTTTCTGTACTCCAAACTGACTGTGGACAAGTCTAGA TGGCAGCAGGGGAATGTCTTTTCTTGCTCTGTCATGCATGAG GCTCTGCATAACCACTACACTCAGAAATCCCTGTCTCTGTCT CCCGGGAAATGA |
| 51 | mAb1 LALA IgG1 HC (protein) | QVQLQQSGAELARPGASVKMSCKASGYTFTRSTMHWVKQRPG QGLEWIGYINPSSAYTNYNQKFKDKATLTADKSSSTAYMQLS SLTSEDSAVYYCASPQVHYDYNGFPYWGQGTLVTVSAASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPEAAGGPSVFLFPPKPK DTLMISRTPEVTCVVVDVSHEDPEVKFNWYVDGVEVHNAKTK PREEQYNSTYRVVSVLTVLHQDWLNGKEYKCKVSNKALPAPI EKTISKAKGQPREPQVYTLPPSREEMTKNQVSLTCLVKGFYP SDIAVEWESNGQPENNYKTTPPVLDSDGSFFLYSKLTVDKSR WQQGNVFSCSVMHEALHNHYTQKSLSLSPGK |

(continued)

| SEQ ID NO: | Description | Sequence |
|---|---|---|
| 52 | Wild-type human IgG1 Fc (allelic variant 356D, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSRDEMTKNQVSLTCLVKGFYPSDIAVEWESNGQPENNYKT<br>TPPVLDSDGSFFLYSKLTVDKSRWQQGNVFSCSVMHEALHNH<br>YTQKSLSLSPGK |
| 53 | Wild-type human IgG1 Fc (allelic variant 358L, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSREELTKNQVSLTCLVKGFYPSDIAVEWESNGQPENNYKT<br>TPPVLDSDGSFFLYSKLTVDKSRWQQGNVFSCSVMHEALHNH<br>YTQKSLSLSPGK |
| 54 | Wild-type human IgG1 Fc (allelic variant 431G, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSREEMTKNQVSLTCLVKGFYPSDIAVEWESNGQPENNYKT<br>TPPVLDSDGSFFLYSKLTVDKSRWQQGNVFSCSVMHEGLHNH<br>YTQKSLSLSPGK |
| 55 | Wild-type human IgG1 Fc (allelic variant 356D, 358L, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSRDELTKNQVSLTCLVKGFYPSDIAVEWESNGQPENNYKT<br>TPPVLDSDGSFFLYSKLTVDKSRWQQGNVFSCSVMHEALHNH<br>YTQKSLSLSPGK |
| 56 | Wild-type human IgG1 Fc (allelic variant 356D, 431G, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSRDEMTKNQVSLTCLVKGFYPSDIAVEWESNGQPENNYKT<br>TPPVLDSDGSFFLYSKLTVDKSRWQQGNVFSCSVMHEGLHNH<br>YTQKSLSLSPGK |
| 57 | Wild-type human IgG1 Fc (allelic variant 358L, 431G, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSREELTKNQVSLTCLVKGFYPSDIAVEWESNGQPENNYKT<br>TPPVLDSDGSFFLYSKLTVDKSRWQQGNVFSCSVMHEGLHNH<br>YTQKSLSLSPGK |
| 58 | Wild-type human IgG1 Fc (allelic variant 356D, 358L, 431G, Kabat numbering, positions 265, 297 and 329 in underlined italics) (protein) | CPPCPAPELLGGPSVFLFPPKPKDTLMISRTPEVTCVVV*D*VS<br>HEDPEVKFNWYVDGVEVHNAKTKPREEQY*N*STYRVVSVLTVL<br>HQDWLNGKEYKCKVSNKAL*P*APIEKTISKAKGQPREPQVYTL<br>PPSRDELTKNQVSLTCLVKGFYPSDIAVEWESNGQPENNYKT<br>TPPVLDSDGSFFLYSKLTVDKSRWQQGNVFSCSVMHEGLHNH<br>YTQKSLSLSPGK |
| 59 | Fyn SH3 domain (protein) | GVTLFVALYDYEARTEDDLSFHKGEKFQILNSSEGDWWEARS<br>LTTGETGYIPSNYVAPVDSIQ |

(continued)

| SEQ ID NO: | Description | Sequence |
|---|---|---|
| 60 | wild-type human IgG1 CH2 domain | APELLGGPSVFLFPPKPKDTLMISRTPEVTCVVVDVSHEDPE VKFNWYVDGVEVHNAKTKPREEQYNSTYRVVSVLTVLHQDWL NGKEYKCKVSNKALPAPIEKTISKAK |
| 61 | wild-type human IgG1 CH3 domain | GQPREPQVYTLPPSRDELTKNQVSLTCLVKGFYPSDIAVEWE SNGQPENNYKTTPPVLDSDGSFFLYSKLTVDKSRWQQGNVFS CSVMHEALHNHYTQKSLSLSPGK |

## Claims

**1.** A recombinant human IgG1 Fc-containing molecule, comprising a CH2 domain that comprises an amino acid sequence that is at least 80% identical to the amino acid sequence of SEQ ID NO: 60 and in which the amino acid at position 265 is different from aspartic acid (D), the amino acid at position 297 is different from asparagine (N), and the amino acid at position 329 is different from proline (P),

wherein the molecule retains binding to FcRn, and wherein the molecule has at least 20-fold reduced binding to Clq and to at least one Fcγ receptor (FcγR), as compared to a wild-type human IgG1 Fc-containing molecule that comprises D at position 265, N at position 297 and P at position 329,
and wherein the numbering is indicated by the EU index as in Kabat.

**2.** The molecule of claim 1, wherein the molecule has at least 50-fold reduced binding to Clq and to at least one FcyR, as compared to a wild-type human IgG1 Fc-containing molecule that comprises D at position 265, N at position 297 and P at position 329.

**3.** The molecule of claim 1 or 2, wherein at least one FcγR is selected from FcγRI, FcγRIIa, FcγRIIb, FcγRIIIa, and FcγRIIIb.

**4.** The molecule of any one of claims 1 to 3, wherein

i. the amino acid at position 265 is alanine (A), asparagine (N) or glutamic acid (E),
ii. the amino acid at position 297 is alanine (A), aspartic acid (D), or glutamine (Q), and
iii. the amino acid at position 329 is replaced with alanine (A), glycine (G), or serine (S).

**5.** The molecule of any one of claims 1-4, wherein the CH2 domain comprises an amino acid sequence that is at least 90% identical to the amino acid sequence of SEQ ID NO: 60.

**6.** The molecule of any one of claims 1-5, wherein the Fc domain comprises an amino acid sequence that is at least 80%, preferably at least 90% identical to the amino acid sequence of the human IgG1 Fc domain comprising SEQ ID NO: 43.

**7.** The molecule of any one of claims 1-6, wherein the molecule is an antibody, an Fc region, an Fc-fusion protein, or antibody fusion protein such as a FynomAb.

**8.** The molecule of any one of claims 1-7, wherein the molecule comprises an Fc region comprising a sequence according to any one of SEQ ID NOs: 43, 52, 53, 54, 55, 56, 57, or 58, wherein amino acids D at position 265, N at position 297 and P at position 329 are replaced by other amino acids.

**9.** A recombinant polynucleotide encoding the molecule of any one of the preceding claims.

**10.** A vector comprising the polynucleotide of claim 9.

**11.** A host cell comprising the recombinant polynucleotide of claim 9 or the vector of claim 10.

**12.** A method of making a recombinant human IgG1 Fc-containing molecule, comprising a CH2 domain that comprises an amino acid sequence that is at least 80% identical to the amino acid sequence of SEQ ID NO: 60 and in which amino acids at position 265, 297, and 329 indicated by the EU index as in Kabat are replaced by other amino acids, the method comprising the steps of:

a. providing a nucleic acid encoding a wild-type human IgG1 Fc-containing molecule,
b. modifying the nucleic acid provided in step (a) so as to obtain a nucleic acid encoding a recombinant human IgG1 Fc-containing molecule wherein the amino acids at position 265, 297, and 329 are replaced with amino acids other than D, N and P, respectively, and
c. expressing the nucleic acid obtaining in step (b) in a host cell and recovering the said recombinant human IgG1 Fc-containing molecule,

wherein the recombinant human IgG1 Fc-containing molecule retains binding to FcRn, and wherein the recombinant human IgG1 Fc-containing molecule has at least 20-fold reduced binding to C1q and to at least one Fcγ receptor (FcγR), as compared to a wild-type human IgG1 Fc-containing molecule that comprises D at position 265, N at position 297 and P at position 329.

**13.** A recombinant polypeptide comprising

a. at least one binding domain capable of binding a target molecule; and
b. a human IgG1 Fc domain that comprises an amino acid sequence that is at least 80% identical to the amino acid sequence of SEQ ID NO: 60 and wherein the amino acids at positions 265, 297, and 329 according to the EU index as in Kabat are different from D, N, and P, respectively,

wherein the recombinant polypeptide retains binding to FcRn, and
wherein the recombinant polypeptide is capable of binding the target molecule without triggering significant lymphocyte activation, complement dependent lysis, and/or cell mediated destruction of the target molecule and/or of a cell that expresses the target molecule on its surface.

**14.** The recombinant polypeptide of claim 13, wherein the at least one binding domain is selected from the group consisting of a binding site of an antibody, a Fynomer, an enzyme, a hormone, an extracellular domain of a receptor, a cytokine, an immune cell surface antigen, a ligand, and an adhesion molecule.

**15.** The recombinant polypeptide of claim 13 or 14, wherein the Fc domain is at least 80%, preferably at least 90% identical to the amino acid sequence of the human IgG1 Fc domain comprising SEQ ID NO: 43.

**16.** The recombinant polypeptide of any one of claims 13-15 wherein the binding domain is the binding site of an antibody.

**17.** A pharmaceutical composition comprising the human IgG1 Fc-containing molecule of any one of claims 1-8, the recombinant polynucleotide of claim 9, the vector of claim 10, or the recombinant polypeptide of any one of claims 13-16, and a pharmaceutically acceptable excipient.

18. A method for producing a recombinant human IgG1 Fc-containing molecule, the method comprising expressing the recombinant polynucleotide of claim 9 in a host cell and harvesting the recombinant polypeptide.

19. A human IgG1 Fc-containing molecule of any one of claims 1-8, a recombinant polynucleotide of claim 9, a vector of claim 10, a recombinant polypeptide of any one of claims 13-16, or a pharmaceutical composition according to claim 17 for use in treating a disease or disorder.

20. The human IgG1 Fc-containing molecule, recombinant polynucleotide, vector, recombinant polypeptide or pharmaceutical composition for use according to claim 19, wherein the disease or disorder is cancer.

**Patentansprüche**

1. Rekombinantes menschliches IgG1-Fc enthaltendes Molekül, umfassend eine CH2-Domäne, die eine Aminosäuresequenz umfasst, die zu wenigstens 80% mit der Aminosäuresequenz unter SEQ ID NO: 60 identisch ist und in der die Aminosäure an Position 265 von Asparaginsäure (D) verschieden ist, die Aminosäure an Position 297 von Asparagin (N) verschieden ist und die Aminosäure an Position 329 von Prolin (P) verschieden ist,

   wobei das Molekül die Bindung an FcRn behält und wobei das Molekül eine wenigstens 20-fach verringerte Bindung an Clq und an wenigstens einen Fcγ-Rezeptor (FcγR) im Vergleich zu einem menschliches Wildtyp-IgGl-Fc enthaltenden Molekül, das D an Position 265, N an Position 297 und P an Position 329 umfasst, aufweist und wobei die Nummerierung über den EU-Index wie bei Kabat angegeben ist.

2. Molekül nach Anspruch 1, wobei das Molekül eine wenigstens 50-fach verringerte Bindung an Clq und an wenigstens einen FcγR im Vergleich zu einem menschliches Wildtyp-IgGl-Fc enthaltenden Molekül, das D an Position 265, N an Position 297 und P an Position 329 umfasst, aufweist.

3. Molekül nach Anspruch 1 oder 2, wobei wenigstens ein FcγR ausgewählt ist aus FcγRI, FcγRIIa, FcγRIIb, FcγRIIIa und FcγRIIIb.

4. Molekül nach einem der Ansprüche 1 bis 3, wobei

   i. die Aminosäure an Position 265 Alanin (A), Asparagin (N) oder Glutaminsäure (E) ist,
   ii. die Aminosäure an Position 297 Alanin (A), Asparaginsäure (D) oder Glutamin (Q) ist und
   iii. die Aminosäure an Position 329 durch Alanin (A), Glycin (G) oder Serin (S) ersetzt ist.

5. Molekül nach einem der Ansprüche 1-4, wobei die CH2-Domäne eine Aminosäuresequenz umfasst, die zu wenigstens 90% mit der Aminosäuresequenz unter SEQ ID NO: 60 identisch ist.

6. Molekül nach einem der Ansprüche 1-5, wobei die Fc-Domäne eine Aminosäuresequenz umfasst, die zu wenigstens 80%, bevorzugt zu wenigstens 90% mit der Aminosäuresequenz der SEQ ID NO: 43 umfassenden menschlichen IgG1-Fc-Domäne identisch ist.

7. Molekül nach einem der Ansprüche 1-6, wobei es sich bei dem Molekül um einen Antikörper, eine Fc-Region, ein Fc-Fusionsprotein oder Antikörper-Fusionsprotein, wie etwa ein FynomAb, handelt.

8. Molekül nach einem der Ansprüche 1-7, wobei das Molekül eine Fc-Region umfasst, die eine Sequenz gemäß einer von SEQ ID NO: 43, 52, 53, 54, 55, 56, 57 oder 58 umfasst, wobei Aminosäuren D an Position 265, N an Position 297 und P an Position 329 durch andere Aminosäuren ersetzt sind.

9. Rekombinantes Polynukleotid, codierend das Molekül nach einem der vorhergehenden Ansprüche.

10. Vektor, umfassend das Polynukleotid nach Anspruch 9.

11. Wirtszelle, umfassend das rekombinante Polynukleotid nach Anspruch 9 oder den Vektor nach Anspruch 10.

12. Verfahren zur Herstellung eines rekombinantes menschliches IgG1-Fc enthaltenden Moleküls, umfassend eine CH2-Domäne, die eine Aminosäuresequenz umfasst, die zu wenigstens 80% mit der Aminosäuresequenz unter SEQ ID NO: 60 identisch ist und in der Aminosäuren an Position 265, 297, und 329, angegeben über den EU-Index wie bei Kabat, durch andere Aminosäuren ersetzt sind, wobei das Verfahren die folgenden Schritte umfasst:

a. Bereitstellen einer Nukleinsäure, die ein menschliches Wildtyp-IgGI-Fc enthaltendes Molekül codiert,
b. Modifizieren der in Schritt (a) bereitgestellten Nukleinsäure, um so eine Nukleinsäure zu erhalten, die ein rekombinantes menschliches IgG1-Fc enthaltendes Molekül codiert, wobei die Aminosäuren an Position 265, 297 bzw. 329 durch andere Aminosäuren außer D, N bzw. P ersetzt sind, und
c. Exprimieren der in Schritt (b) erhaltenen Nukleinsäure in einer Wirtszelle und Gewinnen des rekombinantes menschliches IgG1-Fc enthaltenden Moleküls,

wobei das rekombinantes menschliches IgG1-Fc enthaltende Molekül die Bindung an FcRn behält und wobei das rekombinantes menschliches IgG1-Fc enthaltende Molekül eine wenigstens 20-fach verringerte Bindung an Clq und an wenigstens einen Fcγ-Rezeptor (FcγR) im Vergleich zu einem menschliches Wildtyp-IgGI-Fc enthaltenden Molekül, das D an Position 265, N an Position 297 und P an Position 329 umfasst, aufweist.

**13.** Rekombinantes Polypeptid, umfassend

a. wenigstens eine Bindungsdomäne, die ein Zielmolekül binden kann; und
b. eine menschliche IgG1-Fc-Domäne, die eine Aminosäuresequenz umfasst, die zu wenigstens 80% mit der Aminosäuresequenz unter SEQ ID NO: 60 identisch ist und worin die Aminosäuren an Position 265, 297 bzw. 329 gemäß dem EU-Index wie bei Kabat von D, N, bzw. P verschieden sind,

wobei das rekombinante Polypeptid die Bindung an FcRn behält und
wobei das rekombinante Polypeptid das Zielmolekül binden kann, ohne signifikante Lymphozytenaktivierung, komplementabhängige Lyse und/oder zellvermittelte Zerstörung des Zielmoleküls und/oder einer Zelle, die das Zielmolekül an ihrer Oberfläche exprimiert, auszulösen.

**14.** Rekombinantes Polypeptid nach Anspruch 13, wobei die wenigstens eine Bindungsdomäne ausgewählt ist aus der Gruppe bestehend aus einer Bindungsstelle eines Antikörpers, einem Fynomer, einem Enzym, einem Hormon, einer extrazellulären Domäne eines Rezeptors, einem Cytokin, einem Immunzelloberflächenantigen, einem Liganden und einem Adhäsionsmolekül.

**15.** Rekombinantes Polypeptid nach Anspruch 13 oder 14, wobei die Fc-Domäne zu wenigstens 80%, bevorzugt zu wenigstens 90% mit der Aminosäuresequenz der SEQ ID NO: 43 umfassenden menschlichen IgG1-Fc-Domäne identisch ist.

**16.** Rekombinantes Polypeptid nach einem der Ansprüche 13-15, wobei es sich bei der Bindungsdomäne um die Bindungsstelle eines Antikörpers handelt.

**17.** Pharmazeutische Zusammensetzung, umfassend das menschliches IgG1-Fc enthaltende Molekül nach einem der Ansprüche 1-8, das rekombinante Polynukleotid nach Anspruch 9, den Vektor nach Anspruch 10 oder das rekombinante Polypeptid nach einem der Ansprüche 13-16 sowie einen pharmazeutisch unbedenklichen Exzipienten.

**18.** Verfahren zur Erzeugung eines rekombinantes menschliches IgG1-Fc enthaltenden Moleküls, wobei das Verfahren Exprimieren des rekombinanten Polynukleotids nach Anspruch 9 in einer Wirtszelle und Ernten des rekombinanten Polypeptids umfasst.

**19.** Menschliches IgG1-Fc enthaltendes Molekül nach einem der Ansprüche 1-8, rekombinantes Polynukleotid nach Anspruch 9, Vektor nach Anspruch 10, rekombinantes Polypeptid nach einem der Ansprüche 13-16 oder pharmazeutische Zusammensetzung gemäß Anspruch 17 zur Verwendung bei der Behandlung einer Krankheit oder Störung.

**20.** Menschliches IgG1-Fc enthaltendes Molekül, rekombinantes Polynukleotid, Vektor, rekombinantes Polypeptid oder pharmazeutische Zusammensetzung zur Verwendung gemäß Anspruch 19, wobei es sich bei der Krankheit oder Störung um Krebs handelt.

## Revendications

1. Molécule contenant Fc d'IgG1 humain recombinante, comprenant un domaine CH2 qui comprend une séquence d'acides aminés qui est au moins 80 % identique à la séquence d'acides aminés de SEQ ID NO : 60 et dans laquelle l'acide aminé à la position 265 est différent d'acide aspartique (D), l'acide aminé à la position 297 est différent d'asparagine (N), et l'acide aminé à la position 329 est différent de proline (P),

   où la molécule conserve la liaison à FcRn, et où la molécule a une liaison réduite d'au moins 20 fois à Clq et à au moins un récepteur de Fcγ (FcγR), par rapport à une molécule contenant Fc d'IgG1 humain de type sauvage qui comprend D à la position 265, N à la position 297 et P à la position 329,
   et dans laquelle la numérotation est indiquée par l'index EU comme décrit dans Kabat.

2. Molécule selon la revendication 1, où la molécule a une liaison réduite d'au moins 50 fois à Clq et à au moins un FcγR, par rapport à une molécule contenant Fc d'IgG1 humain de type sauvage qui comprend D à la position 265, N à la position 297 et P à la position 329.

3. Molécule selon la revendication 1 ou 2, dans laquelle au moins un FcγR est choisi parmi FcγRI, FcγRIIa, FcγRIIb, FcγRIIIa, et FcγRIIIb.

4. Molécule selon l'une quelconque des revendications 1 à 3, dans laquelle

   i. l'acide aminé à la position 265 est alanine (A), asparagine (N) ou acide glutamique (E),
   ii. l'acide aminé à la position 297 est alanine (A), acide aspartique (D), ou glutamine (Q), et
   iii. l'acide aminé à la position 329 est remplacé par alanine (A), glycine (G) ou sérine (S).

5. Molécule selon l'une quelconque des revendications 1 à 4, dans laquelle le domaine CH2 comprend une séquence d'acides aminés qui est au moins 90 % identique à la séquence d'acides aminés de SEQ ID NO : 60.

6. Molécule selon l'une quelconque des revendications 1 à 5, dans laquelle le domaine Fc comprend une séquence d'acides aminés qui est au moins 80 %, de préférence au moins 90 % identique à la séquence d'acides aminés du domaine Fc d'IgG1 humain comprenant SEQ ID NO : 43.

7. Molécule selon l'une quelconque des revendications 1 à 6, où la molécule est un anticorps, une région Fc, une protéine de fusion de Fc ou une protéine de fusion d'anticorps telle qu'un FynomAb.

8. Molécule selon l'une quelconque des revendications 1 à 7, où la molécule comprend une région Fc comprenant une séquence selon l'une quelconque de SEQ ID NO : 43, 52, 53, 54, 55, 56, 57 ou 58, dans laquelle les acides aminés D à la position 265, N à la position 297 et P à la position 329 sont remplacés par d'autres acides aminés.

9. Polynucléotide recombinant codant pour la molécule selon l'une quelconque des revendications précédentes.

10. Vecteur comprenant le polynucléotide selon la revendication 9.

11. Cellule hôte comprenant le polynucléotide recombinant selon la revendication 9 ou le vecteur selon la revendication 10.

12. Procédé de fabrication d'une molécule contenant Fc d'IgG1 humain recombinante, comprenant un domaine CH2 qui comprend une séquence d'acides aminés qui est au moins 80 % identique à la séquence d'acides aminés de SEQ ID NO : 60 et dans laquelle les acides aminés aux positions 265, 297 et 329 indiquées par l'index EU comme décrit dans Kabat sont remplacés par d'autres acides aminés, le procédé comprenant les étapes de :

   a. fourniture d'un acide nucléique codant pour une molécule contenant Fc d'IgG1 humain de type sauvage,
   b. modification de l'acide nucléique produit dans l'étape (a) de façon à obtenir un acide nucléique codant pour une molécule contenant Fc d'IgG1 humain recombinante dans laquelle les acides aminés aux positions 265, 297 et 329 sont remplacés par des acides aminés autres que D, N et P, respectivement, et
   c. expression de l'acide nucléique obtenu dans l'étape (b) dans une cellule hôte et récupération de ladite molécule contenant Fc d'IgG1 humain recombinante,

dans lequel la molécule contenant Fc d'IgG1 humain recombinante conserve la liaison à FcRn, et dans lequel la molécule contenant Fc d'IgG1 humain recombinante a une liaison réduite d'au moins 20 fois à Clq et à au moins un récepteur de Fcγ (FcγR), par rapport à une molécule contenant Fc d'IgG1 humain de type sauvage qui comprend D à la position 265, N à la position 297 et P à la position 329.

**13.** Polypeptide recombinant comprenant

a. au moins un domaine de liaison capable de se lier à une molécule cible ; et
b. un domaine Fc d'IgG1 humain qui comprend une séquence d'acides aminés qui est au moins 80 % identique à la séquence d'acides aminés de SEQ ID NO : 60 et dans lequel les acides aminés aux positions 265, 297 et 329 selon l'index EU comme décrit dans Kabat sont différents de D, N et P, respectivement,

où le polypeptide recombinant conserve la liaison à FcRn, et
où le polypeptide recombinant est capable de se lier à la molécule cible sans déclencher une activation de lymphocytes, une lyse dépendante du complément, et/ou la destruction à médiation cellulaire de la molécule cible et/ou d'une cellule qui exprime la molécule cible sur sa surface significatives.

**14.** Polypeptide recombinant selon la revendication 13, dans lequel l'au moins un domaine de liaison est choisi dans le groupe constitué d'un site de liaison d'un anticorps, d'un fynomère, d'une enzyme, d'une hormone, d'un domaine extracellulaire d'un récepteur, d'une cytokine, d'un antigène de surface de cellule immunitaire, d'un ligand, et d'une molécule d'adhésion.

**15.** Polypeptide recombinant selon la revendication 13 ou 14, dans lequel le domaine Fc est au moins 80 %, de préférence au moins 90 % identique à la séquence d'acides aminés du domaine Fc d'IgG1 humain comprenant SEQ ID NO : 43.

**16.** Polypeptide recombinant selon l'une quelconque des revendications 13 à 15 dans lequel le domaine de liaison est le site de liaison d'un anticorps.

**17.** Composition pharmaceutique comprenant la molécule contenant Fc d'IgG1 humain selon l'une quelconque des revendications 1 à 8, le polynucléotide recombinant selon la revendication 9, le vecteur selon la revendication 10, ou le polypeptide recombinant selon l'une quelconque des revendications 13 à 16, et un excipient pharmaceutiquement acceptable.

**18.** Procédé de production d'une molécule contenant Fc d'IgG1 humain recombinante, le procédé comprenant l'expression du polynucléotide recombinant selon la revendication 9 dans une cellule hôte et la collecte du polypeptide recombinant.

**19.** Molécule contenant Fc d'IgG1 humain selon l'une quelconque des revendications 1 à 8, polynucléotide recombinant selon la revendication 9, vecteur selon la revendication 10, polypeptide recombinant selon l'une quelconque des revendications 13 à 16, ou composition pharmaceutique selon la revendication 17 pour utilisation dans le traitement d'une maladie ou d'un trouble.

**20.** Molécule contenant Fc d'IgG1 humain, polynucléotide recombinant, vecteur, polypeptide recombinant ou composition pharmaceutique pour utilisation selon la revendication 19, où la maladie ou le trouble est un cancer.

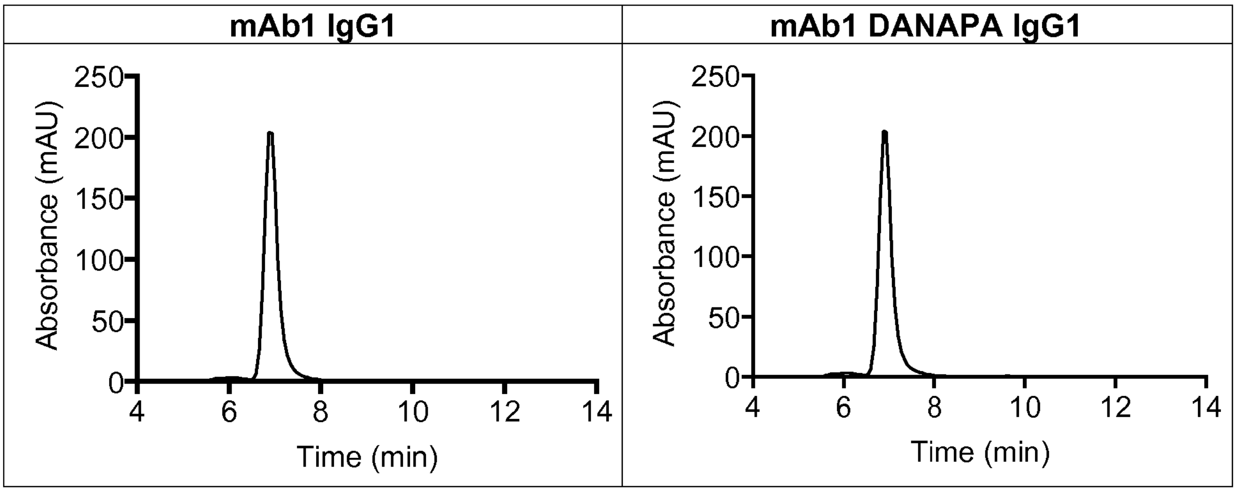
mAb1 IgG1
mAb1 DANAPA IgG1
Absorbance (mAU)
250
200
150
100
50
0
4
6
8
10
12
14
Time (min)

Fig. 1

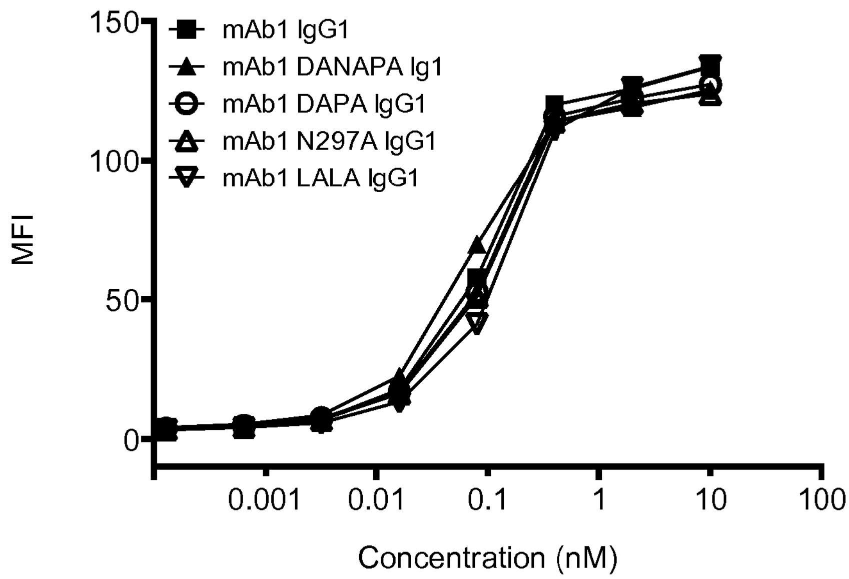
150
100
50
0
MFI
mAb1 IgG1
mAb1 DANAPA Ig1
mAb1 DAPA IgG1
mAb1 N297A IgG1
mAb1 LALA IgG1
0.001
0.01
0.1
1
10
100
Concentration (nM)

Fig. 2

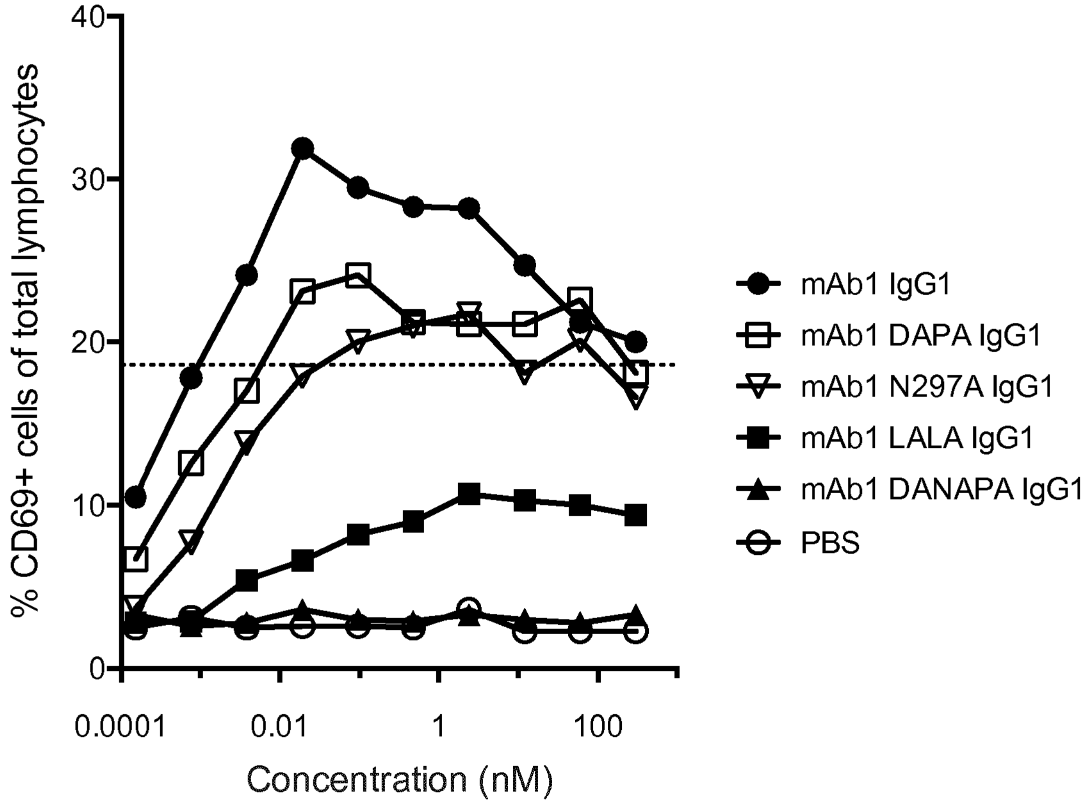
40
30
20
10
0
% CD69+ cells of total lymphocytes
0.0001
0.01
1
100
Concentration (nM)
mAb1 IgG1
mAb1 DAPA IgG1
mAb1 N297A IgG1
mAb1 LALA IgG1
mAb1 DANAPA IgG1
PBS

Fig. 3A

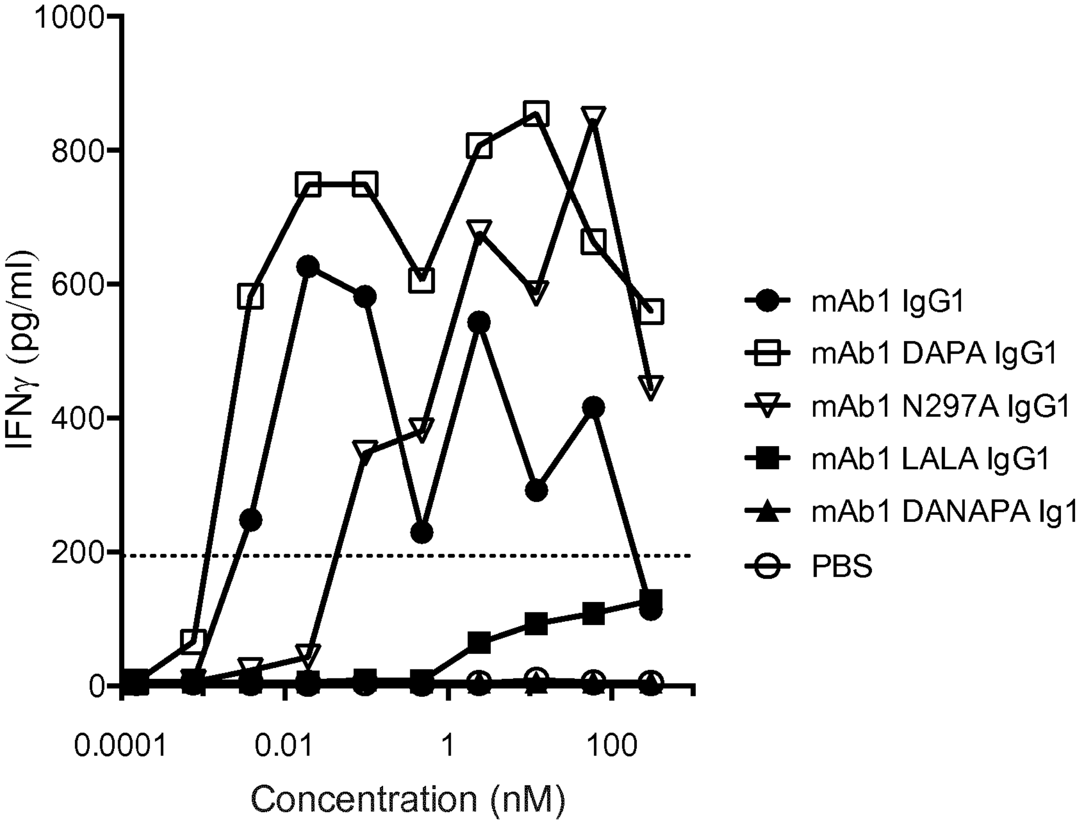
1000
800
600
400
200
0
IFNγ (pg/ml)
0.0001
0.01
1
100
Concentration (nM)
mAb1 IgG1
mAb1 DAPA IgG1
mAb1 N297A IgG1
mAb1 LALA IgG1
mAb1 DANAPA Ig1
PBS

Fig. 3B

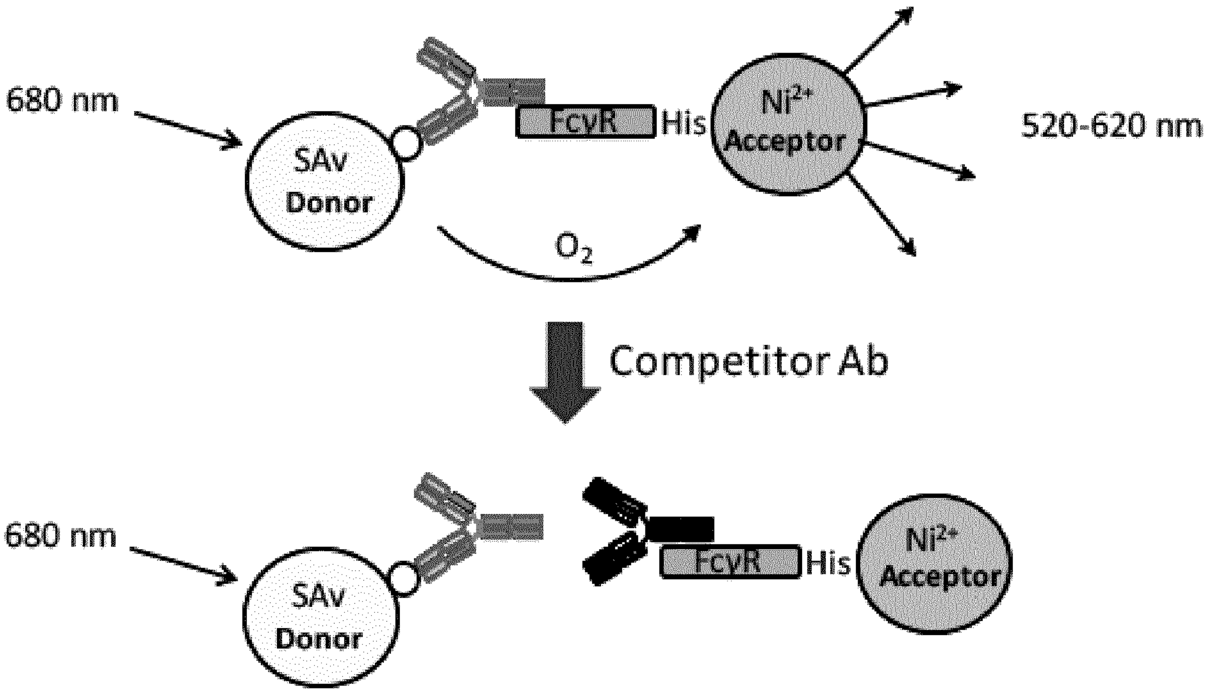
680 nm
SAv
Donor
FcγR
His
Ni2+
Acceptor
520-620 nm
O2
Competitor Ab
680 nm
SAv
Donor
FcγR
His
Ni2+
Acceptor

Fig. 4A

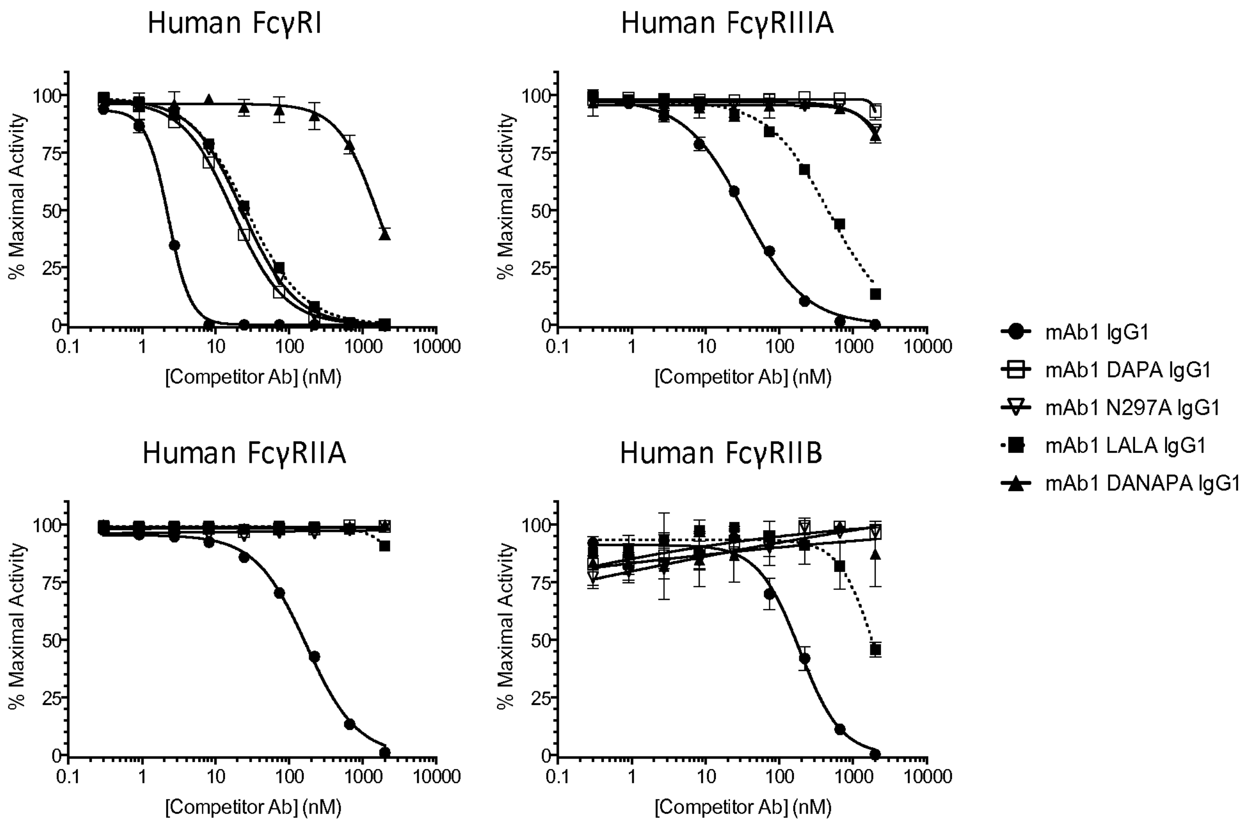
Human FcγRI
Human FcγRIIIA
Human FcγRIIA
Human FcγRIIB
% Maximal Activity
[Competitor Ab] (nM)
0
25
50
75
100
0.1
1
10
100
1000
10000
mAb1 IgG1
mAb1 DAPA IgG1
mAb1 N297A IgG1
mAb1 LALA IgG1
mAb1 DANAPA IgG1

Fig. 4B

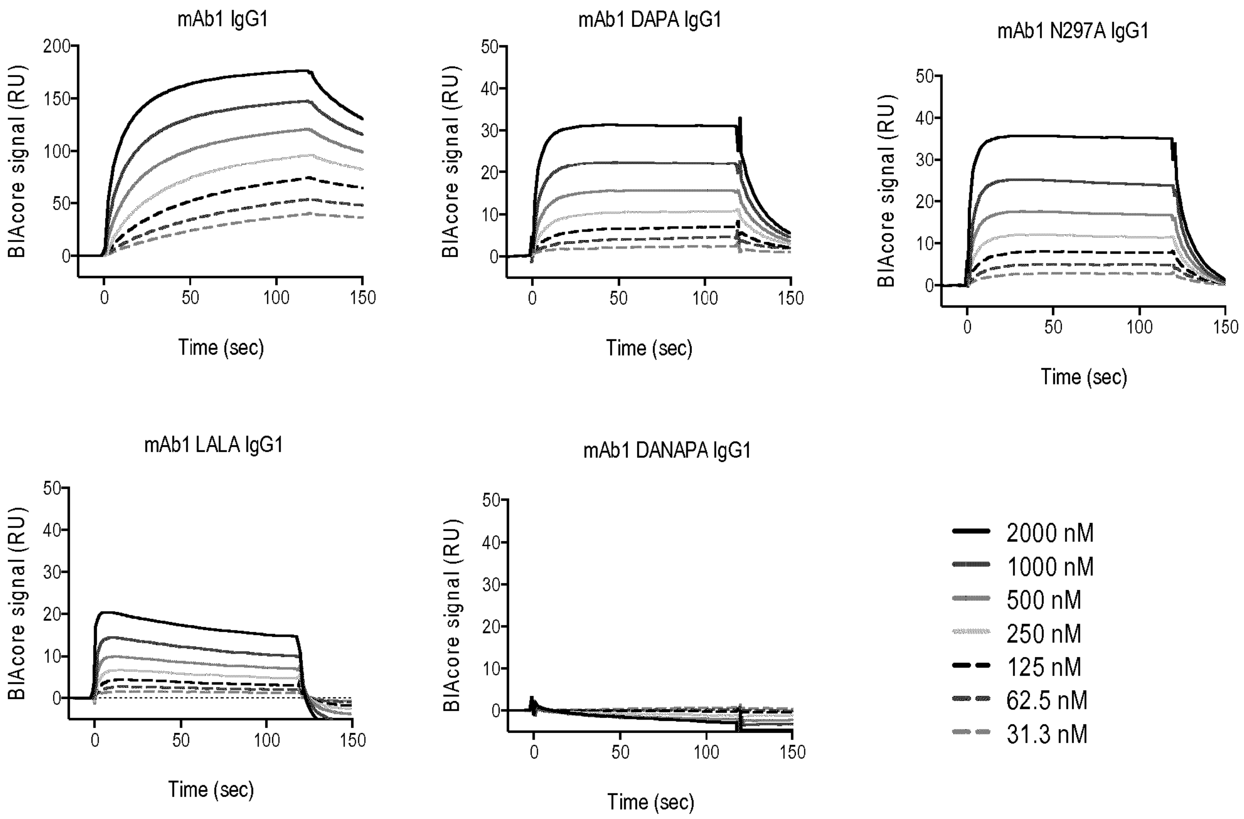
mAb1 IgG1
mAb1 DAPA IgG1
mAb1 N297A IgG1
mAb1 LALA IgG1
mAb1 DANAPA IgG1
BIAcore signal (RU)
Time (sec)
2000 nM
1000 nM
500 nM
250 nM
125 nM
62.5 nM
31.3 nM

Fig. 4C

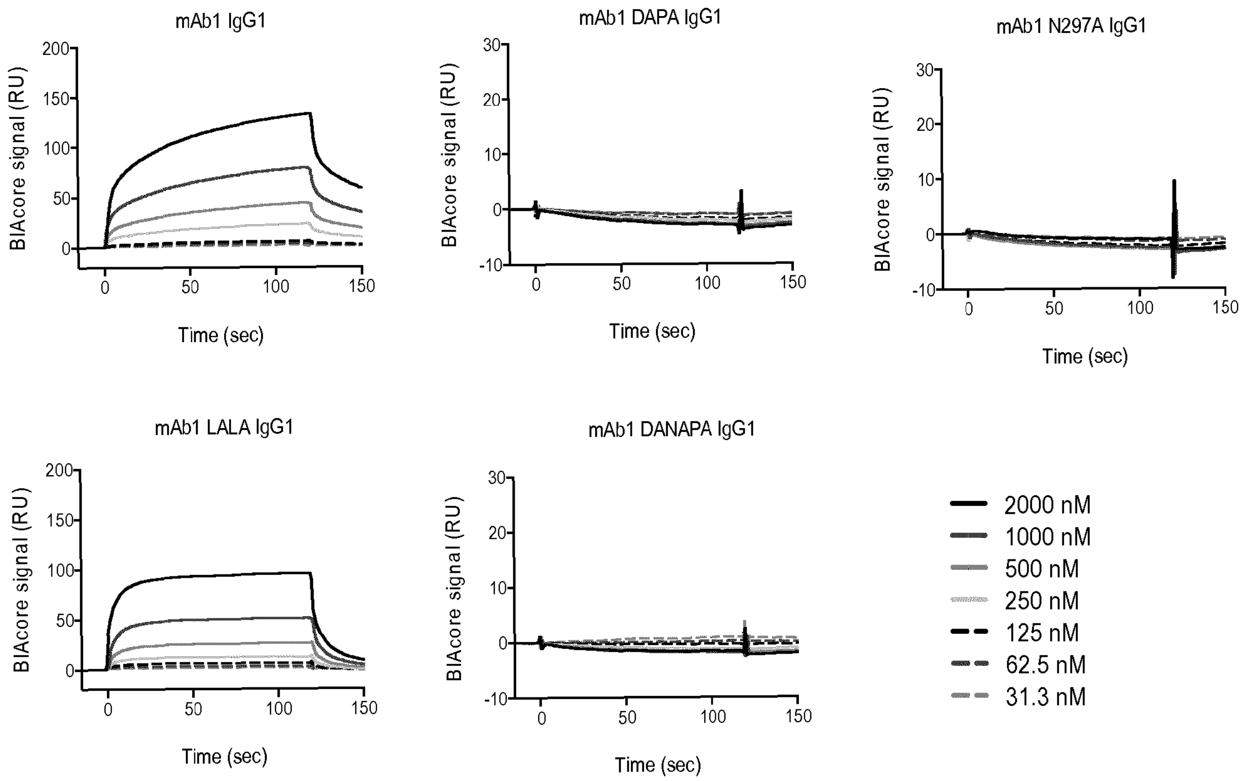
mAb1 IgG1
mAb1 DAPA IgG1
mAb1 N297A IgG1
mAb1 LALA IgG1
mAb1 DANAPA IgG1
BIAcore signal (RU)
Time (sec)
2000 nM
1000 nM
500 nM
250 nM
125 nM
62.5 nM
31.3 nM

Fig. 4D

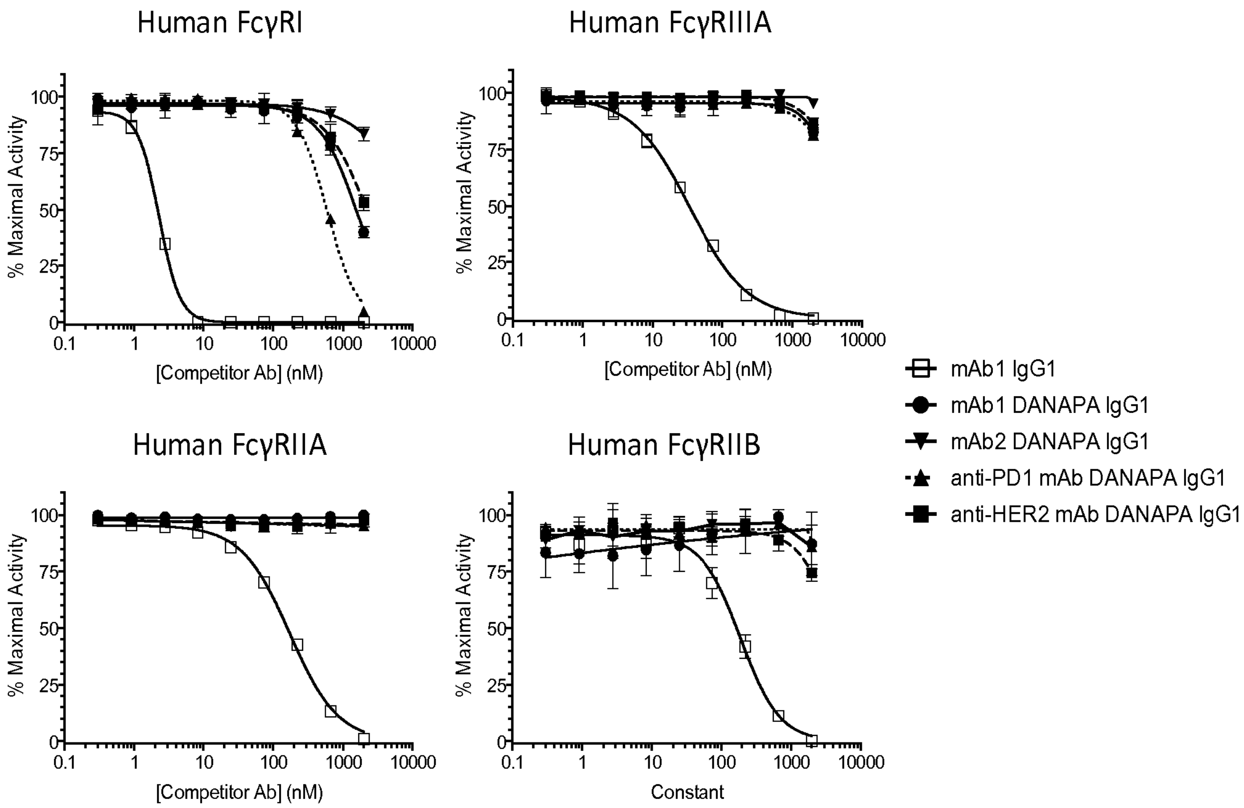
Human FcγRI
Human FcγRIIIA
Human FcγRIIA
Human FcγRIIB
% Maximal Activity
[Competitor Ab] (nM)
Constant
0
25
50
75
100
0.1
1
10
100
1000
10000
mAb1 IgG1
mAb1 DANAPA IgG1
mAb2 DANAPA IgG1
anti-PD1 mAb DANAPA IgG1
anti-HER2 mAb DANAPA IgG1

Fig. 5

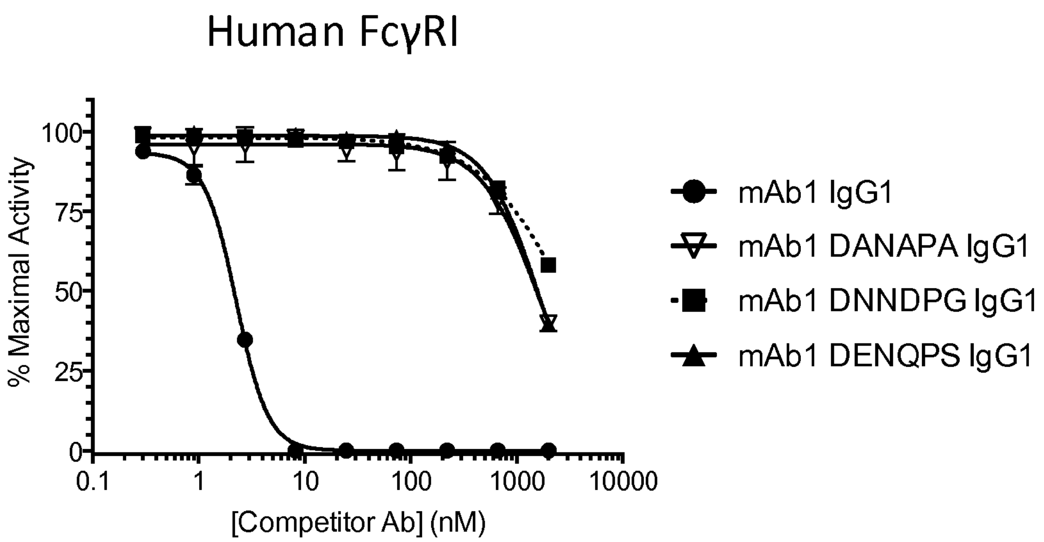
Human FcγRI
% Maximal Activity
100
75
50
25
0
0.1
1
10
100
1000
10000
[Competitor Ab] (nM)
mAb1 IgG1
mAb1 DANAPA IgG1
mAb1 DNNDPG IgG1
mAb1 DENQPS IgG1

Fig. 6

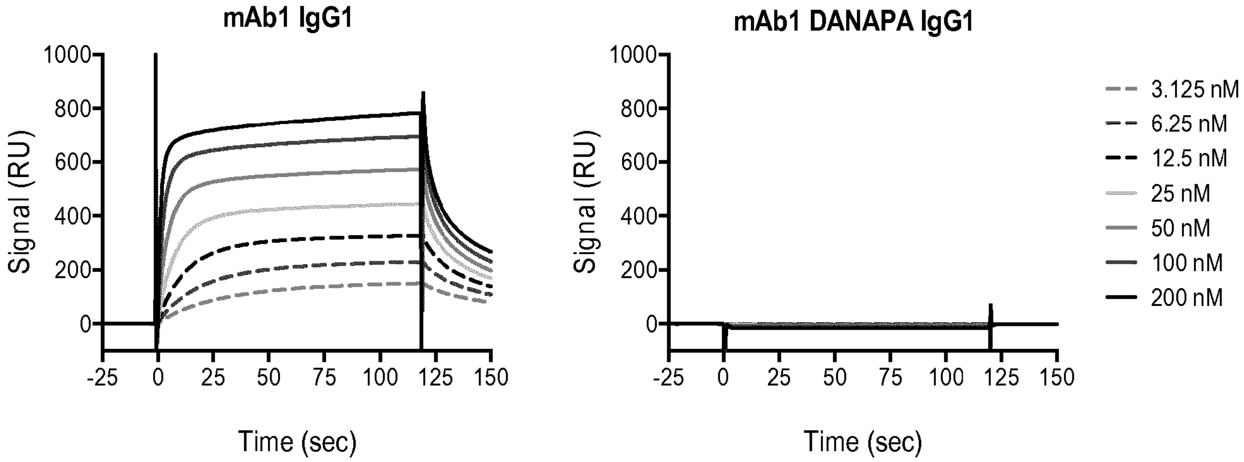
mAb1 IgG1
mAb1 DANAPA IgG1
Signal (RU)
Time (sec)
1000
800
600
400
200
0
-25
0
25
50
75
100
125
150
3.125 nM
6.25 nM
12.5 nM
25 nM
50 nM
100 nM
200 nM

Fig. 7

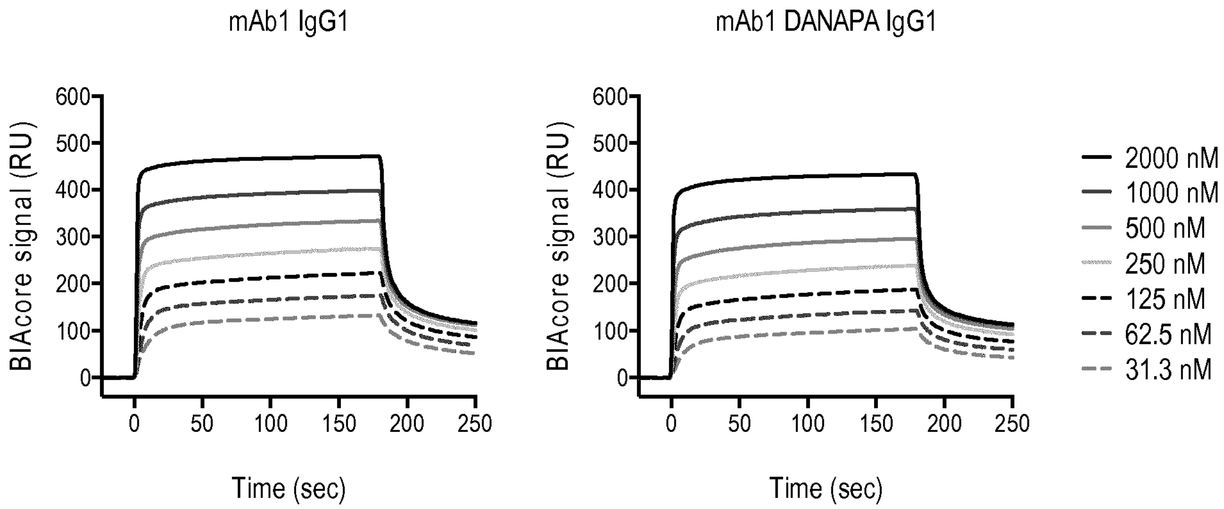
mAb1 IgG1
mAb1 DANAPA IgG1
BIAcore signal (RU)
600
500
400
300
200
100
0
0
50
100
150
200
250
Time (sec)
2000 nM
1000 nM
500 nM
250 nM
125 nM
62.5 nM
31.3 nM

Fig. 8

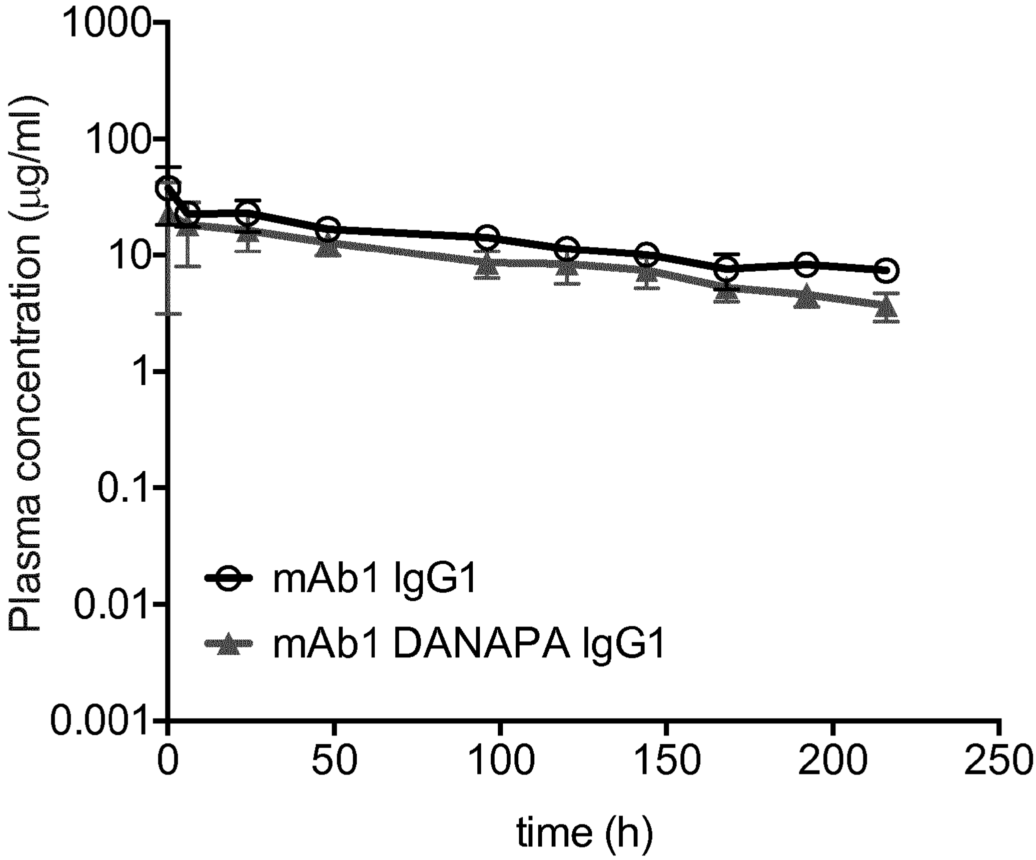
1000
100
10
1
0.1
0.01
0.001
Plasma concentration (µg/ml)
0
50
100
150
200
250
time (h)
mAb1 IgG1
mAb1 DANAPA IgG1

Fig. 9

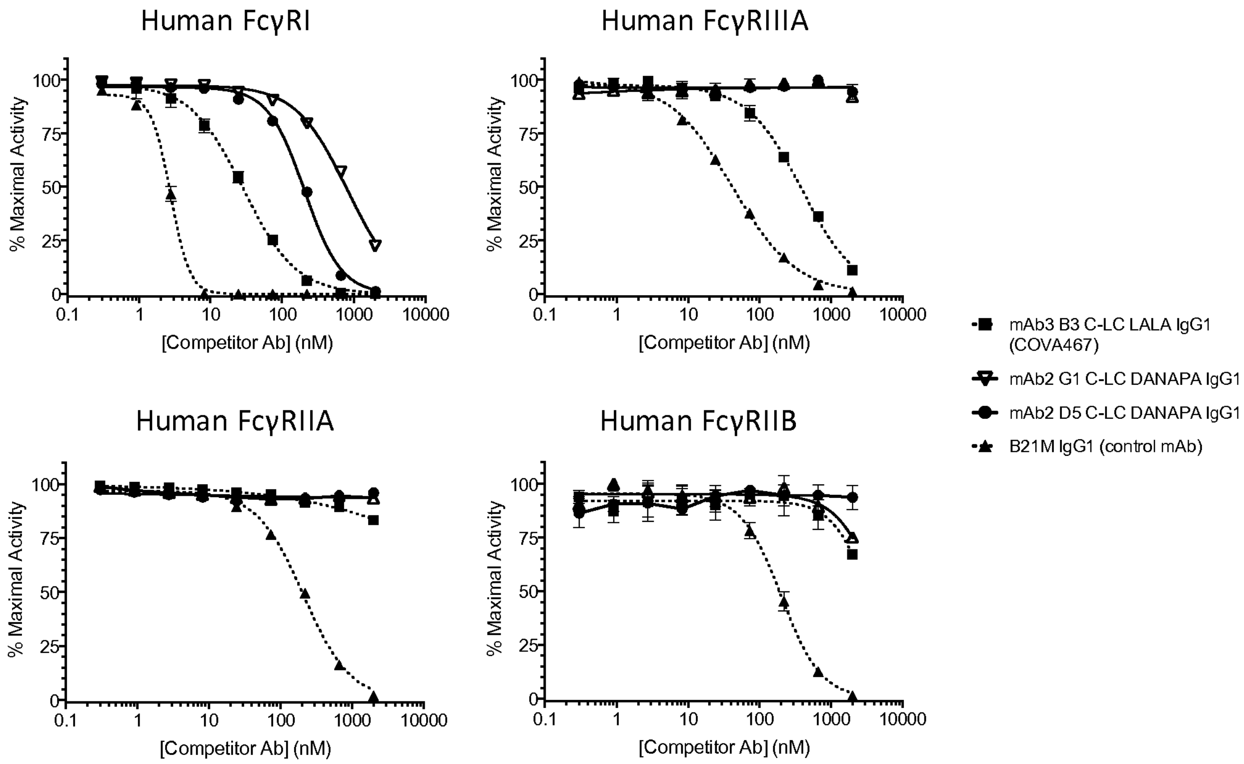
Human FcγRI
Human FcγRIIIA
Human FcγRIIA
Human FcγRIIB
% Maximal Activity
[Competitor Ab] (nM)
0
25
50
75
100
0.1
1
10
100
1000
10000
mAb3 B3 C-LC LALA IgG1 (COVA467)
mAb2 G1 C-LC DANAPA IgG1
mAb2 D5 C-LC DANAPA IgG1
B21M IgG1 (control mAb)

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012143524 A
- WO 2014108483 A
- WO 2008022759 A
- WO 2014044758 A1
- WO 2014170063 A1
- WO 2015141862 A1
- WO 2013135588 A
- WO 9211018 A
- WO 2014170063 A

### Non-patent literature cited in the description


- **WOOF JM ; DR BURTON.** *Nat Rev Immunol,* 2004, vol. 4 (2), 89-99
- **BRUHNS P et al.** *Blood,* 2009, vol. 113 (16), 3716-3725
- **REDMAN JM et al.** *Mol Immunol,* 2015, vol. 67 (2), 28-45
- **STEWART RH et al.** *Journal of ImmunoTherapy of Cancer,* 2014, vol. 2 (29
- **WHITE AL et al.** *J Immunol,* 2011, vol. 187 (4), 1754-1763
- **NOSE M ; H WIGZELL.** *Proc Natl Acad Sci U S A,* 1983, vol. 80 (21), 6632-6636
- **TAO MH ; SL MORRISON.** *J Immunol,* 1989, vol. 143 (8), 2595-2601
- **BOLT S et al.** *Eur J Immunol,* 1993, vol. 23 (2), 403-411
- **STROHL WR.** *Curr Opin Biotechnol,* 2009, vol. 20 (6), 685-691
- **PARREN PW et al.** *J Immunol,* 1992, vol. 148 (3), 695-701
- **XU D et al.** *Cell Immunol,* 2000, vol. 200 (1), 16-26
- **CANFIELD SM ; SL MORRISON.** *J Exp Med,* 1991, vol. 173 (6), 1483-1491
- **HEROLD KC et al.** *Diabetes,* 2005, vol. 54 (6), 1763-1769
- **SHIELDS et al.** *J Biol Chem,* 2001, vol. 276 (9), 6591-6604
- **IDUSOGIE EE et al.** *J Immunol,* 2000, vol. 164 (8), 4178-4184
- **WILSON NS et al.** *Cancer Cell,* 2011, vol. 19 (1), 101-113
- **GONG Q et al.** *J Immunol,* 2005, vol. 174 (2), 817-826
- **SHIELDS RL et al.** *J Biol Chem,* 2001, vol. 276, 6591-6604
- **STAVENHAGEN JB et al.** *Cancer Res,* 2007, vol. 67, 8882-8890
- **SAZINSKY SL et al.** *Proc Natl Acad Sci USA,* 2008, vol. 105, 20167-20172
- **KELTON W et al.** *Chem Biol,* 2014, vol. 21, 1603-1609
- **SCHLOTHAUER T et al.** *Protein Eng Des Sel,* 2016, vol. 29, 457-466
- **SHIELDS RL et al.** *J Biol Chem,* 2001, vol. 276 (9), 6591-6604
- **KUO TT ; VG AVESON.** *MAbs,* 2011, vol. 3 (5), 422-430
- **MARTIN WL et al.** *Mol Cell,* 2001, vol. 7 (4), 867-877
- **KABAT et al.** Sequences of Proteins of Immunological Interest. Public Health Service, National Institutes of Health, 1991
- **NIMMERJAHN ; RAVETCH.** *Nature Reviews Immunology,* 2008, vol. 8, 34-47
- **KISHORE et al.** *Immunopharmacology,* 2000, vol. 49, 159-170
- **SJÖBERG et al.** *Trends Immunol.,* 2009, vol. 30 (2), 83-90
- **ROOPENIAN.** *Nature Reviews Immunology,* 2007, vol. 7, 715-725
- **ALTSCHUL SF et al.** *Nucleic Acids Res,* 1997, vol. 25, 3389-3402
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat. Acad. Sci. U.S.A.,* 1988, vol. 85, 2444
- **DEVEREUX et al.** *Nucl. Acid Res.,* 1984, vol. 12, 387-395
- Current Methods in Sequence Comparison and Analysis. Macromolecule Sequencing and Synthesis, Selected Methods and Applications. Alan R. Liss, Inc, 1988, 127-149
- **ALTSCHUL et al.** *J. Mol. Biol.,* 1990, vol. 215, 403-410
- **ALTSCHUL et al.** *Nucleic Acids Res.,* 1997, vol. 25, 3389-3402

- **KARIN et al.** *Proc. Natl. Acad. Sci. U.S.A.,* 1993, vol. 90, 5873-5787
- **ALTSCHUL et al.** *Methods in Enzymology,* 1996, vol. 266, 460-480
- **ALTSCHUL et al.** *Nucl. Acids Res.,* 1993, vol. 25, 3389-3402
- **JEFFERIS R ; M-P LEFRANC.** *mAbs,* 2009, vol. 1, 1-7
- **MANIATIS.** Molecular Cloning - A Laboratory Manual. Cold Spring Harbor Laboratory Press, 2001
- **SAMBROOK ; RUSSELL.** Molecular cloning: a laboratory manual. CSHL Press, 2006
- Current Protocols in Molecular Biology. John Wiley & Sons, 2004
- **GORDON et al.** *Proc Natl Acad Sci U S A.,* 1980, vol. 77, 7380-4
- Manipulating the Mouse Embryo, A Laboratory Manual. Cold Spring Harbor Laboratory Press, 1994
- Gene Targeting, A Practical Approach. IRL Press at Oxford University Press, 1993
- **EVANS ; KAUFMAN.** *Nature,* 1981, vol. 292, 154-156
- **RYAN et al.** *Science,* 1997, vol. 278, 873-876
- **CIBELLI et al.** *Science,* 1998, vol. 280, 1256-1258
- **ASHKENAZI A ; CHAMOW SM.** *Curr Opin Immunol.,* 1997, vol. 9 (2), 195-200
- **GRABULOVSKI et al.** *JBC,* 2007, vol. 282, 3196-3204
- **BERTSCHINGER et al.** *Protein Eng Des Sel,* 2007, vol. 20 (2), 57-68
- **GEBAUER ; SKERRA.** *Curr Opinion in Chemical Biology,* 2009, vol. 13, 245-255
- **SILACCI et al.** *mAbs,* 2016, vol. 8 (1), 141-149
- **BRACK et al.** *Mol Cancer Ther,* 2014, vol. 13 (8), 2030-9
- **BRACK et al.** *Mol Cancer Ther,* 2014, vol. 13, 2030-2039
- **JONES.** *Nature,* 1986, vol. 321, 522-525
- **VERHOEYEN et al.** *Science,* 1988, vol. 239, 1534-1536
- **TSURUSHITA ; VASQUEZ.** Humanization of Monoclonal Antibodies, Molecular Biology of B Cells. Elsevier Science, 2004, 533-545
- **GILLILAND et al.** *PNAS,* 1980, vol. 77, 4539-4543
- Remington: The Science and Practice of Pharmacy. Lippincott Williams & Wilkins, 2005
- Handbook of Pharmaceuticals Excipients. American Pharmaceutical Association (Pharmaceutical Press, 2009
- **KABAT, E. A.** Sequences of proteins of immunological interest. U.S. Dept. of Health and Human Services, Public Health Service, National Institutes of Health, 1991
- **VAFA, O. ; G. L. GILLILAND ; R. J. BREZSKI ; B. STRAKE ; T. WILKINSON ; E. R. LACY ; B. SCALLON ; A. TEPLYAKOV ; T. J. MALIA ; W. R. STROHL.** *Methods,* 2014, vol. 65 (1), 114-126
- **MOORE GL et al.** *Mabs,* 2010, vol. 2 (2), 181-189
- **GONG, Q et al.** *J Immunol,* 2005, vol. 174 (2), 817-826